# EUROPÄISCHE PATENTSCHRIFT

(11) **EP 1 626 971 B1**
(45) Veröffentlichungstag und Bekanntmachung des Hinweises auf die Patenterteilung: **10.08.2011**
(21) Anmeldenummer: 04729876.5
(22) Anmeldetag: 28.04.2004
(51) Int. Cl.: C07D 487/04, A61K 31/519, A61P 25/00, A61P 25/28

(54) **6-CYCLYLMETHYL- UND 6-ALKYLMETHYL-SUBSTITUIERTE PYRAZOLOPYRIMIDINE**
6-CYCLYLMETHYL- AND 6-ALKYLMETHYL-SUBSTITUTED PYRAZOLOPYRIMIDINES
PYRAZOLOPYRIMIDINES SUBSTITUEES PAR 6-CYCLYLMETHYLE ET 6-ALKYLMETHYLE

(30) Priorität: 09.05.2003 DE 10320784; 07.08.2003 DE 10336183; 28.01.2004 DE 102004004142
(43) Veröffentlichungstag der Anmeldung: 22.02.2006
(60) Teilanmeldung: 10186283.7
(73) Patentinhaber: BOEHRINGER INGELHEIM INTERNATIONAL GMBH, 55216 Ingelheim (DE)
(72) Erfinder: HENDRIX, Martin, Pine Brook, NJ 07058-9714 (US); BÄRFACKER, Lars, 46047 Oberhausen (DE); ERB, Christina, 65189 Wiesbaden (DE); HAFNER, Frank-Thorsten, 42115 Wuppertal (DE); HECKROTH, Heike, 51519 Odenthal (DE); KARTHAUS, Dagmar, 42697 Solingen (DE); TERSTEEGEN, Adrian, 42111 Wuppertal (DE); VAN DER STAAY, Franz-Josef, NL-8251 BB Dronten (NL); VAN KAMPEN, Marja, 41464 Neuss (DE)
(86) Internationale Anmeldenummer: PCT/EP2004/004455
(87) Internationale Veröffentlichungsnummer: WO 2004/099211

(56) Entgegenhaltungen:
- WO-A-02/09713
- WO-A-03/093269
- CH-A- 396 925
- REDDY, K. HEMENDER ET AL: "Versatile synthesis of 6-alkyl(aryl)-1H-pyrazolo[3,4-d]pyrimidin- 4[5H]- ones" INDIAN JOURNAL OF CHEMISTRY, SECTION B: ORGANIC CHEMISTRY INCLUDING MEDICINAL CHEMISTRY , 31B(3), 163-6 CODEN: IJSBDB; ISSN: 0376-4699, 1992, XP009034712
- MIYASHITA, AKIRA ET AL: "Studies on pyrazolo[3,4-d]pyrimidine derivatives. XVIII. Facile preparation of 1H-pyrazolo[3,4-d]pyrimidin-4(5H)-ones" HETEROCYCLES , 31(7), 1309-14 CODEN: HTCYAM; ISSN: 0385-5414, 1990, XP002953484
- CHENG, C. C. ET AL: "Potential purine antagonists. VII. Synthesis of 6-alkylpyrazolo[3,4- d]pyrimidines" JOURNAL OF ORGANIC CHEMISTRY , 23, 191-200 CODEN: JOCEAH; ISSN: 0022-3263, 1958, XP002293718

## Beschreibung

Die Erfindung betrifft neue 6-Cyclylmethyl- und 6-Alkylmethyl-substituierte Pyrazolopyrimidine, Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

Inhibition von Phosphodiesterasen moduliert die Spiegel der zyklischen Nukleotide 5'-3' zyklisches Adenosinmonophosphat (cAMP) bzw. 5'-3' zyklisches Guanosinmonophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Proteinkinasen. Die von cAMP aktivierte Proteinkinase wird Proteinkinase A (PKA) genannt, die von cGMP aktivierte Proteinkinase wird Proteinkinase G (PKG) genannt. Aktivierte PKA bzw. PKG können wiederum eine Reihe zellulärer Effektorproteine phosphorylieren (z.B. Ionenkanäle, G-Protein-gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (Übersicht in: Wei et al., Prog. Neurobiol., 1998, 56: 37-64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklische Monophosphate zu den inaktiven Monophosphaten AMP und GMP. Es sind mittlerweile mindestens 21 PDE-Gene beschrieben (Exp. Opin. Investig. Drugs 2000, 9, 1354-3784). Diese 21 PDE-Gene lassen sich aufgrund ihrer Sequenzhomologie in 11 PDE-Familien einteilen (Nomenklatur-Vorschlag siehe http://depts.washington.edu/pde/Nomenclature.html.). Einzelne PDE-Gene innerhalb einer Familie werden durch Buchstaben unterschieden (z.B. PDE1A und PDE1B). Falls noch unterschiedliche Splice-Varianten innerhalb eines Genes vorkommen, wird dies dann durch eine zusätzliche Nummerierung nach dem Buchstaben angegeben (z.B. PDE1A1).

Die humane PDE9A wurde 1998 kloniert und sequenziert. Die Aminosäurenidentität zu anderen PDEs liegt bei maximal 34 % (PDE8A) und minimal 28 % (PDE5A). Mit einer Michaelis-Menten-Konstante (Km-Wert) von 170 nM ist PDE9A hochaffin für cGMP. Darüber hinaus ist PDE9A selektiv für cGMP (Km-Wert für cAMP = 230 µM). PDE9A weist keine cGMP-Bindungsdomäne auf, die auf eine allosterische Enzymregulation durch cGMP schließen ließe. In einer Western Blot-Analyse wurde gezeigt, dass die PDE9A im Mensch unter anderem in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., J. Biol. Chem., 1998,273 (25): 15559-15564). Das Gen für die humane PDE9A liegt auf Chromosom 21q22.3 und enthält 21 Exons. Bislang wurden 4 alternative Spleißvarianten der PDE9A identifiziert (Guipponi et al., Hum. Genet.,1998,103: 386-392). Klassische PDE-Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., J. Biol. Chem., 1998, 273 (25): 15559-15564).

Die Maus-PDE9A wurde 1998 von Soderling et al. (J. Biol. Chem., 1998, 273 (19): 15553-15558) kloniert und sequenziert. Diese ist wie die humane Form hochaffin für cGMP mit einem Km von 70 nM. In der Maus wurde eine besonders hohe Expression in der Niere, Gehirn, Lunge und Herz gefunden. Auch die Maus-PDE9A wird von IBMX in Konzentrationen unter 200 µM nicht gehemmt; der IC₅₀-Wert für Zaprinast liegt bei 29 µM (Soderling et al., J. Biol. Chem., 1998, 273 (19): 15553-15558). Im Rattengehirn wurde gezeigt, dass PDE9A in einigen Hirnregionen stark exprimiert wird. Dazu zählen der Bulbus olfactorius, Hippocampus, Cortex, Basalganglien und basales Vorderhirn (Andreeva et al., J. Neurosci., 2001, 21 (22): 9068-9076). Insbesondere Hippocampus, Cortex und basales Vorderhirn spielen eine wichtige Rolle bei Lern- und Gedächtnisvorgängen.

Wie oben bereits erwähnt, zeichnet sich PDE9A durch eine besonders hohe Affinität für cGMP aus. Deshalb ist PDE9A im Gegensatz zu PDE2A (Km = 10 µM; Martins et al., J. Biol Chem., 1982, 257: 1973-1979), PDE5A (Km = 4 µM; Francis et al., J. Biol. Chem., 1980, 255: 620-626), PDE6A (Km = 17 µM; Gillespie and Beavo, J. Biol. Chem., 1988, 263 (17): 8133-8141) und PDE11A (Km = 0.52 µM; Fawcett et al., Proc. Nat. Acad. Sci., 2000, 97 (7): 3702-3707) schon bei niedrigen physiologischen Konzentrationen aktiv. Im Gegensatz zu PDE2A (Murashima et al., Biochemistry, 1990, 29: 5285-5292) wird die katalytische Aktvität von PDE9A nicht durch cGMP gesteigert, da es keine GAF-Domäne (cGMP-Bindedomäne, über die die PDE-Aktivität allosterisch gesteigert wird) aufweist (Beavo et al., Current Opinion in Cell Biology, 2000, 12: 174-179). PDE9A-Inhibitoren können deshalb zu einer Erhöhung der basalen cGMP-Konzentration führen.

Die WO 98/40384 offenbart Pyrazolopyrimidine, die sich als PDE1-, 2- und 5-Inhibitoren auszeichnen und für die Behandlung von cardiovascularen und cerebrovascularen Erkrankungen sowie Erkrankungen des Urogenitalbereiches eingesetzt werden können.

In CH 396 924, CH 396 925, CH 396 926, CH 396 927, DE 1 147 234, DE 1 149 013, GB 937,726 werden Pyrazolopyrimidine mit coronarerweiternder Wirkung beschrieben, die zur Behandlung von Durchblutungsstörungen des Herzmuskels eingesetzt werden können.

In US 3,732,225 werden Pyrazolopyrimidine beschrieben, die eine entzündungshemmende und Blutzucker-senkende Wirkung haben.

In DE 2 408 906 werden Styrolpyrazolopyrimidine beschrieben, die als antimikrobielle und entzündungshemmende Mittel für die Behandlung von beispielsweise Ödem eingesetzt werden können.

Die WO03/093269 offenbart auf Seite 19, rechts oben und mit den Beispielen 13, Bsp.23, 25, 31 und 34 1,5-Dihydro-pyrazolo[3,4-d]pyrirnidin-4-one, die u.a. zur Verwendung als Herbizide, Nematizide oder zur Bekämpfung von Anthropoden im Tier aber nicht als Arzneimittel für den Menschen vorgeschlagen werden.

Die vorliegende Erfindung betrifft Verbindungen der Formel (I) bzw. deren Anwendung zur Behandlung und/oder Prophylaxe von Krankheiten gemäß den Patentansprüchen: 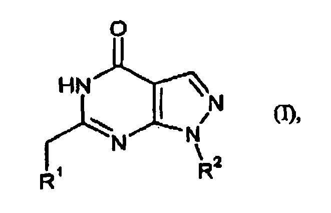 in welcher
- R¹: C₁-C₈-Alkyl, C₂-C₆-Alkenyl, C₂-C₆-Alkinyl oder C₃-C₈-Cycloalkyl,
wobei C₁-C₈-Alkyl gegebenenfalls mit Oxo substituiert ist, und
wobei C₁C₈-Alkyl, C₂-C₆-Alkenyl, C₂-C₆-Alkinyl und C₃-C₈-Cycloalkyl gegebenenfalls mit bis zu 3 Resten unabhängig voneinander ausgewählt aus der Gruppe C₁-C₆-Alkyl, C₁- C₆-Alkoxy, Hydroxycarbonyl, Cyano, Amino, Nitro, Hydroxy, C₁-C₆-Alkylamino, Halogen, Trifluormethyl, Trifluormethoxy, C₆-C₁₀-Arylcarbonylarnino, C₁-C₆-Alkyl- carbonylamino, C₁-C₆-Alkylaminocarbonyl, C₁-C₆-Alkoxycarbonyl, C₆-C₁₀-Arylamino- carbonyl, Heteroaryiaminocarbonyl, Heteroarylcarbonylamino, C₁-C₆-Alkylsulfonylamino, C₁-C₆-Alkytsulfonyl, C₁-C₆-Alkyithio substituiert sind,
wobei
C₁-C₆-Alkyl, C₁-C₆-Alkoxy, C₁-C₆-Alkylalnino, C₆-C₁₀-Arylcarbonylamino, C₁-C₆-Alkyl- carbonylamino, C₁-C₆-Alkylaminocarbonyl, C₁-C₆-Alkoxycarbonyl, C₆-C₁₀-Aryl- aminocarbonyl, Heteroarylaminocarbonyl, Heteroarylcarbonylamino, C₁-C₆- Alkylsulfonylamino, C₁-C₆-Alkylsulfonyl und C₁-C₆-Alkyithio gegebenenfalls mit ein bis drei Resten unabhängig voneinander ausgewählt aus der Gruppe Hydroxy, Cyano, Halogen, Trifluomethyl, Trifluormethoxy, Hydroxycarbonyl und einer Gruppe der Formel -NR³R⁴,
wobei
R³ und R⁴ unabhängig voneinander Wasserstoff oder C₁-C₆Alkyl, oder
R³ und R⁴ zusammen mit dem Stickstoffatom, an das sie gebunden sind, 5- bis 8- gliedriges Heterocyclyl bedeuten, substituiert sind,
- R²: Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆- Alkyl, C₁-C₆-Alkoxy, Hydroxycarbonyl, Cyano, Trifluormethyl, Trifluormethoxy, Amino, Nitro, Hydroxy, C₁-C₆-Alkylamino, Halogen, C₆-C₁₀-Arylcarbonylamino, C₁-C₆-Alkyl- carbonylamino, C₁-C₆-Alkylaminocarbonyl, C₁-C₆-Alkoxycarbonyl, C₆-C₁₀-Arylamino- carbonyl, Heteroarylaminocarbonyl, Heteroarylcarbonylamino, C₁-C₆-Alkylsulfonylamino, C₁-C₆-Alkylsulfonyl, C₁-C₆-Alkylthio substituiert sind,
wobei C₁-C₆-Alkyl, C₁C₆-Alkoxy, C₁-C₆-Alkylamino, C₆-C₁₀-Arylcarbonylamino, C₁-C₆- Alkylcarbonylamio, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl und C₁-C₆-Alkylthio gegebenenfalls mit ein bis drei Resten unabhängig voneinander ausgewählt aus der Gruppe Hydroxy, Cyano, Halogen, Trifluormethyl, Trifluormethoxy, Hydroxycarbonyl und einer Gruppe der Formel NR³R⁴,
wobei
R³ und R⁴ die oben angegebenen Bedeutungen aufweisen,
substituiert sind,
bedeuten, sowie deren Salze, Solvate und/oder Solvate der Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldüsopropylamin, Monoethanolamin, Diethanolamin, Triethanolamin, Dicyclohexylamin, Dimethylaminoethanol, Prokain, Dibenzylamin, N-Methylmorpholin, Dehydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
C₁-C₈-Alkyl steht für einen geradkettigen oder verzweigten Alkylrest mit 1 bis 8, bevorzugt 1 bis 6, besonders bevorzugt 1 bis 5 Kohlenstoffatomen. Bevorzugte Beispiele umfassen Methyl, Ethyl, n-Propyl, Isopropyl, 2-Butyl, 2-Pentyl und 3-Pentyl.

C₂-C₆-Alkenyl steht für einen geradkettigen oder verzweigten Alkenylrest mit 2 bis 6, bevorzugt 2 bis 4 und besonders bevorzugt mit 2 bis 3 Kohlenstoffatomen. Bevorzugte Beispiele umfassen Vinyl, Allyl, n-Prop-1-en-1-yl und n-But-2-en-1-yl.

-C2-C₆-Alkinyl steht für einen geradkettigen oder verzweigten Alkinylrest mit 2 bis 6, bevorzugt mit 2 bis 4 und besonders bevorzugt mit 2 bis 3 Kohlenstoffatomen. Bevorzugte Beispiele umfassen Ethinyl, n-Prop-1-in-2-yl, n-Prop-1-in-3-yl und n-But-2-in-1-yl.

C₁-C₆-Alkoxy steht für einen geradkettigen oder verzweigten Alkoxyrest mit 1 bis 6, bevorzugt 1 bis 4, besonders bevorzugt mit 1 bis 3 Kohlenstoffatomen. Bevorzugte Beispiele umfassen Methoxy, Ethoxy, n-Propoxy, Isopropoxy, tert.-Butoxy, n-Pentoxy und n-Hexoxy.

C₁-C₆-Alkoxycarbonyl steht für einen geradkettigen oder verzweigten Alkoxycarbonykest mit 1 bis 6, bevorzugt 1 bis 4 und besonders bevorzugt 1 bis 3 Kohlenstoffatomen. Bevorzugte Beispiele umfassen Methoxycarbonyl, Ethoxycarbonyl, n-Propoxycarbonyl, Isopropoxycarbonyl und tert.Butoxycarbonyl.

C₁-C₆-Alkylamino steht für einen geradkettigen oder verzweigten Mono- oder Dialkylaminorest mit 1 bis 6, bevorzugt 1 bis 4 und besonders bevorzugt mit 1 bis 3 Kohlenstoffatomen. Bevorzugte Beispiele umfassen Methylamino, Ethylamino, n-Propylamino, Isopropylamino, tert.Butylamino, n-Pentylamino und n-Hexylamino, Dimethylamino, Diethylamino, Di-n-propylamino, Diisopropylamino, Di-t-butylamino, Di-n-pentylamino, Di-n-hexylamino, Ethylmethylamino, Isopropylmethylamino, n-Butylethylamino und n-Hexyl-i-pentylamino.

C₁-C₆-Alkylcarbonylamino steht für einen über eine Amino-Gruppe verknüpften Alkylcarbonylrest, wobei der Alkylrest geradkettig oder verzweigt sein kann und 1 bis 6, bevorzugt 1 bis 4, und besonders bevorzugt 1 bis 3 Kohlenstoffatome enthält. Bevorzugte Beispiele umfassen Methylcarbonylamino, Ethylcarbonylamino, n-Propylcarbonylamino, Isopropylcarbonylamino, tert.Butylcarbonylamino, n-Pentylcarbonylamino und n-Hexylcarbonylamino.

C₁₋C₆-Mkylaminocarbonyl steht für einen über eine Carbonyl-Gruppe verknüpften Mono- oder Dialkylaminorest, wobei die Alkylreste gleich oder verschieden sein können, geradkettig oder verzweigt sind und jeweils 1 bis 6, bevorzugt 1 bis 4, und besonders bevorzugt 1 bis 3 Kohlenstoff atome enthalten. Bevorzugte Beispiele umfassen Methylaminocarbonyl, Ethylaminocarbonyl, n-Propylaminocarbonyl, Isopropylaminocarbonyl, tert.Butylaminocarbonyl, n-Pentylaminocarbonyl, n-Hexylaminocarbonyl, Dimethylaminocarbonyl, Diethylaminocarbonyl, Di-n-propylaminocarbonyl, Diisopropylaminocarbonyl, Di-t-butylaminocarbonyl, Di-n-pentylaminocarbonyl, Di-n-hexylaminocarbonyl, Ethylmethylaminocarbonyl, Isopropylmethylaminocarbonyl, n-Butylethylaminocarbonyl und n-Hexyl-i-pentylaminocarbonyl. Weiterhin können im Falle eines Dialkylaminorestes die beiden Alkylreste zusammen mit dem Stickstoffatom, an das sie gebunden sind, einen 5- bis 8-gliedriges Heterocyclyl bilden.

C₆ -C₁₀-Arylaminocarbonyl steht für einen über eine Carbonyl-Gruppe verknüpften Arylaminorest. Bevorzugte Beispiele umfassen Phenylaminocarbonyl und Naphthylaminocarbonyl.

C₆-C₁₀₋Arylcarbonylamino steht für einen über eine Amino-Gruppe verknüpften Arylaminorest. Bevorzugte Beispiele umfassen Phenylaminocarbonyl und Naphthylaminocarbonyl.

C₁-C₆₋Alkylsulfonylarnino steht für einen geradkettigen oder verzweigten Alkylsulfonylaminorest mit 1 bis 6, bevorzugt 1 bis 4 und besonders bevorzugt mit 1 bis 3 Kohlenstoffatomen. Bevorzugte Beispiele umfassen Methylsulfonylamino, Ethylsulfonylamino, n-Propylsulfonylamino, Isopropylsulfonylamino, tert.Butylsulfonylamino, n-Pentylsulfonylamino und n-Hexylsulfonylamino.

C₁-C₆-Alkylsulfonyl steht für einen geradkettigen oder verzweigten Alkylsulfonylrest mit 1 bis 6, bevorzugt 1 bis 4 und besonders bevorzugt mit 1 bis 3 Kohlenstoffatomen. Bevorzugte Beispiele umfassen Methylsulfonyl, Ethylsulfonyl, n-Propylsulfonyl, Isopropylsulfonyl, tert.Butylstilfonyl, n-Pentylsulfonyl und n-Hexylsulfonyl.

C₁-C₆-Alkylthio steht für einen geradkettigen oder verzweigten Alkylthiorest mit 1 bis 6, bevorzugt 1 bis 4 und besonders bevorzugt mit 1 bis 3 Kohlenstoffatomen. Bevorzugte Beispiele umfassen Methylthio, Ethylthio, n-Propylthio, Isopropylthio, tert.Butylthio, n-Pentylthio und n-Hexylthio.

Halogen steht für Fluor, Chlor, Brom und Iod. Bevorzugt sind Fluor, Chlor, Brom, besonders bevorzugt Fluor und Chlor.

Heteroaryl steht für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Stickstoffatom gebunden sein. Bevorzugte Beispiele umfassen Thienyl, Furyl, Pyrrolyl, Thiazolyl, Oxazolyl, Imidazolyl, Tetrazolyl, Pyridyl, N-Oxidopyridyl, Pyrimidinyl, Pyridazinyl, Indolyl, Indazolyl, Benzofuranyl, Benzothiophenyl, Chinolinyl und Isochinolinyl.

Heteromylanünocarbonyl steht für einen über eine Carbonyl-Gruppe verknüpften Heteroarylamino-Rest. Bevorzugte Beispiele umfassen Thienylaminocarbonyl, Furylaminocarbonyl, Pyrrolylaminocarbonyl, Thiazolylaminocarbonyl, Oxazolylaminocarbonyl, Imidazolylaminocarbonyl, Tetrazolylaminocarbonyl, Pyridylaminocarbonyl, Pyrimidinylarninocarbonyl, Pyridazinylaminocarbonyl, Indolylaminocarbonyl, Indazolylaminocarbonyl, Benzofuranylammocarbonyl, Benzothiophenylaminocarbonyl, Chinolinylaminocarbonyl und Isochinolinylaminocarbonyl.

Heteroarylcarbonylamino steht für einen über eine Araino-Gruppe verknüpften Heteroarylcarbonyl-Rest. Bevorzugte Beispiele umfassen Thienylcarbonylamino, Furylcarbonylamino, Pyaolylcarbonylamino, Thiazolylcarbonylamino, Oxazolylcarbonylamino, Imidazolylcarbonylamino, Tetrazolylcarbonylamino, Pyridylcarbonylamino, Pyrimidinylcarbonylamino, Pyridazinylcarbonylamino, Indelylcarbonylanüno, Indazolylcarbonylanüno, Benzofuranylcarbonylamino, Benzothiophenylcarbonylamino, Chinolinylcarbonylamino und Isochinolinylcarbonylamino.

3- bis 8-gliedriges Cyloalkyl steht für gesättigte und teilweise ungesättigte nicht-aromatische Cyloalkylreste mit 3 bis 8, bevorzugt 3 bis 6 und besonders bevorzugt 5 bis 6 Kohlenstoffatomen im Cyclus. Bevorzugte Beispiele umfassen Cyclopropyl, Cyclobutyl, Cyclopentyl, Cyclopentenyl, Cyclohexyl und Cyclohexenyl.

5- bis 8-gliedriges Heterocyclyl steht für einen mono- oder polycyclischen, heterocyclischen Rest mit 5 bis 8 Ringatomen und bis zu 3, vorzugsweise 2 Heteroatomen bzw. Heterogruppen aus der Reihe N, O, S, SO, SO₂. Mono- oder bicyclisches Heterocyclyl ist bevorzugt. Besonders bevorzugt ist monocyclisches Heterocyclyl. Als Heteroatome sind N und O bevorzugt. Die Heterocyclyl-Reste können gesättigt oder teilweise ungesättigt sein. Gesättigte Heterocyclyl-Reste sind bevorzugt. Besonders bevorzugt sind 5- bis 7-gliedrige Heterocyclylreste. Bevorzugte Beispiele umfassen Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl.

Wenn Reste in den erfindungsgemäßen Verbindungen gegebenenfalls substituiert sind, ist, soweit nicht anders spezifiziert, eine Substitution mit bis zu drei gleichen oder verschiedenen Substituenten bevorzugt.

Die erfindungsgemäßen Verbindungen können auch als Tautomere vorliegen, wie im Folgenden beispielhaft gezeigt wird:

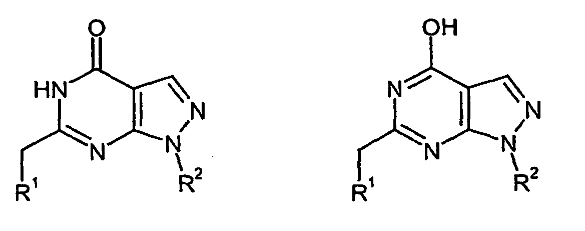

Eine weitere Ausführungsform der Erfindung betrifft Verbindungen der Formel (I), in welcher
- R¹: C₁-C₈-Alkyl, C₂-C₆-Alkenyl, C₂-C₆-Alldnyl oder C₃-C₈-Cycloalkyl, welche gegebenenfalls mit bis zu 3 Resten unabhängig voneinander ausgewählt aus der Gruppe C₁-C₆-Alkyl, C₁- C₆-Alkoxy, Hydroxycarbonyl, Cyano, Amino, Nitro, Hydroxy, C₁-C₆-Alkylamino, Halogen, C₆-C₁₀-Arylcarbonylamino, C₁-C₆-Alkylcarbonylamino, C₁-C₆-Alkylamino- carbonyl, C₁-C₆-Akoxycarbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl, C₁-C₆-Alkyl- thio substituiert sind,
wobei
C₁-C₆-Alkyl, C₁-C₆-Alkoxy, C₁-C₆-Alkylamino, C₆-C₁₀-Arylcarbonylamino, C₁-C₆- Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxy- carbonyl und einer Gruppe der Formel NR³R⁴,
wobei
R³ und R⁴ unabhängig voneinander Wasserstoff oder C₁-C₆-Alkyl, oder
R³ und R⁴ zusammen mit dem Stickstoffatom, an das sie gebunden sind, 5- bis 8- gliedriges Heterocyclyl bedeuten, substituiert sind,
- R²: Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆- Alkyl, C₁-C₆-Alkoxy, Hydroxycarbonyl, Cyano, Trifluormethyl, Amino, Nitro, Hydroxy, C₁-C₆-Alkylamino, Halogen, C₆-C₁₀-Arylcarbonylamino, C₁-C₆-Alkylcarbonylamino, C₁- C₆-Alkylanvnocarbonyl, C₁-C₆-Alkoxycarbonyl, C₆-C₁₀-Arylaminocarbonyl, Heteroaryl- aminocarbonyl, Heteroarylcarbonylamino, C₁-C₆-Alkylsulfonylamino, C₁-C₆-Alkyl- sulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆- Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Akylsulfonyl und C₁-C₆-Alkylthio gegebenenfalls mit einem Rest ausgewählt aus der Gruppe Hydroxy, Cyano, Halogen, Hydroxy- carbonyl und einer Gruppe der Formel -NR³R⁴,
wobei
R³ und R⁴ die oben angegebenen Bedeutungen aufweisen,
substituiert sind,
bedeuten, sowie deren Salze, Solvate und/oder Solvate der Salze.

Eine weitere Ausführungsform der Erfindung betrifft Verbindungen der Formel (I),
in welcher
- R¹: C₁-C₅-Alkyl oder C₃-C₆-Cycloalkyl, welche gegebenenfalls mit bis zu 3 Resten unabhängig voneinander ausgewählt aus der Gruppe C₁-C₄-Alkyl, C₁-C₄-Alkoxy, Trifluormethyl, Hydroxycarbonyl, Cyano, Amino, Hydroxy, C₁-C₄-Alkylamino, Fluor, Chlor, Brom, C₆- C₁₀-Arylcarbonylamino, C₁-C₄-Alkylcarbonylamino, C₁-C₄-Akylaminocarbonyl, C₁-C₄- Alkoxycarbonyl, C₆-C₁₀-Arylaminocarbonyl, Heteroarylaminocarbonyl, Heteroarylcarbonylamino, C₁-C₄-Alkylsulfonylamino, C₁-C₄-Alkylsulfonyl, C₁-C₄- Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel NR³R⁴,
wobei
R³ und R⁴ unabhängig voneinander Wasserstoff oder C₁-C₄-Alkyl, oder
R³ und R⁴ zusammen mit dem Stickstoffatom, an das sie gebunden sind, 5- bis 6-gliedriges Heterocyclyl bedeuten, substituiert sind,
- R²: Phenyl, Pyrimidyl, N-Oxidopyridyl oder Pyridyl, wobei Phenyl mit 1 bis 3 Resten und Pyrimidyl, N-Oxidopyridyl und Pyridyl gegebenenfalls mit 1 bis 3 Resten jeweils unabhängig voneinander ausgewählt aus der Gruppe C₁-C₄-Alkyl, C₁-C₄-Alkoxy, Hydroxy- carbonyl, Cyano, Trifluormethyl, Amino, Hydroxy, C₁-C₄-Alkylamino, Fluor, Chlor, Brom, C₆-C₁₀-Arylcarbonylamino, C₁-C₄-Alkylcarbonylamino, C₁-C₄-Alkylaminocarbonyl, C₁-C₄-Alkoxycarbonyl, C₆-C₁₀-Arylaminocarbonyl, Heteroarylaminocarbonyl, Heteroaryl- carbonylamino, C₁-C₄-Alkylsulfonylamino, C₁-C₄-Alkylsulfonyl, C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel NR³R⁴,
wobei
R³ und R⁴ die oben angegebenen Bedeutungen aufweisen,
substituiert sind,
bedeutet, sowie deren Salze, Solvate und/oder Solvate der Salze.

Eine weitere Ausführungsform der Erfindung betrifft Verbindungen der Formel (I),
in welcher
- R¹: die oben angegebenen Bedeutungen aufweist, und
- R²: Phenyl, N-Oxidopyridyl oder Pyridyl, wobei Phenyl mit 1 bis 3 Resten und Pyridyl und N- Oxidopyridyl gegebenenfalls mit 1 bis 3 Resten jeweils unabhängig voneinander ausge- wählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze.

Eine weitere Ausführungsform der Erfindung betrifft Verbindungen der Formel (I),
in welcher
- R¹: C₁-C₅-Alkyl oder C₅-C₆-Cycloalkyl, welche gegebenenfalls mit bis zu 3 Resten unabhängig voneinander ausgewählt aus der Gruppe C₁-C₄-Alkyl, Fluor, Trifluormethyl, Hydroxy, Phenylcarbonylamino, C₁-C₄-Alkylcarbonylamino, C₁-C₄-Alkylaminocarbonyl oder Phenylaminocarbonyl substituiert sind, und
- R²: Phenyl, N-Oxidopyridyl oder Pyridyl, wobei Phenyl mit 1 bis 3 Resten und Pyridyl und N- Oxidopyridyl gegebenenfalls mit 1 bis 3 Resten jeweils unabhängig voneinander ausge- wählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze.

Eine weitere Ausführungsform der Erfindung betrifft Verbindungen der Formel (I),
in welcher
- R¹: C₁-C₅-Alkyl oder C₅-C₆-Cycloalkyl, welche gegebenenfalls mit bis zu 3 Resten unabhängig voneinander ausgewählt aus der Gruppe C₁-C₄-Alkyl, Fluor, Trifluormethyl, Hydroxy, Phenylcarbonylamino, C,-C₄-Alkylcarbonylamino, C₁-C₄-Alkylaminocarbonyl oder Phenylaminocarbonyl substituiert sind, und
- R²: Phenyl, N-Oxidopyridyl oder Pyridyl, wobei Phenyl mit einem Rest und Pyridyl und N- Oxidopyridyl gegebenenfalls mit einem Rest jeweils unabhängig voneinander ausgewählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze.

Außerdem wurde ein Verfahren zur Herstellung der erfindungsgemäßen Verbindungen der Formel (I) gefunden, dadurch gekennzeichnet, dass man entweder
[A] Verbindungen der Formel 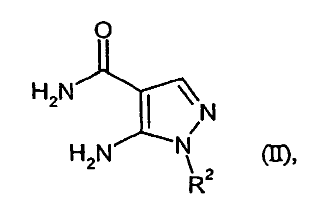 in welcher
   - R²: die oben angegebenen Bedeutungen hat,
   durch Umsetzung mit einer Verbindung der Formel 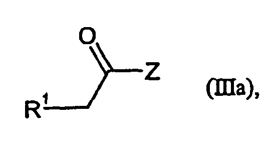 in welcher
   - R¹: die oben angegebenen Bedeutungen hat,
   und
   - Z: für Chlor oder Brom steht,
   in einem inerten Lösemittel und in Anwesenheit einer Base zunächst in Verbindungen der Formel 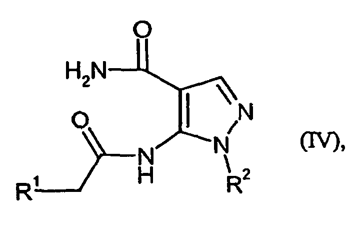 in welcher
   R¹ und R² die oben angegebenen Bedeutungen haben,
   überführt, dann in einem inerten Lösemittel in Gegenwart einer Base zu Verbindungen der Formel (I) cyclisiert,
   oder
[B] Verbindungen der Formel (II) unter direkter Cyclisierung zu (I) mit einer Verbindung der Formel 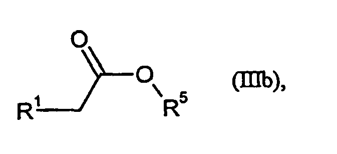 in welcher
   - R¹: die oben angegebenen Bedeutungen hat,
   und
   - R⁵: für Methyl oder Ethyl steht,
   in einem inerten Lösemittel und in Anwesenheit einer Base umsetzt,
   oder
[C] Verbindungen der Formel 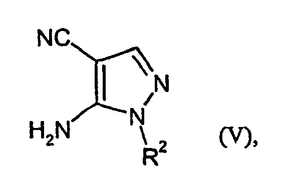 in welcher
   - R²: die oben angegebenen Bedeutungen hat,
   zunächst durch Umsetzung mit einer Verbindung der Formel (IIIa) in einem inerten Lösemittel und in Anwesenheit einer Base in Verbindungen der Formel 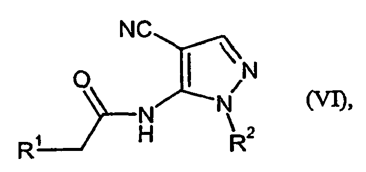 in welcher
   R¹ und R² die oben angegebenen Bedeutungen haben,
   überführt,
   und diese in einem zweiten Schritt in einem inerten Lösemittel und in Anwesenheit einer Base und eines Oxidationsmittels zu (I) cyclisiert,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Für den ersten Schritt des Verfahrens [A] und des Verfahrens [C] eignen sich inerte organische Lösemittel, die sich unter den Reaktionsbedingungen nicht verändern. Hierzu gehören bevorzugt Ether wie beispielsweise Diethylether, Dioxan, Tetrahydrofuran oder Glykoldimethylether, oder Toluol oder Pyridin. Ebenso ist es möglich, Gemische der genannten Lösemittel einzusetzen. Besonders bevorzugt sind Tetrahydrofuran, Toluol oder Pyridin.

Als Basen eignen sich im allgemeinen Alkalihydride, wie beispielsweise Natriumhydrid, oder cyclische Amine, wie beispielsweise Piperidin, Pyridin, Dimethylaminopyridin (DMAP), oder C₁-C₄-Alkylamine, wie beispielsweise Triethylamin. Bevorzugt sind Natriumhydrid, Pyridin und/oder Dimethylaminopyridin.

Die Base wird im allgemeinen in einer Menge von 1 mol bis 4 mol, bevorzugt von 1.2 mol bis 3 mol, jeweils bezogen auf 1 mol der Verbindungen der allgemeinen Formel (II) bzw. (V), eingesetzt.

In einer Variante wird die Umsetzung in Pyridin, dem eine katalytische Menge DMAP zugesetzt wird, durchgeführt. Gegebenenfalls kann noch Toluol zugefügt werden.

Die Reaktionstemperatur kann im allgemeinen in einem größeren Bereich variiert werden. Im allgemeinen arbeitet man in einem Bereich von 20°C bis +200°C, bevorzugt von 0°C bis +100°C.

Als Lösemittel für die Cyclisierung im zweiten Schritt der Verfahren [A] und [C] eignen sich die üblichen organischen Lösemittel. Hierzu gehören bevorzugt Alkohole wie Methanol, Ethanol, Propanol, Isopropanol, n-Butanol oder tert.-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol oder tert.-Butanol verwendet. Ebenso ist es möglich, Gemische der genannten Lösemittel einzusetzen.

Als Basen für die Cyclisierung im zweiten Schritt der Verfahren [A] und [C] eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.-butanolat. Besonders bevorzugt sind Kaliumcarbonat, Natriumhydroxid und Kalium-tert.-butanolat.

Bei der Durchführung der Cyclisierung wird die Base im allgemeinen in einer Menge von 2 mol bis 6 mol, bevorzugt von 3 mol bis 5 mol, jeweils bezogen auf 1 mol der Verbindungen der allgemeinen Formel (IV) bzw. (VI), eingesetzt.

Als Oxidationsmittel für die Cyclisierung im zweiten Schritt des Verfahrens [C] eignen sich beispielsweise Wasserstoffperoxid oder Natriumborat. Bevorzugt ist Wasserstoffperoxid.

Die Cyclisierung in den Verfahren [A], [B] und [C] wird im allgemeinen in einem Temperaturbereich von 0°C bis +160°C, bevorzugt bei der Siedetemperatur des jeweiligen Lösemittels durchgeführt. Die Cyclisierung wird im allgemeinen bei Normaldruck durchgeführt. Es ist aber auch möglich, das Verfahren bei überdruck oder bei Unterdruck durchzuführen (z.B. in einem Bereich von 0.5 bis 5 bar).

Als Lösemittel für das Verfahren [B] eignen sich die oben für den zweiten Schritt der Verfahren [A] und [C] aufgeführten Alkohole, wobei Ethanol bevorzugt ist.

Als Basen für das Verfahren [B] eignen sich Alkalihydride, wie beispielsweise Natrium- oder Kaliumhydrid, oder Alkalialkoholate, wie beispielsweise Natriummethanolat, -ethanolat, -isopropylat oder Kalium-tert.-butylat. Bevorzugt ist Natriumhydrid.

Die Base wird in einer Menge von 2 mol bis 8 mol, bevorzugt von 3 mol bis 6 mol, jeweils bezogen auf 1 mol der Verbindungen der Formel (II), eingesetzt.

Die Verbindungen der Formel (II) sind bekannt oder können beispielsweise hergestellt werden, indem man zunächst Ethoxymethylenmalonsäuredinitril mit Hydrazin-Derivaten der Formel (VII)

R²-NH-NH₂ (VII),

in welcher
- R²: die oben angegebenen Bedeutungen hat,
in einem inerten Lösemittel zu den Pyrazolnitrilen der Formel (V) kondensiert und diese dann mit einem der oben aufgeführten Oxidationsmittel, vorzugsweise Wasserstoffperoxid, in Anwesenheit von Ammoniak umsetzt [vgl. z.B. A. Miyashita et al., Heterocycles 1990, 31, 1309ff].

Die Verbindungen der Formeln (IIIa), (IIIb) und (VII) sind kommerziell erhältlich, literaturbekannt oder können in Analogie zu literaturbekannten Verfahren hergestellt werden.

Das erfindungsgemäße Verfahren kann durch das folgendes Formelschema beispielhaft erläutert werden:

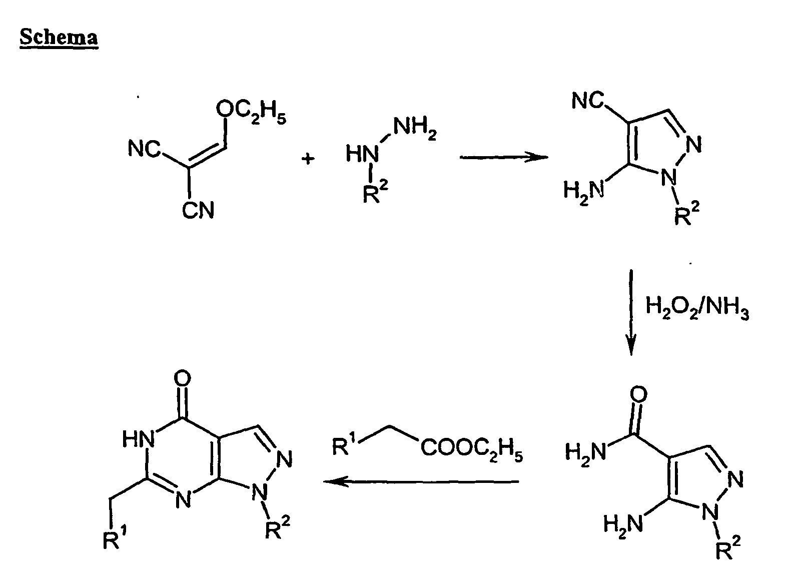

Weitere Verfahren zur Herstellung von Pyrazolo[3,4-d]pyrimidin-4-onen sind bekannt und können ebenfalls zur Synthese der erfindungsgemäßen Verbindungen eingesetzt werden (siehe zum Beispiel: P. Schmidt et al., Helvetica Chimica Acta 1962,189, 1620ff.).

Die erfindungsgemäßen Verbindungen zeigen ein nicht vorhersehbares, wertvolles pharmakologisches und pharmakokinetisches Wirkspektrum. Sie zeichnen sich insbesondere durch eine Inhibition von PDE9A aus.

Sie eignen sich daher zur Verwendung als Arzneimittel zur Behandlung und/oder Prophylaxe von Krankheiten bei Menschen und Tieren.

Der Begriff "Behandlung" im Rahmen der vorliegenden Erfindung schließt die Prophylaxe ein.

Überraschenderweise wurde gefunden, dass die erfindungsgemäßen Verbindungen zur Herstellung von Arzneimitteln zur Verbesserung der Wahrnehmung, Konzentrationsleistung, Lernleistung oder Gedächtnisleistung geeignet sind.

Die erfindungsgemäßen Verbindungen können aufgrund ihrer pharmakologischen und pharmakokinetischen Eigenschaften allein oder in Kombination mit anderen Arzneimitteln zur Verbesserung von Wahrnehmung, Konzentrationsleistung, Lern- und/oder Gedächtnisleistung eingesetzt werden.

Besonders eignen sich die erfindungsgemäßen Verbindungen zur Verbesserung der Wahrnehmung, Konzentrationsleistung, Lemleistung, oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia''), post-traumatische Demenz, allgemeine Konzentrationsstörungen, Konzentrationsstörungen in Kindern mit Lern- und Gedächtnisproblemen, Alzheimer'sche Kranhheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die *in vitro*-Wirkung der erfindungsgemäßen Verbindungen kann mit folgenden biologischen Assays gezeigt werden:

### PDE-Inhibition

Rekombinante PDE1C (GenBank/EMBL Accession Number: NM_005020, Loughney et al. J. Biol. Chem. 1996 271, 796-806), PDE2A (GenBank/EMBL Accession Number: NM_002599, Rosman et al. Gene 1997 191, 89-95), PDE3B (GenBank/EMBL Accession Number: NM_000922, Miki et al. Genomics 1996, 36, 476-485), PDE4B (GenBank/EMBL Accession Number: NM_002600, Obernolte et al. Gene. 1993, 129, 239-247), PDE5A (GenBank/EMBL Accession Number: NM_001083, Loughney et al. Gene 1998, 216, 139-147), PDE7B (GenBank/EMBL Accession Number: NM_018945, Hetman et al. Proc. Natl. Acad. Sci. USA. 2000, 97, 472-476), PDE8A (GenBank/EMBL Accession Number: AF_056490, Fisher et al. Biochem. Biophys. Res. Commun. 1998 246, 570-577), PDE9A (Fisher et al., J. Biol. Chem, 1998, 273 (25): 15559-15564), PDE10A (GenBank/EMBL Accession Number: NM_06661, Fujishige et al. J Biol Chem. 1999, 274, 18438-45), PDE11A (GenBank/EMBL Accession Number: NM_016953, Fawcett et al. Proc. Natl. Acad. Sci. 2000, 97, 3702-3707) wurden mit Hilfe des pFASTBAC Baculovirus-Expressionssystems (GibcoBRL) in Sf9-Zellen exprimiert.

Die Testsubstanzen werden zur Bestimmung ihrer *in vitro* Wirkung an PDE 9A in 100 % DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µl der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A-Präparates hinzugefügt. Die Verdünnung des PDE9A-Präparates wird so gewählt, dass während der späteren Inkubation weniger als 70% des Substrates umgesetzt wird (typische Verdünnung: 1:10000; Verdünnungspuffer: 50 mM Tris/HCl pH 7.5, 8.3 mM MgCl₂, 1.7 mM EDTA, 0.2% BSA). Das Substrat, [8-³H] guanosine 3',5'-cyclic phosphate (1 µCi/µL; Amersham Pharmacia Biotech., Piscataway, NJ) wird 1:2000 mit Assaypuffer (50 mM Tris/HCl pH 7.5, 8.3 mM MgCl₂, 1.7 mM EDTA) auf eine Konzentration von 0.0005 µC/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDE9A-Inhibitors (z.B. der Inhibitor aus Herstellbeispiel 1, 10 µM Endkonzentration) gestoppt. Direkt im Anschluß werden 25 µL einer Suspension mit 18 mg/mL Yttrium Scintillation Proximity Beads (Amersham Pharmacia Biotech., Piscataway, NJ) hinzugefügt. Die Mikrotiterplatten werden mit einer Folie versiegelt und für 60 min bei Raumtemperatur stehen gelassen. Anschließend werden die Platten für 30 s pro Vertiefung in einem Microbeta-Szintillationzähler (Wallac Inc., Atlanta, GA) vermessen. IC₅₀-Werte werden anhand der graphischen Auftragung der Substanzkonzentration gegen die prozentuale Inhibition bestimmt.

Repräsentative Beispiele für die inhibierende Wirkung der erfindungsgemäßen Verbindungen an PDE9A werden anhand der IC₅₀-Werte in Tabelle 1 aufgeführt:

**Tabelle 1**

| **Beispiel** | **IC₅₀-Wert [nM]** |
|---|---|
| 2 | 38 |
| 5 | 12 |
| 11 | 5 |
| 34 | 12 |
| 37-1 | 60 |
| 38 | 13 |
| 39-1 | 30 |

Die *in vitro* Wirkung von Testsubstanzen an rekombinanter PDE3B, PDE4B, PDE7B, PDE8A, PDE10A und PDE11A wird nach dem oben für PDE 9A beschriebenen Testprotokoll mit folgenden Anpassungen bestimmt: Als Substrat wird [5',8-³H] adenosine 3',5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird im Anschluß an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepaßt: Bei PDE1C werden zusätzlich Calmodulin 10⁻⁷ M und CaCl₂ 3 mM zum Reaktionsansatz gegeben. PDE2A wird im Test durch Zugabe von cGMP 1 µM stimuliert und mit einer BSA-Konzentration von 0.01% getestet. Für PDE1C und PDE2A wird als Substrat [5',8-³H] adenosine 3',5'-cyclic phosphate (1 µCi/µL; Amersham Pharmacia Biotech., Piscataway, NJ), für PDE5A [8-³H] guanosine 3',5'-cyclic phosphate (1 µCi/µL; Amersham Pharmacia Biotech., Piscataway, NJ) eingesetzt.

### Langzeitpotenzierung

Langzeitpotenzierung wird als ein zelluläres Korrelat für Lern- und Gedächtnisvorgänge angesehen. Zur Bestimmung, ob PDE 9-Inhibition einen Einfluss auf Langzeitpotenzierung hat, kann folgende Methode angewandt werden:
Rattenhippokampi werden in einem Winkel von etwa 70 Grad im Verhältnis zur Schnittklinge platziert (Chopper). In Abständen von 400 µm wird der Hippokampus zerschnitten. Die Schnitte werden mit Hilfe eines sehr weichen, stark benetzten Pinsels (Marderhaar) von der Klinge genommen und in ein Glasgefäß mit carbogenisierter gekühlter Nährlösung (124 mM NaCl, 4.9 mM KCl, 1.3 mM MgSO₄ x 7 H₂O, 2.5 mM CaCl₂ wasserfrei, 1.2 mM KH₂PO₄, 25.6 mM NaHCO₃, 10 mM Glucose, pH 7.4) überführt. Während der Messung befinden sich die Schnitte in einer temperierten Kammer unter einem Flüssigkeitsspiegel von 1-3 mm Höhe. Die Durchflussrate beträgt 2.5 ml/min. Die Vorbegasung erfolgt unter geringem Überdruck (etwa 1 atm) sowie über eine Mikrokanüle in der Vorkammer. Die Schnittkammer ist mit der Vorkammer so verbunden, dass eine Minizirkulation aufrechterhalten werden kann. Als Antrieb der Minizirkulation wird das durch die Mikrokanüle ausströmende Carbogen eingesetzt. Die frisch präparierten Hippokampusschnitte werden mindestens 1 Stunde bei 33°C in der Schnittkammer adaptiert.

Die Reizstärke wird so gewählt, dass die fokalen exzitatorischen postsynaptischen Potentiale (fEPSP) 30% des maximalen exzitatorischen postsynaptischen Potentials (EPSP) betragen. Mit Hilfe einer monopolaren Stimulationselektrode, die aus lackiertem Edelstahl besteht, und eines stromkonstanten, biphasischen Reizgenerators (AM-Systems 2100) werden lokal die Schaffer-Kollateralen erregt (Spannung: 1-5 V, Impulsbreite einer Polarität 0.1 ms, Gesamtimpuls 0.2 ms). Mit Hilfe von Glaselektroden (Borosilikatglas mit Filament, 1-5 MOhm, Durchmesser: 1.5 mm, Spitzendurchmesser: 3-20 µm), die mit normaler Nährlösung gefüllt sind, werden aus dem Stratum radiatum die exzitatorischen postsynaptischen Potentiale (fEPSP) registriert. Die Messung der Feldpotentiale geschieht gegenüber einer chlorierten Referenzelektrode aus Silber, die sich am Rande der Schnittkammer befindet, mit Hilfe eines Gleichspannungsverstärkers. Das Filtern der Feldpotentiale erfolgt über einen Low-Pass Filter (5 kHz). Für die statistische Analyse der Experimente wird der Anstieg (slope) der fEPSPs (fEPSP-Anstieg) ermittelt. Die Aufnahme, Analyse und Steuerung des Experimentes erfolgt mit Hilfe eines Softwareprogrammes (PWIN), welches in der Abteilung Neurophysiologie entwickelt worden ist. Die Mittelwertbildung der fEPSP-Anstiegswerte zu den jeweiligen Zeitpunkten und die Konstruktion der Diagramme erfolgt mit Hilfe der Software EXCEL, wobei ein entsprechendes Makro die Aufnahme der Daten automatisiert.

Superfusion der Hippokampusschnitte mit einer 10 µM Lösung der erfindungsgemäßen Verbindungen führt zu einer signifikanten Steigerung der LTP.

Die in *vivo*-Wirkung der erfindungsgemäßen Verbindungen kann zum Beispiel wie folgt gezeigt werden:

### Sozialer Wiedererkennungstest

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessernden Wirkung der erfindungsgemäßen Verbindungen.

Adulte Ratten, die in Gruppen gehalten werden, werden 30 min vor Testbeginn einzeln in Testkäfige gesetzt. Vier min vor Testbeginn wird das Testtier in eine Beobachtungsbox gebracht. Nach dieser Adaptationszeit wird ein juveniles Tier zu dem Testtier gesetzt und 2 min lang die absolute Zeit gemessen, die das adulte Tier das Junge inspiziert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wird das Juvenile herausgenommen, das Adulte mit einer erfindungsgemäßen Verbindung oder Vehikel behandelt und anschließend in seinen Heimkäfig zurückgesetzt. Nach einer Retentionszeit von 24 Stunden wird der Test wiederholt (Trial 2). Eine verringerte Soziale Interaktionszeit im Vergleich zu Trial 1 zeigt an, dass die adulte Ratte sich an das Jungtier erinnert.

Die adulten Tiere werden entweder in einem festgelegten Zeitabstand (z.B. 1 Stunde) vor Trial 1 oder direkt im Anschluss an Trial 1 entweder mit Vehikel (10% Ethanol, 20% Solutol, 70% physiologische Kochsalzlösung) oder 0.1 mg/kg, 0.3 mg/kg, 1.0 mg/kg bzw. 3.0 mg/kg erfindungsgemäßer Verbindung, gelöst in 10% Ethanol, 20% Solutol, 70% physiologische Kochsalzlösung intraperitoneal injiziert. Vehikel-behandelte Ratten zeigen keine Reduktion der sozialen Interaktionszeit in Trial 2 verglichen mit Trial 1. Sie haben folglich vergessen, dass sie schon einmal Kontakt mit dem Jungtier hatten. Überraschenderweise ist die soziale Interaktionszeit im zweiten Durchgang nach Behandlung mit den erfindungsgemäßen Verbindungen signifikant gegenüber den Vehikel-behandelten reduziert. Dies bedeutet, dass die substanzbehandelten Ratten sich an das juvenile Tier erinnert haben und somit die erfindungsgemäßen Verbindungen eine verbessernde Wirkung auf Lernen und Gedächtnis aufweisen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im allgemeinen hat es sich als vorteilhaft erwiesen, bei parenteraler Applikation pro Tag Mengen von etwa 0.001 bis 10 mg/kg Körpergewicht zur Erzielung wirksamer Ergebnisse zu verabreichen. Bei oraler Applikation beträgt die Menge pro Tag etwa 0.005 bis 3 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile, Lösungsmittelverhältnisse, Verdünnungsverhältnisse und Konzentrationsangaben von flüssig/flüssig-Lösungen beziehen sich jeweils auf das Volumen.

### Abkürzungen:

- BSA: Rinderserumalbumin
- DC: Dünnschichtchromatographie
- DCI: direkte chemische Ionisation (bei MS)
- DMSO: Dimethylsulfoxid
- d.Th.: der Theorie (bei Ausbeute)
- EDTA: Ethylendiamintetraessigsäure
- ESI: Elektrospray-Ionisation (bei MS)
- Fp.: Schmelzpunkt
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- Rₜ: Retentionszeit (bei HPLC)
- Tris: Tris-(hydroxymethyl)-aminomethan

### LC-MS-Methoden:

### Methode 1

Gerätetyp MS: Micromass ZQ; Gerätetyp HPLC: TSP P4000, TSP AS300, TSP UV3000; Säule: Grom-Sil 120 ODS-4 HE, 50 x 2 mm, 3.0 µm; Eluent A: Wasser + 250 µl 50%-ige Ameisensäure / 1, Eluent B: Acetonitril + 250 µl 50%-ige Ameisensäure / l; Gradient: 0.0 min 0% B → 0.2 min 0% B → 2.9 min 70% B → 3.1 min 90% B → 4.5 min 90% B; Ofen: 50°C; Fluss: 0.8 ml/min; UV-Detektion: 210 nm.

### Methode 2

Instrument: Micromass Platform LCZ mit HPLC Agilent Serie 1100; Säule: Grom-Sil 120 ODS-4 HE, 50 mm x 2.0 mm, 3 µm; Eluent A: 1 l Wasser + 1 ml 50%-ige Ameisensäure, Eluent B: 1 l Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 3

Gerätetyp MS: Micromass ZQ; Gerätetyp HPLC: Waters Alliance 2790; Säule: Grom-Sil 120 ODS-4 HE, 50 x 2 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B → 5.5 min 90% B; Ofen: 45°C; Fluss: 0.0 min 0.75 ml/min → 4.5 min 0.75 ml/min → 5.5 min 1.25 ml/min; UV-Detektion: 210 nm.

### Methode 4

Instrument: Micromass Quattro LCZ, mit HPLC Agilent Serie 1100; Säule: Grom-Sil 120 ODS-4 HE, 50 mm x 2.0 mm, 3 µm; Eluent A: 1 l Wasser + 1 ml 50%-ige Ameisensäure, Eluent B: 1 l Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Ausgangsverbindungen:

### Beispiel 1A

### 5-Amino-1-(2,6-dimethylphenyl)-1H-pyrazol-4-carbonitril

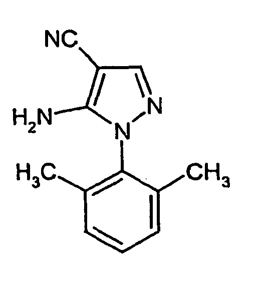

3.0 g (17.3 mmol) 2,6-Dimethylphenylhydrazin-Hydrochlorid werden mit 2.1 g (17.3 mmol) Ethoxymethylenmalonsäuredinitril in 40 ml Ethanol suspendiert und mit 7.3 ml (52.1 mmol) Triethylamin versetzt. Die Reaktionsmischung wird 3 h zum Rückfluss erhitzt, wobei sich eine klare Lösung bildet. Nach Abkühlen auf Raumtemperatur wird mit Diethylether versetzt. Das dabei ausfallende Triethylammoniumchlorid wird abfiltriert. Das Lösungsmittel wird im Vakuum entfernt und der Rückstand mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Man erhält 2.3 g (62% d.Th.) des Produktes als gelbe Kristalle.

LC-MS (Methode 1): Rₜ = 2.77 min.

MS (ESI pos): m/z = 213 (M+H)⁺.

### Beispiel 2A

### 5-Amino-1-(2,3-dimethylphenyl)-1H-pyrazol-4-carbonitril

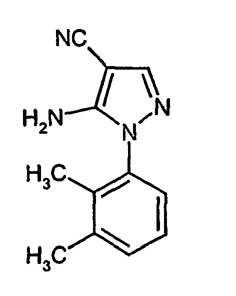

Analog zur Herstellung von Beispiel 1A werden ausgehend von 3 g (17.4 mmol) 2,3-Dimethylphenylhydrazin-Hydrochlorid, 2.12 g (17.4 mmol) Ethoxymethylenmalonsäuredinitril und 7.3 ml (52.1 mmol) Triethylamin 2.08 g (56% d.Th.) des gewünschten Produktes erhalten.

LC-MS (Methode 1): Rₜ = 2.79 min.

MS (ESI pos): m/z = 213 (M+H)⁺.

### Beispiel 3A

### 5-Amino-1-(4-methylphenyl)-1H-pyrazol-4-carbonitril

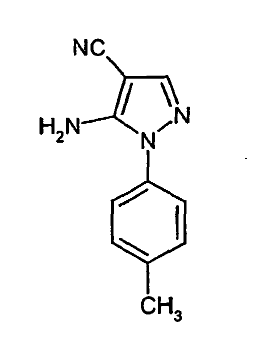

Analog zur Herstellung von Beispiel 1A werden ausgehend von 3 g (18.9 mmol) 4-Methylphenylhydrazin-Hydrochlorid, 2.3 g (18.9 mmol) Ethoxymethylenmalonsäuredinitril und 7.9 ml (56.7 mmol) Triethylamin 2.16 g (57% d.Th.) des gewünschten Produktes erhalten.

LC-MS (Methode 2): Rₜ = 3.0 min.

MS (ESI pos): m/z = 199 (M+H)⁺.

### Beispiel 4A

### 5-Amino-1-(2,6-dichlorphenyl)-1H-pyrazol-4-carbonitril

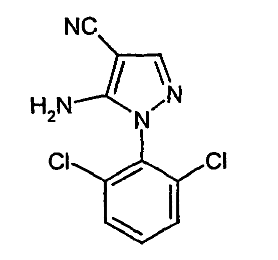

Analog zur Herstellung von Beispiel 1A werden ausgehend von 3 g (14.1 mmol) 2,6-Dichlorphenylhydrazin-Hydrochlorid, 1.7 g (14.1 mmol) Ethoxymethylenmalonsäuredinitril und 5.8 ml (42.2 mmol) Triethylamin nach säulenchromatographischer Reinigung (Laufmittel Dichlormethan/Methanol 98:2) 2.9 g (83% d.Th.) des gewünschten Produktes erhalten.

LC-MS (Methode 3): Rₜ = 2.8 min.

MS (ESI pos): m/z = 253 (M+H)⁺

¹H-NMR (300 MHz, DMSO-d₆); δ = 6.82 (s, 2H), 7.59 (m, 2H), 7.69 (m, 1H), 7.80 (s, 1H) ppm.

### Beispiel 5A

### 5-Amino-1-(2,5-dichlorphenyl)-1H-pyrazol-4-carbonitril

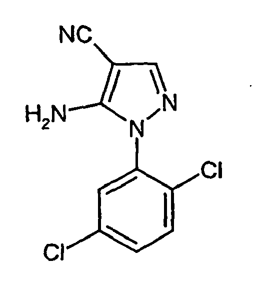

Analog zur Herstellung von Beispiel 1A werden ausgehend von 3 g (16.9 mmol) 2,5-Dichlorphenylhydrazin, 2.0 g (16.9 mmol) Ethoxymethylenmalonsäuredinitril und 7.1 ml (50.8 mmol) Triethylamin 2.2 g (51% d.Th.) des gewünschten Produktes erhalten.

LC-MS (Methode 2): Rₜ = 3.2 min.

MS (ESI pos): m/z = 253 (M+H)⁺.

### Beispiel 6A

### 5-Amino-1-(2-nitrophenyl)-1H-pyrazol-4-carbonitril

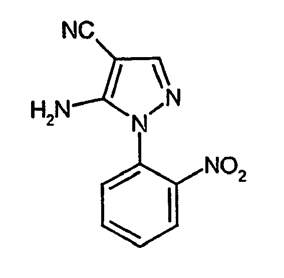

Analog zur Herstellung von Beispiel 1A werden ausgehend von 3 g (15.8 mmol) 2-Nitrophenylhydrazin-Hydrochlorid, 1.93 g (16.9 mmol) Ethoxymethylenmalonsäuredinitril und 6.6 ml (47.6 mmol) Triethylamin 1.9 g (53% d.Th.) des gewünschten Produktes erhalten.

LC-MS (Methode 2): Rₜ = 2.8 min.

MS (ESI pos): m/z = 230 (M+H)⁺

¹H-NMR (300 MHz, DMSO-d₆): δ = 6.87 (s, 2H), 7.72 (m, 1H), 7.77 (s, 1H), 7.78 (m, 1H), 7.88 (m, 1H), 8.16 (dd, 1H) ppm.

### Beispiel 7A

### 5-Amino-1-(3-fluorphenyl)-1H-pyrazol-4-carbonitril

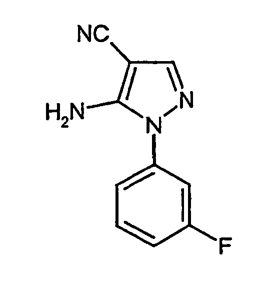

Analog zur Herstellung von Beispiel 1A werden ausgehend von 4 g (24.6 mmol) 3-Fluorphenylhydrazin-Hydrochlorid, 3 g (24.6 mmol) Ethoxymethylenmalonsäuredinitril und 10.3 ml (73.8 mmol) Triethylamin 1.5 g (31% d.Th.) des gewünschten Produktes erhalten.

LC-MS (Methode 2): Rₜ = 2.9 min.

MS (ESI pos): m/z = 203 (M+H)⁺

¹H-NMR (300 MHz, DMSO-d₆): δ = 6.81 (s, 2H), 7.28 (m, 1H), 7.36 (m, 2H), 7.57 (m, 1H), 7.80 (s, 1H) ppm.

### Beispiel 8A

### 5-Amino-l-(2-methylphenyl)-1H-pyrazol-4-carbonitril

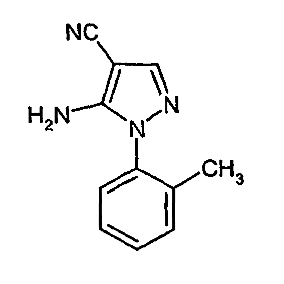

10.2 g (64.4 mmol) 2-Methylphenylhydrazin-Hydrochlorid werden mit 7.8 g (64.4 mmol) Ethoxymethylenmalonsäuredinitril in 100 ml Methanol suspendiert und mit 26.9 ml (193.3 mmol) Triethylamin versetzt. Die Reaktionsmischung wird über Nacht zum Rückfluss erhitzt, wobei sich eine klare Lösung bildet. Das Lösungsmittel wird anschließend unter reduziertem Druck abdestilliert und das Rohprodukt säulenchromatographisch aufgereinigt (Kieselgel, Laufmittel Dichlormethan). Es werden 10.8 g (85% d.Th.) des gewünschten Produkts erhalten.

LC-MS (Methode 2): Rₜ = 3.10 min.

MS (ESI pos): m/z = 199 (M+H)⁺.

### Beispiel 9A

### 5-Amino-1-(2-ethylphenyl)-1H-pyrazol-4-carbonitril

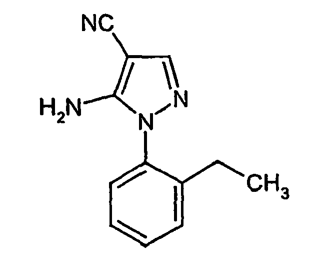

Analog zur Herstellung von Beispiel 1A werden ausgehend von 3.0 g (17.0 mmol) 2-Ethylphenylhydrazin-Hydrochlorid, 2.12 g (17.0 mmol) Ethoxymethylenmalonsäuredinitril und 7.1 ml (51.1 mmol) Triethylamin 3.05 g (83.5% d.Th.) des gewünschten Produktes erhalten.

Fp.: 130°C

MS (ESI pos): m/z = 213 (M+H)⁺

¹H-NMR (300 MHz, DMSO-d₆): δ = 1.0 (t, 3H), 2.35 (q, 2H), 6.4 (s, 2H), 7.2-7.5 (m, 4H), 7.7 (s, 1H) ppm.

### Beispiel 10A

### 5-Amino-1-(2-trifluormethylphenyl)-1H-pyrazol-4-carbonitril

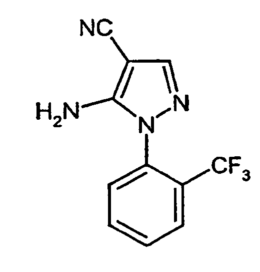

Analog zur Herstellung von Beispiel 1A werden ausgehend von 4.8 g (25.9 mmol) 2-Trifluormethylphenylhydrazin-Hydrochlorid, 3.16 g (25.9 mmol) Ethoxymethylen-malonsäuredinitril und 7.2 ml (51.7 mmol) Triethylamin 5.02 g (76.9% d.Th.) des gewünschten Produktes erhalten.

Fp.: 190°C

MS (ESI pos): m/z = 253 (M+H)⁺

¹H-NMR (300 MHz, DMSO-d₆): δ = 6.6 (s, 2H), 7.5 (d, 1H), 7.7-8.0 (m, 4H) ppm.

### Beispiel 11A

### 5-Amino-1-(2-fluorphenyl)-1H-pyrazol-4-carbonitril

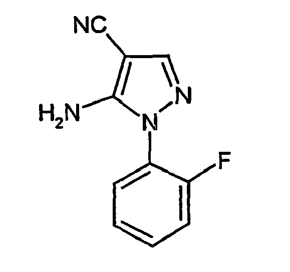

Analog zur Herstellung von Beispiel 1A werden ausgehend von 5.0 g (30.8 mmol) 2-Fluorphenylhydrazin-Hydrochlorid, 3.27 g (26.7 mmol) Ethoxymethylenmalonsäuredinitril und 11.3 ml (81.3 mmol) Triethylamin 5.13 g (88% Reinheit, 84% d.Th.) des gewünschten Produktes erhalten.

MS (ESI pos): m/z = 203 (M+H)⁺

¹H-NMR, (300 MHz, DMSO-d₆): δ = 6.7 (s, 2H), 7.3-7.6 (m, 4H), 7.8 (s, 1H) ppm.

### Beispiel 12A

### 5-Amino-1-(2-chlorphenyl)-1H-pyrazol-carbonitril

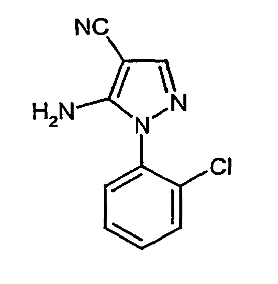

Analog zur Herstellung von Beispiel 1A werden ausgehend von 5.0 g (27.1 mmol) 2-Chlorphenylhydrazin-Hydrochlorid, 3.31 g (27.1 mmol) Ethoxymethylenmalonsäuredinitril und 11.3 ml (81.3 mmol) Triethylamin 4.64 g (78% d.Th.) des gewünschten Produktes erhalten.

Fp.: 135°C

MS (ESI pos): m/z = 219 (M+H)⁺

¹H-NMR (300 MHz, DMSO-d₆): δ = 6.6 (s, 2H), 7.45-7.75 (m, 4H), 7.8 (s, 1H) ppm.

### Beispiel 13A

### 5-Amino-1-(2-pyridinyl)-1H-pyrazol-4-carbonitril

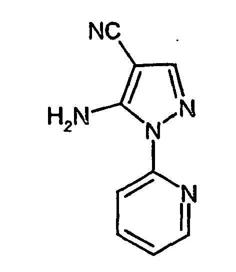

Analog zur Herstellung von Beispiel 1A werden ausgehend von 3.0 g (26.7 mmol, 97% Reinheit) 2-Hydrazinopyridin, 3.26 g (26.7 mmol) Ethoxymethylenmalonsäuredinitril und 7.4 ml (53.3 mmol) Triethylamin 2.3 g (46.6% d.Th.) des gewünschten Produktes erhalten.

Fp.: 193°C

MS (ESI pos): m/z = 186 (M+H)⁺

¹H-NMR (300 MHz, DMSO-d₆): δ = 7.35 (m, 1H), 7.8-8.12 (m, 3H), 8.15 (s, 2H), 8.5 (m, 1H) ppm.

### Beispiel 14A

### 5-Amino-1-(2-methoxyphenyl)-1H-pyrazol-4-carbonitril

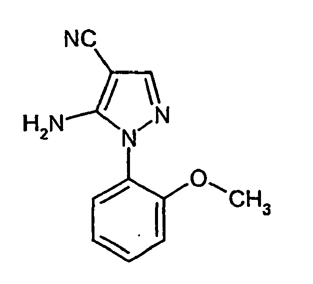

Analog zur Herstellung von Beispiel 1A werden ausgehend von 4.1 g (18 mmol) 2-Methoxyphenylhydrazin-Hydrochlorid, 2.19 g (18 mmol) Ethoxynlethylenmalonsäuredinitril und 10 ml (71.9 mmol) Triethylamin 3.5 g (88% d.Th.) des gewünschten Produktes erhalten.

Fp.: 129°C

MS (ESI pos): m/z = 215 (M+H)⁺

¹H-NMR (300 MHz, DMSO-d₆): δ = 3.8 (s, 3H), 6.3 (s, 2H), 7.05 (t, 1H), 7.2 (d, 1H), 7.25 (d, 1H), 7.5 (t, 1H), 7.7 (s, 1H) ppm.

### Beispiel 15A

### 5-Amino-1-(2,6-dimethylphenyl)-1H-pyrazol-4-carbonsäureamid

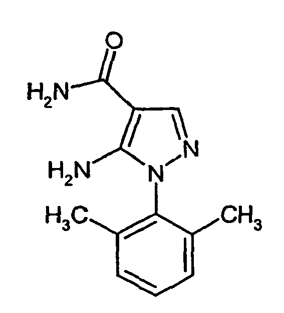

2 g (9.4 mmol) 5-Amino-1-(2,6-dimethylphenyl)-1H-pyrazol-4-carbonitril (Beispiel 1A) werden in 25 ml Ethanol gelöst und mit einer Mischung aus 20 ml 30%-igem Wasserstoffperoxid und 40 ml 25%-igem Ammoniak versetzt. Man rührt über Nacht bei Raumtemperatur und engt anschließend am Rotationsverdampfer die Lösung bis auf ca. 15 ml ein. Die dabei entstehende ölige Emulsion wird in Dichlormethan aufgenommen. Man wäscht mehrfach mit Wasser und gesättigter Natriumthiosulfat-Lösung. Nach Trocknen über Magnesiumsulfat wird das Lösungsmittel im Vakuum entfernt. Der Rückstand wird mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Es werden 0.88 g (40% d.Th.) des Produktes als farbloser Feststoff erhalten.

LC-MS (Methode 2): Rₜ = 2.6 min.

MS (ESI pos): m/z = 231 (M+H)⁺.

### Beispiel 16A

### 5-Amino-1-(2,3-dimethylphenyl)-1H-pyrazol-4-carbonsäureamid

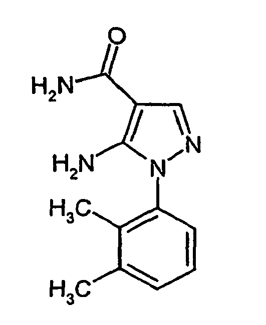

Analog zur Herstellung von Beispiel 15A werden aus 1.5 g (7.1 mmol) 5-Amino-1-(2,3-dimethylphenyl)-1H-pyrazol-4-carbonitril (Beispiel 2A) in einer Mischung aus 25 ml Ethanol, 10 ml 30%-igem Wasserstoffperoxid und 40 ml 25%-igem Ammoniak 1.29 g (70% d.Th.) des gewünschten Produktes erhalten.

LC-MS (Methode 2): Rₜ = 2.7 min.

MS (ESI pos): m/z = 231 (M+H)⁺.

### Beispiel 17A

### 5-Amino-1-(4-methylphenyl)-1H-pyrazol-4-carbonsäureamid

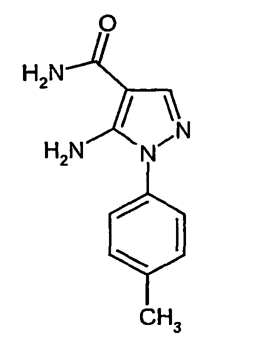

Analog zur Herstellung von Beispiel 15A werden aus 2 g (10.1 mmol) 5-Amino-1-(4-methylphenyl)-1H-pyrazol-4-carbonitril (Beispiel 3A) in einer Mischung aus 25 ml Ethanol, 20 ml 30%-igem Wasserstoffperoxid und 40 ml 25%-igem Ammoniak 1.02 g (47% d.Th.) des gewünschten Produktes erhalten.

LC-MS (Methode 2): Rₜ = 2.7 min.

MS (ESI pos): m/z = 217 (M+H)⁺.

### Beispiel 18A

### 5-Amino-1-(2,6-dichlorphenyl)-1H-pyrazol-4-carbonsäureamid

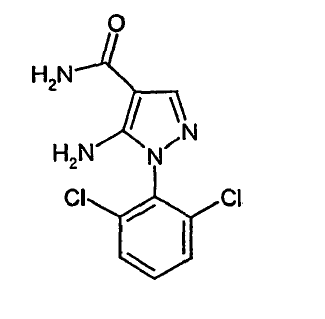

Analog zur Herstellung von Beispiel 15A werden aus 2 g (7.9 mmol) 5-Amino-1-(2,6-dichlorphenyl)-1H-pyrazol-4-carbonitril (Beispiel 4A) in einer Mischung aus 25 ml Ethanol, 10 ml 30%-igem Wasserstoffperoxid und 40 ml 25%-igem Ammoniak 1.6 g (74% d.Th.) des gewünschten Produktes durch Kristallisation aus der Reaktionslösung erhalten.

LC-MS (Methode 2): Rₜ = 2.5 min.

MS (ESI pos): m/z = 271 (M+H)⁺.

### Beispiel 19A

### 5-Amino-1-(2,5-dichlorphenyl)-1H-pyrazol-4-carbonsäureamid

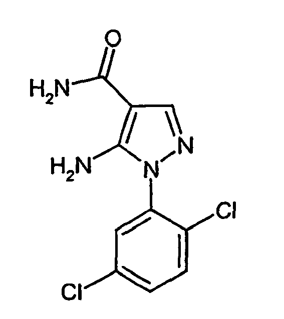

Analog zur Herstellung von Beispiel 15A werden aus 2 g (7.9 mmol) 5-Amino-1-(2,5-dichlorphenyl)-1H-pyrazol-4-carbonitril (Beispiel 5A) in einer Mischung aus 25 ml Ethanol, 18 ml 30%-igem Wasserstoffperoxid und 40 ml 25%-igem Ammoniak 2.02 g (94% d.Th.) des gewünschten Produktes durch Kristallisation aus der Reaktionslösung erhalten.

LC-MS (Methode 2): Rₜ = 2.8 min.

MS (ESI pos): m/z = 271 (M+H)⁺.

### Beispiel 20A

### 5-Amino-1-(2-nitrophenyl)-1H-pyrazol-4-carbonsäureamid

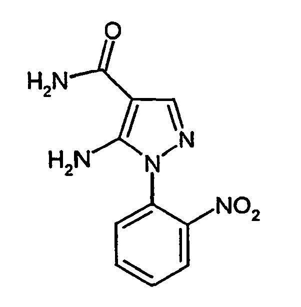

Analog zur Herstellung von Beispiel 15A werden aus 1.5 g (6.5 mmol) 5-Amino-1-(2-nitrophenyl)-1H-pyrazol-4-carbonitril (Beispiel 6A) in einer Mischung aus 25 ml Ethanol, 16 ml 30%-igem Wasserstoffperoxid und 40 ml 25%-igem Ammoniak 1.4 g (86% d.Th.) des gewünschten Produktes durch Kristallisation aus der Reaktionslösung erhalten.

LC-MS (Methode 2): Rₜ = 2.3 min.

MS (ESI pos): m/z = 248 (M+H)⁺.

### Beispiel 21A

### 5-Amino-1-(2-aminophenyl)-1H-pyrazol-4-carbonsäureamid

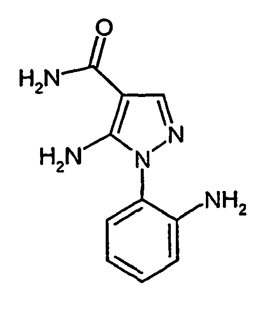

1.28 g (5.27 mmol) 5-Amino-1-(2-nitrophenyl)-1H-pyrazol-4-carbonsäureamid (Beispiel 20A) werden in 30 ml Essigsäureethylester vorgelegt und mit 5.8 g (25.8 mmol) Zinn(II)chlorid-Dihydrat 16 h lang bei 70°C gerührt. Nach Abkühlen auf Raumtemperatur wird die Lösung mit gesättigter Natriumhydrogencarbonat-Lösung auf pH 9-10 gebracht. Die dabei ausfallenden Zinnsalze werden über Kieselgur abfiltriert. Das Filtrat wird mit Essigsäureethylester extrahiert. Die vereinigten organischen Phasen werden mit gesättigter Natriumchlorid-Lösung gewaschen. Nach Trocknen über Natriumsulfat wird das Lösungsmittel im Vakuum entfernt. Man erhält 0.82 g (72% d.Th.) des gewünschten Produktes.

LC-MS (Methode 4): Rₜ = 3.0 min.

MS (ESI pos): m/z = 218 (M+H)⁺

¹H-NMR (300 MHz, DMSO-d₆): δ = 5.04 (s, 2H), 6.00 (s, 2H), 6.66 (m, 1H), 6.89 (m, 1H), 7.03 (m, 2H), 7.92 (s, 1H) ppm.

### Beispiel 22A

### 5-Amino-1-(3-fluorphenyl)-1H-pyrazol-4-carbonsäureamid

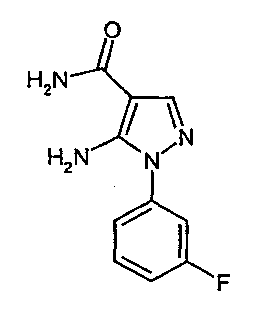

Analog zur Herstellung von Beispiel 15A werden aus 1.3 g (6.4 mmol) 5-Amino-1-(3-fluorphenyl)-1H-pyrazol-4-carbonitril (Beispiel 7A) in einer Mischung aus 25 ml Ethanol, 10 ml 30%-igem Wasserstoffperoxid und 40 ml 25%-igem Ammoniak 1.1 g (75% d.Th.) des gewünschten Produktes durch Kristallisation aus der Reaktionslösung erhalten.

LC-MS (Methode 2): Rₜ = 2.6 min.

MS (ESI pos): m/z = 221 (M+H)⁺.

### Beispiel 23A

### 5-Amino-1-(2-methylphenyl)-1H-pyrazol-4-carbonsäureamid

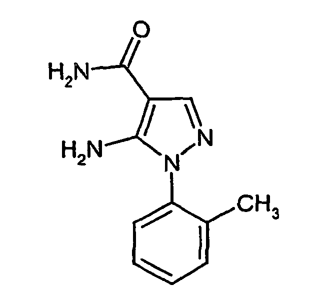

40.0 g (201.8 mmol) 5-Amino-1-(2-methylphenyl)-1H-pyrazol-4-carbonitril (Beispiel 8A) werden unter Eiskühlung vorsichtig mit 300 ml 96%-iger Schwefelsäure versetzt. Anschließend wird auf 40°C erhitzt und 2 h lang bei dieser Temperatur gerührt. Nach dem Abkühlen wird auf 2 l Eiswasser gegossen und vorsichtig mit 50%-iger Natriumhydroxid-Lösung neutralisiert. Nach dreimaliger Extraktion mit Essigsäureethylester (jeweils 2 l) werden die vereinigten organischen Phasen mit gesättigter Natriumchlorid-Lösung gewaschen, über Natriumsulfat getrocknet und das Lösungsmittel unter reduziertem Druck abdestilliert. Es werden 36.0 g (82% d.Th.) Produkt (Reinheit >90%) erhalten, welches ohne weitere Aufreinigung in Folgereaktionen eingesetzt wird.

LC-MS (Methode 1): Rₜ = 2.14 min.

MS (ESI pos): m/z = 217 (M+H)⁺.

### Beispiel 24A

### 5-Amino-1-(2-ethylphenyl)-1H-pyrazol-4-carbonsäureamid

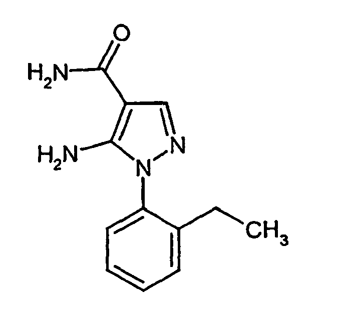

Analog zur Herstellung von Beispiel 15A werden aus 2.75 g (12.8 mmol) 5-Amino-1-(2-ethylphenyl)-1H-pyrazol-4-carbonitril (Beispiel 9A) in einer Mischung aus 106 ml Ethanol, 27 ml 30%-igem Wasserstoffperoxid und 133 ml 25%-igem Ammoniak 2.58 g (87% d.Th.) des gewünschten Produktes nach Kieselgelchromatographie (Laufmittel Dichlormethan mit 0-10% Methanol) erhalten.

Fp.: 147°C

MS (ESI pos): m/z = 231 (M+H)⁺

¹H-NMR (300 MHz, DMSO-d₆): δ = 1.0 (t, 3H), 2.4 (q, 2H), 5.95 (s, 2H), 6.3 (breites d, 2H), 7.2-7.5 (m, 4H), 7.8 (s, 1H) ppm.

### Beispiel 25A

### 5-Amino-1-(2-trifluormethylphenyl)-1H-pyrazol-4-carbonsäurearnid

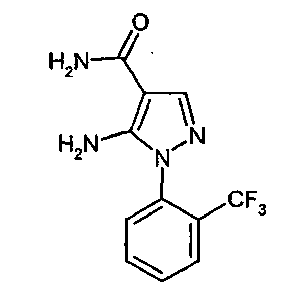

Analog zur Herstellung von Beispiel 15A werden aus 5.0 g (19.8 mmol) 5-Amino-1-(2-trifluormethylphenyl)-1H-pyrazol-4-carbonitril (Beispiel 10A) in einer Mischung aus 195 ml Ethanol, 49 ml 30%-igem Wasserstoffperoxid und 244 ml 25%-igem Ammoniak 4.01 g (87% d.Th.) des gewünschten Produktes nach Kieselgelchromatographie (Laufmittel Dichlormethan mit 0-10% Methanol) erhalten.

Fp.: 186°C

MS (ESI pos): m/z = 271 (M+H)⁺

¹H-NMR (300 MHz, DMSO-d₆): δ = 6.1 (s, 2H), 7.0 (breites d, 2H), 7.45-8.0 (m, 5H) ppm.

### Beispiel 26A

### 5-Amino-1-(2-fluorphenyl)-1H-pyrazol-4-bonsäureamid

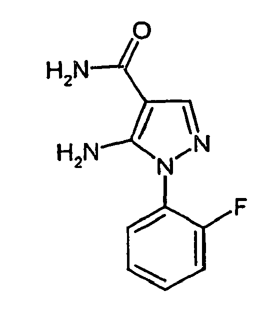

Analog zur Herstellung von Beispiel 15A werden aus 5.0 g (21.9 mmol, 89% Reinheit) 5-Amino-1-(2-fluorphenyl)-1H-pyrazol-4-carbonitril (Beispiel 11A) in einer Mischung aus 173 ml Ethanol, 43 ml 30%-igem Wasserstoffperoxid und 216 ml 25%-igem Ammoniak 3.89 g (81% d.Th.) des gewünschten Produktes nach Kieselgelchromatographie (Laufmittel Dichlormethan mit 0-10% Methanol) erhalten.

Fp.: 181°C

MS (ESI pos): m/z = 221 (M+H)⁺

¹H-NMR (300 MHz, DMSO-d₆): δ = 6.2 (s, 2H), 7.0 (breites d, 2H), 7.3-7.6 (m, 4H), 7.9 (s, 1H) ppm.

### Beispiel 27A

### 5-Amino-1-(2-chlorphenyl)-1H-pyrazol-4-carbonsäureamid

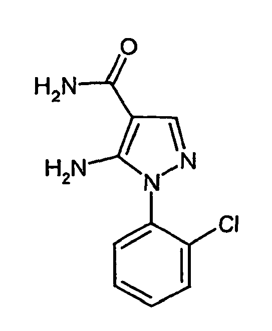

Analog zur Herstellung von Beispiel 15A werden aus 4.6 g (21.0 mmol) 5-Amino-1-(2-chlorphenyl)-1H-pyrazol-4-carbonitril (Beispiel 12A) in einer Mischung aus 159 ml Ethanol, 39 ml 30%-igem Wasserstoffperoxid und 198 ml 25%-igem Ammoniak 3.93 g (79% d.Th.) des gewünschten Produktes nach Kieselgelchromatographie (Laufmittel Dichlormethan mit 0-10% Methanol) erhalten.

Fp.: 166°C

MS (ESI pos): m/z = 237 (M+H)⁺

¹H-NMR (300 MHz, DMSO-d₆): δ = 6.1 (s, 2H), 7.0 (breites d, 2H), 7.4-7.7 (m, 4H), 7.85 (s, 1H) ppm.

### Beispiel 28A

### 5-Amino-1-(2-pyridinyl)-1H-pyrazol-4-carbonsäureamid

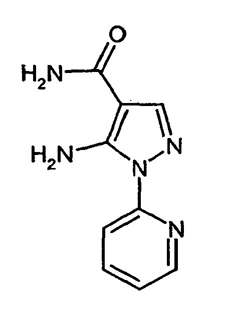

Analog zur Herstellung von Beispiel 15A werden aus 2.3 g (12.4 mmol) 5-Amino-1-(2-pyridinyl)-1H-pyrazol-4-carbonitril (Beispiel 13A) in einer Mischung aus 90 ml Ethanol, 23 ml 30%-igem Wasserstoffperoxid und 113 ml 25%-igem Ammoniak 2.28 g (90% d.Th.) des gewünschten Produktes nach Kieselgelchromatographie (Laufmittel Dichlormethan mit 0-10% Methanol) erhalten.

Fp.: 218°C

MS (DCI): m/z = 204 (M+H)⁺

¹H-NMR (300 MHz, DMSO-d₆): δ = 7.1 (breites d, 2H), 7.3 (dd, 1H), 7.5 (s, 2H), 7.85 (d, 1H), 7.95 (s, 1H), 8.0 (dd, 1H), 8.45 (d, 1H) ppm.

### Beispiel 29A

### 5-Amino-1-(2-methoxyphenyl)-1H-pyrazol-4-carbonsäureamid

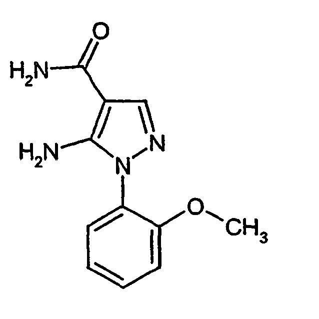

Analog zur Herstellung von Beispiel 15A werden aus 3.5 g (16.0 mmol, 98% Reinheit) 5-Amino-1-(2-methoxyphenyl)-1H-pyrazol-4-carbonitril (Beispiel 14A) in einer Mischung aus 172 ml Ethanol, 34 ml 30%-igem Wasserstoffperoxid und 137 ml 25%-igem Ammoniak 2.61 g (70% d.Th.) des gewünschten Produktes nach Kieselgelchromatographie (Laufmittel Dichlormethan mit 0-10% Methanol) erhalten.

Fp.: 191°C

MS (ESI pos): m/z = 233 (M+H)⁺

¹H-NMR (300 MHz, DMSO-d₆): δ = 3.8 (s, 3H), 5.9 (s, 2H), 7.0 (breites s, 2H), 7.05-7.55 (m, 4H), 7.8 (s, 1H) ppm.

### Beispiel 30A

### 5-Amino-1-(2-ethoxyphenyl)-1H-pyrazol-4-carbonitril

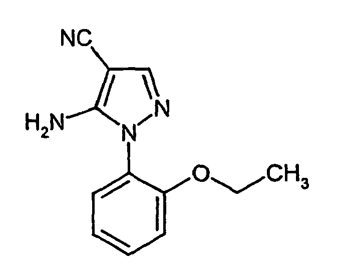

Analog zur Herstellung von Beispiel 1A werden ausgehend von 4.0 g (21.2 mmol) 2-Ethoxyphenylhydrazin-Hydrochlorid, 2.5 g (21.2 mmol) Ethoxymethylenmalonsäuredinitril und 8.8 ml (63.6 mmol) Triethylamin 2.9 g (59% d.Th.) des gewünschten Produktes erhalten.

LC-MS (Methode 1): Rₜ = 2.32 min.

MS (ESI pos): m/z = 229 (M+H)⁺

¹H-NMR (300 MHz, DMSO-d₆): δ = 1.25 (t, 3H), 4.08 (q, 2H), 6.37 (s, 2H), 7.04 (m, 1H), 7.25 (m, 2H), 7.45 (m, 1H), 7.71 (s, 1H) ppm.

### Beispiel 31A

### 5-Amino-1-(2-ethoxyphenyl)-1H-pyrazol-4-carbonsäureamid

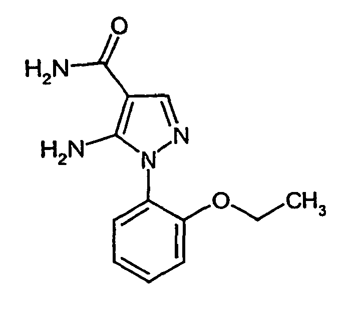

Analog zur Herstellung von Beispiel 15A werden aus 2.5 g (10.9 mmol) 5-Amino-1-(2-ethoxyphenyl)-1H-pyrazol-4-carbonitril (Beispiel 30A) in einer Mischung aus 20 ml Ethanol, 10 ml 30%-igem Wasserstoffperoxid und 10 ml 25%-igem Ammoniak 2.2 g (84% d.Th.) des gewünschten Produktes erhalten.

LC-MS (Methode 4): Rₜ = 1.73 min.

MS (ESI pos): m/z = 247 (M+H)⁺.

### Beispiel 32A

### cis-Hexahydro-2H-cyclopenta[b]furan-2-on

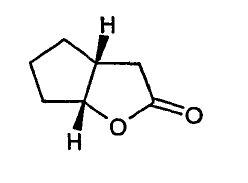

32 ml konzentrierte Schwefelsäure (96%-ig) werden auf -10°C gekühlt. Anschließend werden langsam 5.0 g (39.6 mmol) 2-Cyclopenten-1-ylessigsäure zudosiert und die Reaktionsmischung 1 h bei gleicher Temperatur gerührt. Es wird auf 100 ml Eiswasser gegossen und mit 100 ml Diethylether extrahiert. Die organische Phase wird über Natriumsulfat getrocknet und das Lösungsmittel vorsichtig abdestilliert. Man erhält 2.9 g des racemischen Lactons in 70%-iger Reinheit (LC-MS), welches als Rohprodukt weiter eingesetzt wird.

MS (ESI pos): m/z = 127 (M+H)⁺.

### Beispiel 33A

### 3-Hydrazino-4-methylpyridin

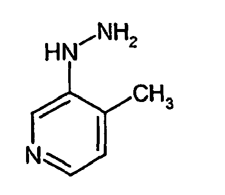

Eine Lösung von 4.0 g (37 mmol) 3-Amino-4-methylpyridin in 19 ml 6 N Salzsäure wird unter Kühlung im Eis-Kochsalz-Bad mit 2.55 ml einer 2.5 M wässrigen Natriumnitrit-Lösung versetzt. Die resultierende Diazoniumsalz-Lösung wird bei -10°C bis -15°C langsam zu einer Lösung von 21 g (111 mmol) Zinn(II)chlorid in 26 ml Salzsäure getropft. Die Lösung wird über Nacht im Kühlschrank stehen gelassen, um die Reaktion zu vervollständigen. Der ausgefallene Feststoff wird abgesaugt, in 26 ml Wasser suspendiert, mit konzentrierter Natronlauge basisch gestellt und filtriert. Das Filtrat wird zehnmal mit je 20 ml Dichlormethan extrahiert, und die vereinigten organischen Phasen über Natriumsulfat getrocknet und eingeengt. Nach Trocknung im Hochvakuum erhält man 1.45 g (31% d.Th.) des gewünschten Produkts als farbloses Öl.

MS (ESI pos): m/z = 124 (M+H)⁺

¹H-NMR (400 MHz, DMSO-d₆): δ = 2.05 (s, 3H), 4.05 (s, 2H), 6.4 (s, 1H), 6.9 (d, 1H), 7.75 (d, 1H), 8.3 (s, 1H) ppm.

### Beispiel 34A

### 5-Amino-1-(4-methylpyridin-3-yl)-1H-pyrazol-4-carbonitril

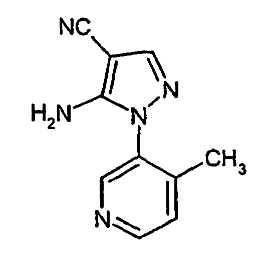

Analog zur Herstellung von Beispiel 1A werden ausgehend von 1.44 g (11.7 mmol) 3-Hydrazino-4-methylpyridin (Beispiel 33A), 1.47 g (11.7 mmol) Ethoxymethylenmalonsäuredinitril und 4.9 ml (35 mmol) Triethylamin 1.75 g (75% d.Th.) des gewünschten Produktes erhalten.

MS (ESI pos): m/z = 200 (M+H)⁺

¹H-NMR (300 MHz, DMSO-d₆): δ = 2.1 (s, 3H), 6.7 (s, 2H), 7.45 (d, 1H), 7.8 (s, 1H), 8.4 (s, 1H), 8.55 (d, 1H) ppm.

### Beispiel 35A

### 5-Amino-1-(4-methylpyridin-3-yl)-1H-pyrazol-4-carbonsäureamid

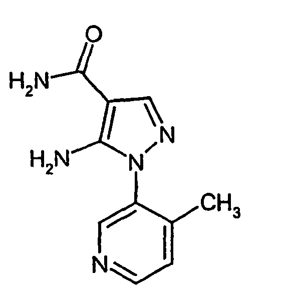

Analog zur Herstellung von Beispiel 15A werden aus 1.75 g (8.78 mmol) 5-Amino-1-(4-methylpyridin-3-yl)-1H-pyrazol-4-carbonitril (Beispiel 34A) in einer Mischung aus 105 ml Ethanol, 9.2 ml 30%-igem Wasserstoffperoxid und 84 ml 25%-igem Ammoniak 1.75 g (91% d.Th.) des gewünschten Produktes erhalten.

MS (ESI pos): m/z = 218 (M+H)⁺

¹H-NMR (200 MHz, DMSO-d₆): δ = 2.1 (s, 3H), 6.2 (s, 2H), 6.6-7.5 (2 breite s, 2H), 7.45 (d, 1H), 7.9 (s, 1H), 8.4 (s, 1H), 8.5 (d, 1H) ppm.

### Ausführungsbeispiele:

### Beispiel 1

### 6-Cyclopentylmethyl-1-(2,6-dimethylphenyl)-1,5-dihydro-pyrazolo[3,4-d]pyrimidin-4-on

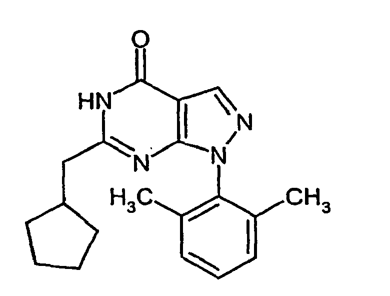

0.1 g (0.43 mmol) 5-Amino-1-(2,6-dimethylphenyl)-1H-pyrazol-4-carborisäureamid (Beispiel 15A) werden unter Argon in 6 ml absolutem Ethanol gelöst und mit 0.24 g (1.7 mmol) Cyclopentylessigsäuremethylester und 0.17 g (4.34 mmol) 60%-igem Natriumhydrid (Suspension in Mineralöl) versetzt. Man erhitzt die Reaktionsmischung über Nacht zum Rückfluss. Nach Abkühlen auf Raumtemperatur wird mit konzentrierter Salzsäure angesäuert. Das dabei ausfallende Natriumchlorid wird abfiltriert. Das Filtrat wird im Vakuum eingeengt und der verbleibende Rückstand mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Es werden 74 mg (53% d.Th.) des Produktes als farbloser Feststoff erhalten.

LC-MS (Methode 3): Rₜ = 3.79 min.

MS (ESI pos): m/z = 323 (M+H)⁺.

### Beispiel 2

### 6-Cyclopentylmethyl-1-(2,3-dimethylphenyl)-1,5-dihydro-pyrazolo[3,4-d]pyrimidin-4-on

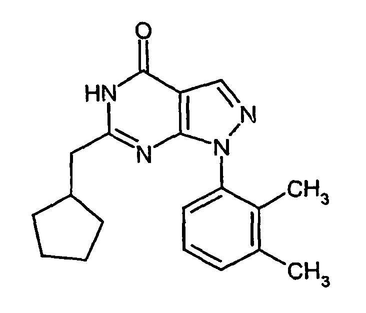

0.1 g (0.43 mmol) 5-Amino-1-(2,3-dimethylphenyl)-1H-pyrrazol-4-carbonsäureamid (Beispiel 16A) werden unter Argon in 6 ml absolutem Ethanol gelöst und mit 0.24 g (1.7 mmol) Cyclopentylessigsäuremethylester und 0.17 g (4.34 mmol) 60%-igem Natriumhydrid (Suspension in Mineralöl) versetzt. Man erhitzt die Reaktionsmischung über Nacht zum Rückfluss. Nach Abkühlen auf Raumtemperatur wird mit konzentrierter Salzsäure angesäuert. Die dabei ausfallende Mischung aus Natriumchlorid und dem Produkt wird abfiltriert und mehrfach mit Wasser und Diethylether gewaschen. Nach Trocknen im Hochvakuum werden 69 mg (49% d.Th.) des Produktes als farbloser Feststoff erhalten.

LC-MS (Methode 3): Rₜ = 3.57 min.

MS (ESI pos): m/z = 323 (M+H)⁺

¹H-NMR (300 MHz, DMSO-d₆): δ = 1.17 (m, 2H), 1.48 (m, 2H), 1.59 (m, 4H), 1.87 (s, 3H), 2.19 (m, 1H), 2.33 (s, 3H), 2.54 (d, 2H), 7.16 (d, 1H), 7.25 (t, 1H), 7.36 (d, 1H), 8.21 (s, 1H), 12.12 (s, 1H) ppm.

### Beispiel 3

### 6-Cyclopentylmethyl-1-(4-methylphenyl)-1,5-dihydropyrazolo[3,4-d]pyrimidin-4-on

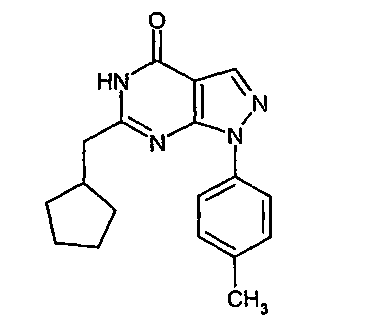

Analog zur Herstellung von Beispiel 1 werden ausgehend von 0.88 g (0.41 mmol) 5-Amino-1-(4-methylphenyl)-1H-pyrazol-4-carbonsäureamid (Beispiel 17A), 0.26 g (1.8 mmol) Cyclopentylessigsäuremethylester und 0.16 g (4.09 mmol) 60%-igem Natriumhydrid 97 mg (68% d.Th.) des gewünschten Produktes als farbloser Feststoff erhalten.

LC-MS (Methode 3): Rₜ = 4.09 min.

MS (ESI pos): m/z = 309 (M+H)⁺

¹H-NMR (300 MHz, DMSO-d₆): δ = 1.23 (m, 2H), 1.57 (m, 2H), 1.72 (m, 4H), 2.34 (m, 1H), 2.36 (s, 3H), 2.66 (d, 2H), 7.34 (d, 1H), 7.92 (d, 1H), 8.23 (s, 1H), 12.27 (s, 1H) ppm.

### Beispiel 4

### 6-Cyclopentylmethyl-1-(2,6-dichlorphenyl)-1,5-dihydro-pyrazolo[3,4-d]pyrimidin-4-on

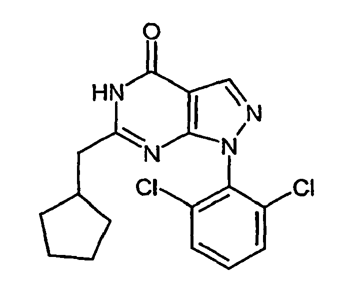

Analog zur Herstellung von Beispiel 2 werden ausgehend von 0.1 g (0.37 mmol) 5-Amino-1-(2,6-dichlorphenyl)-1H-pyrazol-4-carbonsäureamid (Beispiel 18A), 0.2 g (1.4 mmol) Cyclopentylessigsäuremethylester und 0.14 g (3.6 mmol) 60%-igem Natriumhydrid 61 mg (45% d.Th.) des gewünschten Produktes als farbloser Feststoff erhalten.

LC-MS (Methode 3): Rₜ = 3.73 min.

MS (ESI pos): m/z = 363 (M+H)⁺

¹H-NMR (300 MHz, DMSO-d₆): δ = 1.15 (m, 2H), 1.49 (m, 2H), 1.60 (m, 4H), 2.21 (m, 1H), 2.57 (d, 2H), 7.60 (m, 2H), 7.69 (m, 1H), 8.41 (s, 1H), 12.51 (s, 1H) ppm.

### Beispiel 5

### 6-Cyclopentylmethyl-1-(2,5-dichlorphenyl)-1,5-dihydro-pyrazolo[3,4-d]pyrimidin-4-on

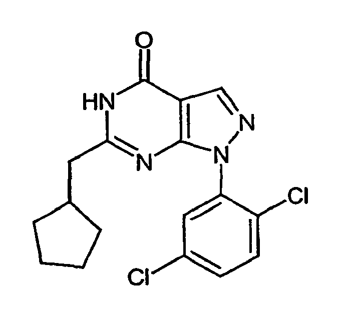

Analog zur Herstellung von Beispiel 1 werden ausgehend von 0.1 g (0.37 mmol) 5-Amino-1-(2,5-dichlorphenyl)-1H-pyrazol-4-carbonsäureamid (Beispiel 19A), 0.2 g (1.4 mmol) Cyclopentylessigsäuremethylester und 0.14 g (3.6 mmol) 60%-igem Natriumhydrid 32 mg (23% d.Th.) des gewünschten Produktes als farbloser Feststoff erhalten.

LC-MS (Methode 3): Rₜ = 4.0 min.

MS (ESI pos): m/z = 363 (M+H)⁺

¹H-NMR (300 MHz, DMSO-d₆): δ = 1.15 (m, 2H), 1.49 (m, 2H), 1.60 (m, 4H), 2.22 (m, 1H), 2.55 (d, 2H), 7.16 (d, 1H), 7.31 (m, 1H), 7.32 (m, 2H), 8.23 (s, 1H), 12.39 (s, 1H) ppm.

### Beispiel 6

### 1-(2-Aminophenyl)-6-cyclopentylmethyl-1,5-dihydro-pyrazolo[3,4-d]pyrimidin-4-on

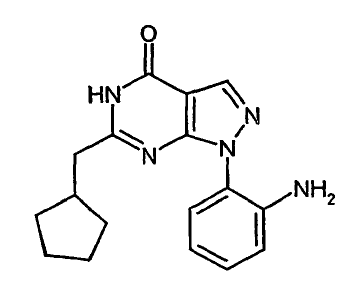

Analog zur Herstellung von Beispiel 1 werden ausgehend von 0.1 g (0.46 mmol) 5-Amino-1-(2-aminophenyl)-1H-pyrazol-4-carbonsäureamid (Beispiel 21A), 0.19 g (1.4 mmol) Cyclopentylessigsäuremethylester und 0.18 g (4.6 mmol) 60%-igem Natriumhydrid 61 mg (42% d.Th.) des gewünschten Produktes als farbloser Feststoff erhalten.

LC-MS (Methode 4): Rₜ = 3.9 min.

MS (ESI pos): m/z = 310 (M+H)⁺

¹H-NMR (300 MHz, DMSO-d₆): δ = 1.17 (m, 2H), 1.45 (m, 2H), 1.56 (m, 4H), 2.19 (m, 1H), 2.52 (d, 2H), 6.12 (s, 2H), 6.64 (m, 1H), 6.90 (m, 1H), 7.05 (m, 2H), 8.25 (s, 1H), 12.47 (s, 1H) ppm.

### Beispiel 7

### 6-Cyclopentylmethyl-1-(3-fluorphenyl)-1,5-dihydro-pyrazolo[3,4-d]pyrimidin-4-on

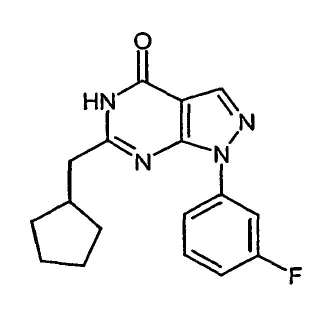

Analog zur Herstellung von Beispiel 1 werden ausgehend von 0.1 g (0.45 mmol) 5-Amino-1-(3-fluorphenyl)-1H-pyrazol-4-carbonsäureamid (Beispiel 22A), 0.26 g (1.8 mmol) Cyclopentylessigsäuremethylester und 0.18 g (4.5 mmol) 60%-igem Natriumhydrid 82 mg (58% d.Th.) des gewünschten Produktes als farbloser Feststoff erhalten.

LC-MS (Methode 3): Rₜ = 3.74 min.

MS (ESI pos): m/z = 313 (M+H)⁺

¹H-NMR (300 MHz, DMSO-d₆): δ = 1.12 (m, 2H), 1.58 (m, 2H), 1.75 (m, 4H), 2.34 (m, 1H), 2.69 (d, 2H), 7.23 (m, 1H), 7.63 (m, 1H), 8.00 (m, 2H), 8.31 (s, 1H), 12.37 (s, 1H) ppm.

### Beispiel 8

### 6-(2-Cyclopenten-1-ylmethyl)-1-(2-ethylphenyl)-1,5-dihydro-4H-pyrazolo[3,4-d]pyrimidin-4-on

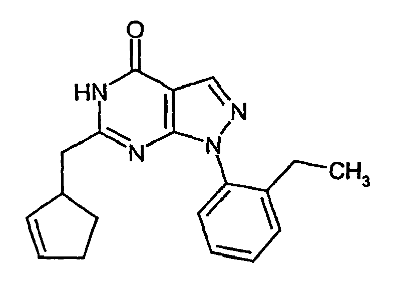

Analog zur Herstellung von Beispiel 1 werden ausgehend von 0.15 g (0.65 mmol) 5-Amino-1-(2-ethylphenyl)-1H-pyrazol-4-carbonsäureamid (Beispiel 24A), 0.27 g (1.95 mmol) 2-Cyclopenten-1-ylessigsäuremethylester und 0.13 g (3.2 mmol) 60%-igem Natriumhydrid 64 mg (31%.d.Th.) des gewünschten Produktes als farbloser Feststoff erhalten.

Fp.: 146°C

MS (ESI pos): m/z = 321 (M+H)⁺

¹H-NMR (300 MHz, DMSO-d₆): δ = 0.95 (t, 3H), 1.45 (m, 1H), 1.95 (m, 1H), 2.1-2.75 (m, 6H), 3.0 (m, 1H), 5.5-5.8 (m, 2H), 7.25-7.5 (m, 4H), 8.2 (s, 1H), 12.2 (s, 1H) ppm.

### Beispiel 9

### 6-(2-Cyclopenten-1-ylmethyl)-1-(2-methylphenyl)-1,5-dihydro-4H-pyrazolo[3,4-d]pyrimidin-4-on

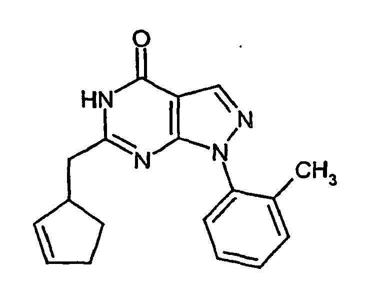

Analog zur Herstellung von Beispiel 1 werden ausgehend von 0.12 g (0.56 mmol) 5-Amino-1-(2-methylphenyl)-1H-pyrazol-4-carbonsäureamid (Beispiel 23A), 0.24 g (1.7 mmol) 2-Cyclopenten-1-ylessigsäuremethylester und 0.11 g (2.8 mmol) 60%-igem Natriumhydrid 44 mg (26% d.Th.) des gewünschten Produktes als farbloser Feststoff erhalten.

Fp.: 179°C

MS (ESI pos): m/z = 307 (M+H)⁺

¹H-NMR (300 MHz, DMSO-d₆): δ = 1.45 (m, 1H), 1.95 (m, 1H), 2.1 (s, 3H), 2.1-2.75 (m, 4H), 3.05 (m, 1H), 5.5-5.8 (m, 2H), 7.3-7.5 (m, 4H), 8.25 (s, 1H), 12.2 (s, 1H) ppm.

### Beispiel 10

### 6-Cyclohexylmethyl-1-(2-methylphenyl)-1,5-dihydro-4H-pyrazolo[3,4-d]pyrimidin-4-on

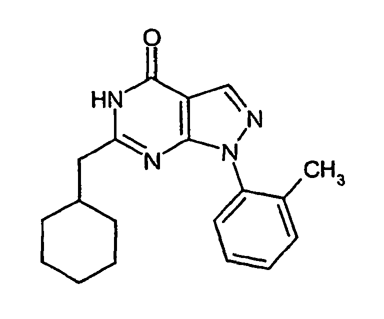

Analog zur Herstellung von Beispiel 1 werden ausgehend von 0.15 g (0.68 mmol) 5-Amino-1-(2-methylphenyl)-1H-pyrazol-4-carbonsäureamid (Beispiel 23A), 0.35 g (2.04 mmol) Cyclohexylessigsäureethylester und 0.136 g (3.4 mmol) 60%-igem Natriumhydrid 65 mg (29% d.Th.) des gewünschten Produktes als farbloser Feststoff erhalten.

Fp.: 169°C

MS (ESI pos): m/z = 323 (M+H)⁺

¹H-NMR (300 MHz, DMSO-d₆): δ = 0.9-1.3 (m, 5H), 1.5-1.9 (m, 6H), 2.1 (s, 3H), 2.45 (d, 2H), 7.3-7.5 (m, 4H), 8.2 (s, 1H), 12.2 (s, 1H) ppm.

### Beispiel 11

### 6-Cylopentylmethyl-1-(2-methylphenyl)-1,5-dihydro-4H-pyrazolo[3,4-d]pyrimidin-4-on

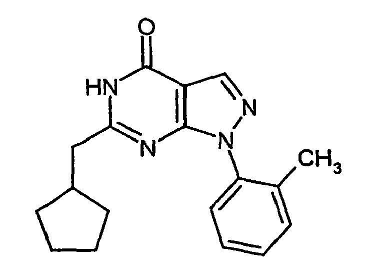

Analog zur Herstellung von Beispiel 1 werden ausgehend von 0.1 g (0.46 mmol) 5-Amino-1-(2-methylphenyl)-1H-pyrazol-4-carbonsäureamid (Beispiel 23A), 0.237 g (92% Reinheit, 1.39 mmol) Cyclopentylessigsäureethylester und 0.093 g (2.32 mmol) 60%-igem Natriumhydrid 43 mg (30% d.Th.) des gewünschten Produktes als farbloser Feststoff erhalten.

Fp.: 181°C .

MS (ESI pos): m/z = 309 (M+H)⁺

¹H-NMR (300 MHz, DMSO-d₆): δ = 1.1-1.55 (m, 8H), 2.1 (s, 3H), 2.2 (m, 1H), 2.55 (d, 2H), 7.3-7.5 (m, 4H), 8.2 (s, 1H), 12.15 (s, 1H) ppm.

### Beispiel 12

### 6-Cyclopentylmethyl-1-(2-ethoxyphenyl)-1,5-dihydro-4H-pyrazolo[3,4-d]pyrimidin-4-on

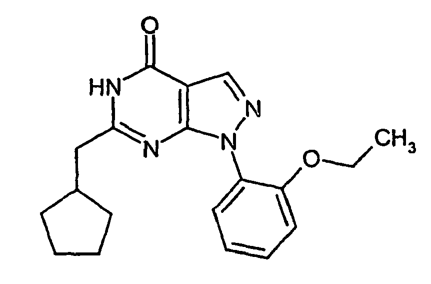

Analog zur Herstellung von Beispiel 1 werden ausgehend von 0.1 g (0.41 mmol) 5-Amino-1-(2-ethoxyphenyl)-1H-pyrazol-4-carbonsäureamid (Beispiel 31A), 0.231 g (1.6 mmol) Cyclopentylessigsäureethylester und 0.162 g (4.1 mmol) 60%-igem Natriumhydrid 73 mg (52% d.Th.) des gewünschten Produktes als farbloser Feststoff erhalten.

LC-MS (Methode 3): Rₜ = 3.5 min.

MS (ESI pos): m/z = 339 (M+H)⁺

¹H-NMR (300 MHz, DMSO-d₆): δ = 1.10 (t, 3H), 1.22 (m, 2H), 1.45 (m, 2H), 1.59 (m, 4H), 1.96 (m, 1H), 2.54 (d, 2H), 4.02 (q, 2H), 7.08 (m, 1H), 7.23 (m, 1H), 7.37 (m, 1H), 7.48 (m, 1H), 8.16 (s, 1H), 12.06 (s, 1H) ppm.

### Beispiel 13

### 6-Cyclopentylmethyl-1-(2-hydroxyphenyl)-1,5-dihydro-4H-pyrazolo[3,4-d]pyrimidin-4-on

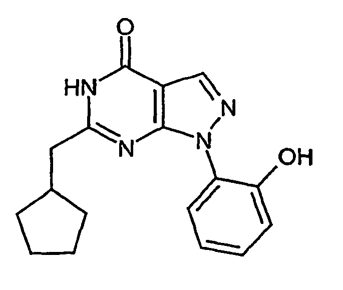

Es werden 0.2 g (0.59 mmol) 6-Cyclopentylmethyl-1-(2-ethoxyphenyl)-1,5-dihydro-4H-pyrazolo[3,4-d]pyrimidin-4-on (Beispiel 12) mit 4 ml 1 M Bortribromid-Lösung in Dichlormethan versetzt und die Reaktionsmischung 1 h bei Raumtemperatur gerührt. Nach Hydrolyse mit Wasser wird mit Dichlormethan extrahiert. Das Produkt wird mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Es werden 0.167 g (91% d.Th.) des Produktes als farbloser Feststoff erhalten.

LC-MS (Methode 4): Rₜ = 2.54 min.

MS (ESI pos): m/z = 311 (M+H)⁺

¹H-NMR (300 MHz, DMSO-d₆): δ = 1.17 (m, 2H), 1.42 (m, 6H), 2.19 (m, 1H), 2.54 (d, 2H), 6.93 (m, 1H), 7.04 (m, 1H), 7.32 (m, 1H), 8.18 (s, 1H), 9.92 (s, 1H), 12.12 (s, 1H) ppm.

### Beispiel 14

### 6-(2-Cyclopenten-1-ylmethyl)-1-[2-(trifluormethyl)phenyl]-1,5-dihydro-4H-pyrazolo[3,4-d]pyrimidin-4-on

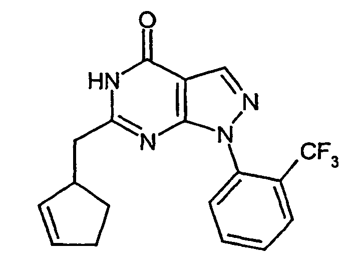

Analog zur Herstellung von Beispiel 1 werden ausgehend von 0.15 g (0.56 mmol) 5-Amino-1-[2-(trifluormethyl)phenyl]-1H-pyrazol-4-carbonsäureamid (Beispiel 25A), 0.233 g (1.67 mmol) 2-Cyclopenten-1-ylessigsäuremethylester und 0.111 g (2.78 mmol) 60%-igem Natriumhydrid 57 mg (29% d.Th.) des gewünschten Produktes als farbloser Feststoff erhalten.

Fp.: 153°C

MS (ESI pos): m/z = 361 (M+H)⁺

¹H-NMR (300 MHz, DMSO-d₆): δ = 1.45 (m, 1H), 1.9 (m, 1H), 2.1-2.4 (m, 2H), 2.45-2.7 (m, 2H), 3.0 (m, 1H), 5.5-5.8 (m, 2H), 7.6 (d, 1H), 7.75-8.0 (m, 3H), 8.25 (s, 1H), 12.2 (s, 1H) ppm.

### Beispiel 15

### 6-(2-Cyclopenten-1-ylmethyl)-1-(2-fluorphenyl)-1,5-dihydro-4H-pyrazolo[3,4-d]pyrimidin-4-on

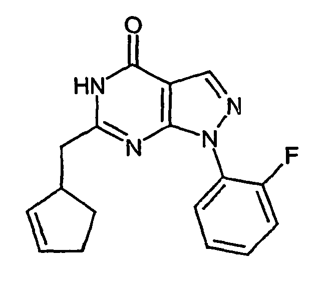

Analog zur Herstellung von Beispiel 1 werden ausgehend von 0.15 g (0.66 mmol) 5-Amino-1-(2-fluorphenyl)-1H-pyrazol-4-carbonsäureamid (Beispiel 26A), 0.279 g (1.99 mmol) 2-Cyclopenten-1-ylessigsäuremethylester und 0.133 g (2.78 mmol) 60%-igem Natriumhydrid 77 mg (37% d.Th.) des gewünschten Produktes als farbloser Feststoff erhalten.

Fp.: 163°C

MS (ESI pos): m/z = 311 (M+H)⁺

¹H-NMR (300 MHz, DMSO-d₆): δ = 1.5 (m, 1H), 1.95 (m, 1H), 2.1-2.45 (m, 2H), 2.45-2.7 (m, 2H), 3.0 (m, 1H), 5.6-5.8 (m, 2H), 7.3-7.7 (m, 4H), 8.3 (s, 1H), 12.3 (s, 1H) ppm.

### Beispiel 16

### 6-(2-Cyclopenten-1-ylmethyl)-1-(2-chlorphenyl)-1,5-dihydro-4H-pyrazolo[3,4-d]pyrimidin-4-on

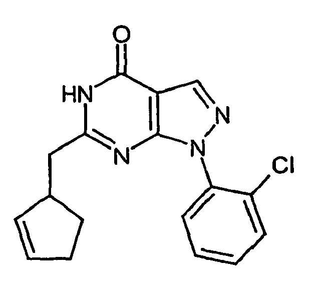

Analog zur Herstellung von Beispiel 1 werden ausgehend von 0.15 g (0.63 mmol) 5-Amino-1-(2-chlorphenyl)-1H-pyrazol-4-carbonsäureamid (Beispiel 27A), 0.266 g (1.90 mmol) 2-Cyclopenten-1-ylessigsäuremethylester und 0.127 g (3.17 mmol) 60%-igem Natriumhydrid 50 mg (24% d.Th.) des gewünschten Produktes als farbloser Feststoff erhalten.

Fp.: 150°C

MS (ESI pos): m/z = 327 (M+H)⁺

¹H-NMR (300 MHz, DMSO-d₆): δ = 1.5 (m, 1H), 1.95 (m, 1H), 2.1-2.4 (m, 2H), 2.5-2.7 (m, 2H), 3.05 (m, 1H), 5.6-5.8 (m, 2H), 7.5-7.8 (m, 4H), 8.25 (s, 1H), 12.2 (s, 1H) ppm.

### Beispiel 17

### 6-(2-Cyclopenten-1-ylmethyl)-1-(2-pyridinyl)-1,5-dihydro-4H-pyrazolo[3,4-d]pyrimidin-4-on

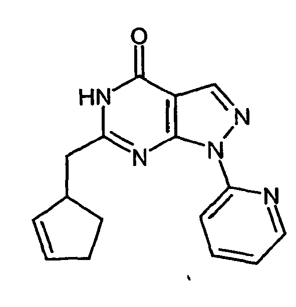

Analog zur Herstellung von Beispiel 1 werden ausgehend von 0.15 g (0.74 mmol) 5-Amino-1-(2-pyridinyl)-1H-pyrazol-4-carbonsäureamid (Beispiel 28A), 0.31 g (2.21 mmol) 2-Cyclopenten-1-ylessigsäuremethylester und 0.147 g (3.69 mmol) 60%-igem Natriumhydrid 76 mg (35% d.Th.) des gewünschten Produktes als farbloser Feststoff erhalten.

Fp.: 239°C

MS (ESI pos): m/z = 294 (M+H)⁺

¹H-NMR (300 MHz, DMSO-d₆): δ = 1.55 (m, 1H), 2.0 (m, 1H), 2.15-2.45 (m, 2H), 2.55-2.75 (m, 2H), 3.15 (m, 1H), 5.65-5.8 (m, 2H), 7.5 (dd, 1H), 8.0 (d, 1H), 8.05 (m, 1H), 8.3 (s, 1H), 8.6 (d, 1H), 12.3 (s, 1H) ppm.

### Beispiel 18

### 6-(2-Cyclopenten-1-ylmethyl)-1-(2-methoxyphenyl)-1,5-dihydro-4H-pyrazolo[3,4-d]pyrimidin-4-on

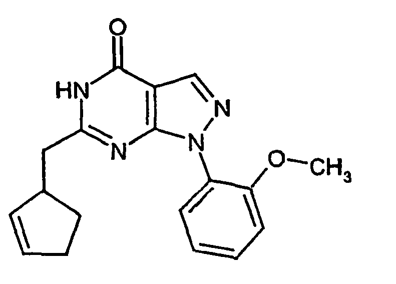

Analog zur Herstellung von Beispiel 1 werden ausgehend von 0.15 g (0.65 mmol) 5-Amino-1-(2-methoxyphenyl)-1H-pyrazol-4-carbonsäureamid (Beispiel 29A), 0.272 g (1.94 mmol) 2-Cyclopenten-1-ylessigsäuremethylester und 0.129 g (3.23 mmol) 60%-igem Natriumhydrid 82 mg (39% d.Th.) des gewünschten Produktes als farbloser Feststoff erhalten.

Fp.: 182°C

MS (ESI pos): m/z = 323 (M+H)⁺

¹H-NMR (300 MHz, DMSO-d₆): δ = 1.5 (m, 1H), 1.95 (m, 1H), 2.1-2.45 (m, 2H), 2.45-2.75 (m, 2H), 3.05 (m, 1H), 3.0 (s, 3H), 5.6-5.8 (m, 2H), 7.0-7.55 (m, 4H), 8.2 (s, 1H), 12.15 (s, 1H) ppm.

Die in der folgenden Tabelle 2 aufgeführten Ausführungsbeispiele 19-31 werden ebenso wie die entsprechende Ausgangsverbindungen in Analogie zu den zuvor beschriebenen Beispielen erhalten:

**Tabelle 2:**

| **Bsp.-Nr.** | **Struktur** | **Ausbeute [% d.Th.]** | **MS: m/z [M+H]⁺** | **Rₜ [min]** | **LC-MS-Methode** |
|---|---|---|---|---|---|
| 19 | 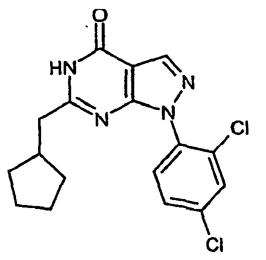 | 14.1 | 364 | 4.05 | 3 |
| 20 | 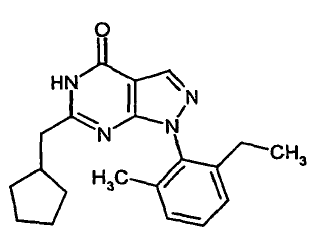 | 29.8 | 337 | 3.97 | 3 |
| 21 | 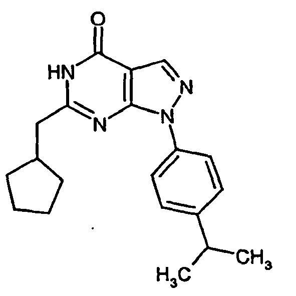 | 26.1 | 337 | 4.52 | 3 |
| 22 | 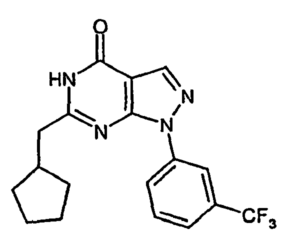 | 48.5 | 363 | 4.39 | 3 |
| 23 | 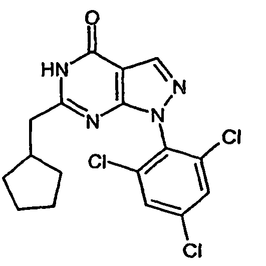 | 14.6 | 398 | 4.20 | 3 |
| 24 | 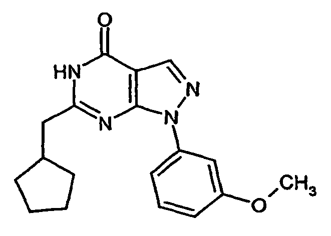 | 78.7 | 325 | 3.88 | 3 |
| 25 | 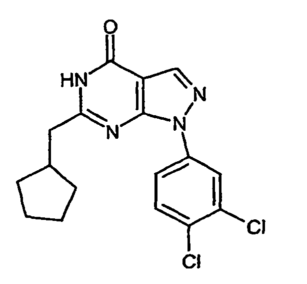 | 28.4 | 364 | 4.70 | 3 |
| 26 | 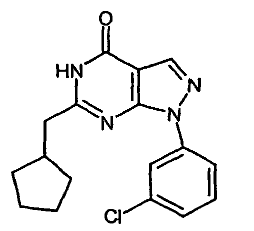 | 48.9 | 329 | 4.30 | 3 |
| 27 | 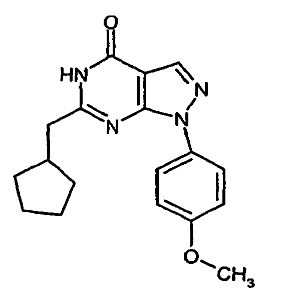 | 60.1 | 325 | 3.79 | 3 |
| 28 | 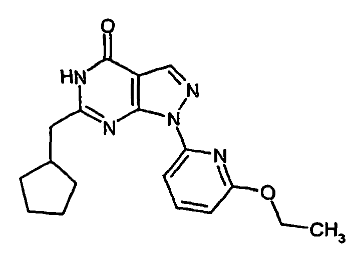 | 10.5 | 340 | 3.61 | 1 |
| 29 | 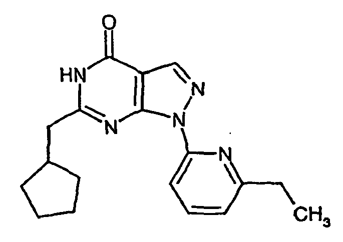 | 7.9 | 324 | 4.00 | 4 |
| 30 | 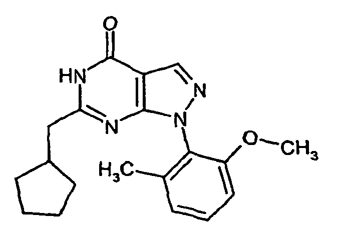 | 48.8 | 339 | 4.10 | 4 |
| 31 | 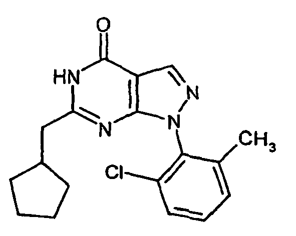 | 38.8 | 343 | 3.07 | 1 |

### Beispiel 32

### 6-[(4-Methylcyclohexyl)methyl]-1-(2-methylphenyl)-1,5-dihydropyrazolo[3,4-d]pyrimidin-4-on

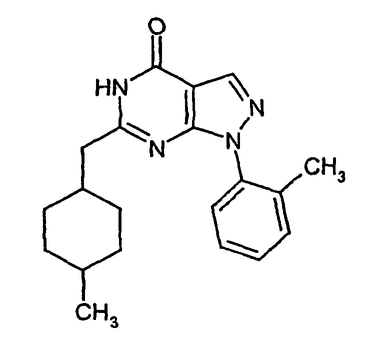

150 mg (0.69 mmol) 5-Amino-1-(2-methylphenyl)-1H-pyrazol-4-carboxamid (Beispiel 23A) und 130 mg (0.83 mmol) 2-(4-Methylcyclohexyl)-essigsäure werden mit 3 ml Polyphosphorsäuretrimethylsilylester versetzt und 3 h lang bei 130°C gerührt. Das heiße Reaktionsgemisch wird auf 20 ml Wasser gegeben und anschließend mit Dichlormethan extrahiert (2 x 20 ml). Die vereinigten organischen Phasen werden mit Wasser (20 ml) und mit gesättigter Natriumchlorid-Lösung (20 ml) gewaschen und über Natriumsulfat getrocknet. Das Lösungsmittel wird unter vermindertem Druck abdestilliert und das Rohprodukt mittels präparativer HPLC aufgereinigt (YMC Gel ODS-AQ S 5/15 µm; Eluent A: Wasser, Eluent B: Acetonitril; Gradient: 0 min 30% B, 5 min 30% B, 50 min 95% B). Es werden 182 mg (78% d.Th.) des Produktes erhalten.

LC-MS (Methode 3): Rₜ = 4.09 min.

MS (ESI pos): m/z = 337 (M+H)⁺

¹H-NMR (200 MHz, DMSO-d₆): δ = 0.68-0.90 (5H), 0.99-1.61 (8H), 1.98-2.07 (4H), 2.16 (d, 1H), 7.19 (d, 1H), 7.28-7.51 (m, 3H), 8.26 (s, 1H), 10.27 (s, 1H) ppm.

### Beispiel 33

### 6-{[(1,2-cis)-2-Hydroxycyclopentyl]methyl}-1-(2-methylphenyl)-1,5-dihydro-4H-pyrazolo[3,4-d]pyrimidin-4-on (Racemat)

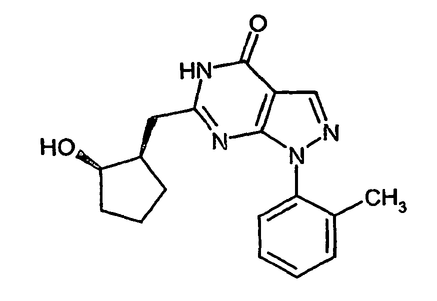

200 mg (0.93 mmol) 5-Amino-1-(2-methylphenyl)-1H-pyrazol-4-carbonsäureamid (Beispiel 23A) und 525 mg cis-Hexahydro-2*H*-cyclopenta[*b*]furan-2-on (ca. 70%-ig, Beispiel 32A) werden unter Argon in 10 ml absolutem Ethanol gelöst und mit 315 mg (4.6 mmol) Natriumethylat versetzt. Man erhitzt die Reaktionsmischung über Nacht zum Rückfluss. Nach Abkühlen auf Raumtemperatur wird mit 25 ml Wasser hydrolysiert und anschließend mit Essigsäureethylester (2 x 25 ml) extrahiert. Die vereinigten organischen Phasen werden über Natriumsulfat getrocknet und das Lösungsmittel unter reduziertem Druck abdestilliert. Das Rohprodukt wird mittels präparativer HPLC aufgereinigt (YMC Gel ODS-AQ S 5/15 µm; Eluent A: Wasser, Eluent B: Acetonitril; Gradient: 0 min 30% B, 5 min 30% B, 50 min 95% B). Es werden 90 mg (30% d.Th.) des gewünschten Produktes erhalten.

MS (ESI pos): m/z = 325 (M+H)⁺

¹H-NMR (300 MHz, DMSO-d₆): δ = 1.28-1.74 (7H), 2.07 (s, 3H), 2.55 (dd, 1H), 2.80 (dd, 1H), 3.97 (m, 1H), 4.43 (d, 1H), 7.36 (m, 2H), 7.43 (m, 2H), 8.22 (s, 1H), 12.07 (s, 1H) ppm.

### Beispiel 34

### 6-{[(1,2-trans)-2-Hydroxycyclohexyl]methyl}-1-(2-methylphenyl)-1,5-dihydro-4H-pyrazolo[3,4-d]pyrimidin-4-on

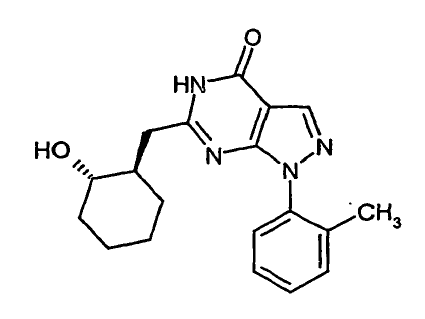

200 mg (0.93 mmol) 5-Amino-1-(2-methylpheny)-1H-pyrazol-4-carbonsäureamid (Beispiel 23A) und 583 mg (4.16 mmol) *rac*-Hexahydro-1-benzofuran-2(3H)-on (Gemisch der cis- und *trans-*Diastereomere; Herstellung siehe z.B. K.F. Podraza et al., J. Heterocycl. Chem. 1987, 24, 293-295) werden unter Argon in 10 ml absolutem Ethanol gelöst und mit 315 mg (4.6 mmol) Natriumethylat versetzt. Man erhitzt die Reaktionsmischung über Nacht zum Rückfluss. Nach Abkühlen auf Raumtemperatur wird mit 25 ml Wasser hydrolysiert und anschließend mit Essigsäureethylester (2 x 25 ml) extrahiert. Die vereinigten organischen Phasen werden über Natriumsulfat getrocknet und das Lösungsmittel unter reduziertem Druck abdestilliert. Das Rohprodukt wird mittels präparativer HPLC aufgereinigt (YMC Gel ODS-AQ S 5/15 µm; Eluent A: Wasser, Eluent B: Acetonitril; Gradient: 0 min 30% B, 5 min 30% B, 50 min 95% B). Es werden 68 mg (21% d.Th.) des gewünschten Produktes erhalten.

MS (ESI pos): m/z = 339 (M+H)⁺

¹H-NMR (400 MHz, DMSO-d₆): δ = 0.97 (m, 2H), 1.15 (m, 2H), 2.51 (d, 2H), 1.64 (m, 2H), 1.81 (m, 1H), 2.07 (s, 3H), 2.26 (dd, 1H), 2.99-3.10 (2H), 4.61 (d, 1H), 7.37 (m, 2H), 7.44 (m, 2H), 8.23 (s, 1H), 12.11 (s, 1H) ppm.

### Beispiel 35

### 6-(2-Methylbutyl)-1-(2-methylphenyl)-1,5-dihydro-4H-pyrazolo[3,4-d]pyrimidin-4-on (Racemat)

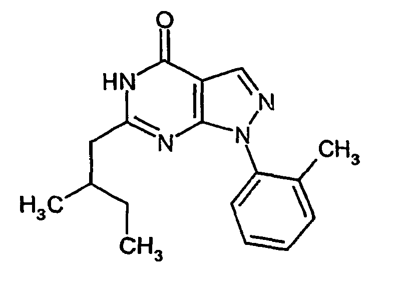

Analog zur Herstellung von Beispiel 1 werden ausgehend von 0.8 g (3.7 mmol) 5-Amino-1-(2-methylphenyl)-1H-pyrazol-4-carbonsäureamid (Beispiel 23A), 2.72 g (98% Reinheit, 18.5 mmol) 3-Methylvaleriansäureethylester und 0.740 g (24 mmol) 60%-igem Natriumhydrid 784 mg (71% d.Th.) des gewünschten Produktes als farbloser Feststoff erhalten.

Fp.: 132°C

MS (ESI pos): m/z = 297 (M+H)⁺

¹H-NMR (300 MHz, DMSO-d₆): δ = 0.8 (m, 6H), 1.1-1.4 (m, 2H), 1.9 (m, 1H), 2.1 (s, 3H), 2.4 (dd, 1H), 2.55 (dd, 1H), 7.3-7.5 (m, 4H), 8.2 (s, 1H), 12.2 (s, 1H) ppm.

### Beispiel 35-1

### 6-(2-Methylbutyl)-1-(2-methylphenyl)-1,5-dihydro-4H-pyrazolo[3,4-d]pyrimidin-4-on (Enantiomer I)

Das Racemat aus Beispiel 35 (380 mg) wird mittels HPLC an einer chiralen stationären Phase [basierend auf dem chiralen Selektor Poly(N-methacryloyl-L-leucin-L-menthylamid), zum Prinzip der Herstellung und Verwendung siehe EP-A-379 917; 380 mm x 100 mm Säule, Fluss 100 ml/min, Temperatur 24°C, Laufmittel: iso-Hexan / Ethylacetat 20:80] in die Enantiomeren getrennt. Beispiel 35-1 ist das unter diesen Bedingungen schneller eluierende Enantiomer I (Rₜ = 15.2 min).

Fp.: 122°C

### Beispiel 35-2

### 6-(2-Methylbutyl)-1-(2-methylphenyl)-1,5-dihydro-4H-pyrazolo[3,4-d]pyrimidin-4-on (Enantiomer II)

Das Racemat aus Beispiel 35 (380 mg) wird mittels HPLC an einer chiralen stationären Phase [basierend auf dem chiralen Selektor Poly(N-methacryloyl-L-leucin-L-menthylamid), zum Prinzip der Herstellung und Verwendung siehe EP-A-379 917; 380 mm x 100 mm Säule, Fluss 100 ml/min, Temperatur 24°C, Laufmittel: iso-Hexan / Ethylacetat 20:80] in die Enantiomeren getrennt. Beispiel 35-2 ist das unter diesen Bedingungen langsamer eluierende Enantiomer II (Rₜ = 18.1 min).

Fp.: 122°C

### Beispiel 36

### 1-(2-Methylphenyl)-6-(3,3,3-trifluor-2-methylpropyl)-1,5-dihydro-4H-pyrazolo[3,4-d]pyrimidin-4-on (Racemat)

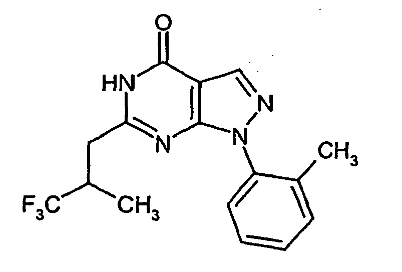

Analog zur Herstellung von Beispiel 1 werden ausgehend von 0.2 g (0.92 mmol) 5-Amino-1-(2-methylphenyl)-1H-pyrazol-4-carbonsäureamid (Beispiel 23A), 0.852 g (4.62 mmol) 3-Methyl-4,4,4-trifluorbuttersäureethylester und 0.129 g (3.24 mmol) 60%-igem Natriumhydrid 216 mg (69% d.Th.) des gewünschten Produktes als farbloser Feststoff erhalten.

Fp.: 160°C

MS (ESI pos): m/z = 337 (M+H)⁺

¹H-NMR (400 MHz, DMSO-d₆): δ = 1.1 (d, 3H), 2.1 (s, 3H), 2.7 (dd, 1H), 2.85-3.0 (m, 2H), 7.3-7.5 (m, 4H), 8.3 (s, 1H), 12.4 (s, 1H) ppm.

### Beispiel 36-1

### 1-(2-Methylphenyl)-6-(3,3,3-trifluor-2-methylpropyl)-1,5-dihydro-4H-pyrazolo[3,4-d]pyrimidin-4-on (Enantiomer I)

Das Racemat aus Beispiel 36 (180 mg) wird mittels HPLC an einer chiralen stationären Phase (Säule: Chiralpak AD, 250 mm x 20 mm; Fluss: 20 ml/min; Temperatur: 24°C; Laufmittel: iso-Hexan / iso-Propanol 92:8) in die Enantiomeren getrennt. Beispiel 36-1 ist das unter diesen Bedingungen schneller eluierende Enantiomer I (Rₜ = 10.37 min).

Fp.: 154°C

### Beispiel 36-2

### 1-(2-Methylphenyl)-6-(3,3,3-trifluor-2-methylpropyl)-1,5-dihydro-4H-pyrazolo[3,4-d]pyrimidin-4-on (Enantiomer II)

Das Racemat aus Beispiel 36 (180 mg) wird mittels HPLC an einer chiralen stationären Phase (Säule: Chiralpak AD, 250 mm x 20 mm; Fluss: 20 ml/min; Temperatur: 24°C; Laufmittel: iso-Hexan / iso-Propanol 92:8) in die Enantiomeren getrennt. Beispiel 36-2 ist das unter diesen Bedingungen langsamer eluierende Enantiomer II (Rₜ = 11.73 min).

Fp.: 153°C

### Beispiel 37

### 1-(2-Chlorphenyl)-6-(3,3,3-trifluor-2-methylpropyl)-1,5-dihydro-4H-pyrazolo[3,4-d]pyrimidin-4-on (Racemat)

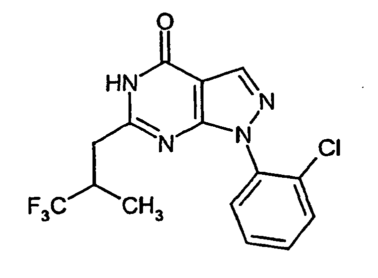

Analog zur Herstellung von Beispiel 1 werden ausgehend von 0.3 g (1.27 mmol) 5-Amino-1-(2-chlorphenyl)-1H-pyrazol-4-carbonsäureamid (Beispiel 27A), 1.17 g (6.34 mmol) 3-Methyl-4,4,4-trifluorbuttersäureethylester und 0.254 g (6.34 mmol) 60%-igem Natriumhydrid 321 mg (69% d.Th.) des gewünschten Produktes als farbloser Feststoff erhalten.

Fp.: 166°C

MS (ESI pos): m/z = 357 (M+H)⁺

¹H-NMR (400 MHz, DMSO-d₆): δ = 1.1 (d, 3H), 2.7 (dd, 1H), 2.85-3.0 (m, 2H), 7.5-7.8 (m, 4H), 8.3 (s, 1H), 12.4 (s, 1H) ppm.

### Beispiel 37-1

### 1-(2-Chlorphenyl)-6-(3,3,3-trifluor-2-methylpropyl)-1,5-dihydro-4H-pyrazolo[3,4-d]pyrimidin-4-on (Enantiomer I)

Das Racemat aus Beispiel 37 (240 mg) wird mittels HPLC an einer chiralen stationären Phase (Säule: Chiralpak AD, 250 mm x 20 mm; Fluss: 20 ml/min; Temperatur: 24°C; Laufmittel: iso-Hexan / iso-Propanol 92:8) in die Enantiomeren getrennt. Beispiel 37-1 ist das unter diesen Bedingungen schneller eluierende Enantiomer I (Rₜ = 11.92 min).

Fp.: 220°C

### Beispiel 37-2

### 1-(2-Chlorphenyl)-6-(3,3,3-trifluor-2-methylpropyl)-1,5-dihydro-4H-pyrazolo[3,4-d]pyrimidin-4-on (Enantiomer II)

Das Racemat aus Beispiel 37 (240 mg) wird mittels HPLC an einer chiralen stationären Phase (Säule: Chiralpak AD, 250 mm x 20 mm; Fluss: 20 ml/min; Temperatur: 24°C; Laufmittel: iso-Hexan / iso-Propanol 92:8) in die Enantiomeren getrennt. Beispiel 37-2 ist das unter diesen Bedingungen langsamer eluierende Enantiomer II (Rₜ = 12.67 min).

Fp.: 218°C

### Beispiel 38

### 6-Cyclopentylmethyl-1-(4-methylpyridin-3-yl)-1,5-dihydro-4H-pyrazolo[3,4-d]pyrimidin-4-on

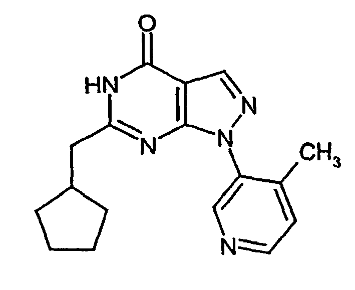

Analog zur Herstellung von Beispiel 1 werden ausgehend von 0.1 g (0.45 mmol) 5-Amino-1-(4-methylpyridin-3-yl)-1H-pyrazol-4-carbonsäureamid (Beispiel 35A), 0.353 g (2.26 mmol) Cyclopentylessigsäureethylester und 0.09 g (2.26 mmol) 60%-igem Natriumhydrid 102 mg (73% d.Th.) des gewünschten Produktes als farbloser Feststoff erhalten.

Fp.: 206°C

MS (ESI pos): m/z = 310 (M+H)⁺

¹H-NMR (400 MHz, DMSO-d₆): δ = 1.1-1.8 (m, 8H), 2.2 (s, 3H), 2.22 (m, 1H), 2.6 (d, 2H), 7.5 (d, 1H), 8.3 (s, 1H), 8.6 (m, 2H), 12.3 (s, 1H) ppm.

### Beispiel 39

### 6-(2-Methylbutyl)-1-(4-methylpyridin-3-yl)-1,5-dihydro-4H-pyrazolo[3,4-d]pyrimidin-4-on (Racemat)

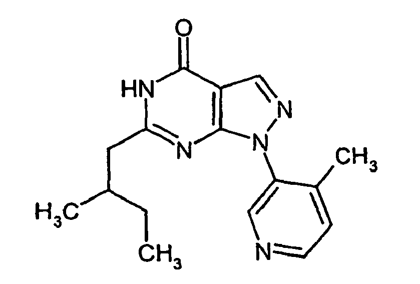

Analog zur Herstellung von Beispiel 1 werden ausgehend von 0.2 g (0.92 mmol) 5-Amino-1-(4-methylpyridin-3-yl-1H-pyrazol-4-carbonsäureamid (Beispiel 35A), 0.677 g (4.6 mmol) 3-Methylvaleriansäureethylester und 0.184 g (4.6 mmol) 60%-igem Natriumhydrid 186 mg (68% d.Th.) des gewünschten Produktes als farbloser Feststoff erhalten.

Fp.: 149°C

MS (ESI pos): m/z = 298 (M+H)⁺

¹H-NMR (400 MHz, DMSO-d₆): δ = 0.8 (m, 6H), 1.1-1.4 (m, 2H), 1.9 (m, 1H), 2.2 (s, 3H), 2.4 (dd, 1H), 2.6 (dd, 1H), 7.5 (d, 1H), 8.3 (s, 1H), 8.6 (m, 2H), 12.25 (s, 1H) ppm.

### Beispiel 39-1

### 6-(2-Methylbutyl)-1-(4-methylpyridin-3-yl)-1,5-dihydro-4H-pyrazolo[3,4-d]pyrimidin-4-on (Enantiomer I)

Das Racemat aus Beispiel 39 (160 mg) wird mittels HPLC an einer chiralen stationären Phase [basierend auf dem chiralen Selektor Poly(N-methacryloyl-L-leucin-L-menthylamid), zum Prinzip der Herstellung und Verwendung siehe EP-A-379 917; 380 mm x 75 mm Säule, Fluss 100 ml/min, Temperatur 24°C, Laufmittel: iso-Hexan / Ethylacetat 30:70] in die Enantiomeren getrennt. Beispiel 39-1 ist das unter diesen Bedingungen schneller eluierende Enantiomer I.

Fp.: 149°C

Rₜ = 7.25 min [chiraler Selektor Poly(N-methacryloyl-L-leucin-L-menthylamid), 250 mm x 4.6 mm Säule; Fluss 1 ml/min; Temperatur 24°C; Laufmittel Ethylacetat].

### Beispiel 39-2

### 6-(2-Methylbutyl)-1-(4-methylpyridin-3-yl)-1,5-dihydro-4H-pyrazolo[3,4-d]pyrimidin-4-on (Enantiomer II)

Das Racemat aus Beispiel 39 (160 mg) wird mittels HPLC an einer chiralen stationären Phase [basierend auf dem chiralen Selektor Poly(N-methacryloyl-L-leucin-L-menthylamid), zum Prinzip der Herstellung und Verwendung siehe EP-A-379 917; 380 mm x 75 mm Säule, Fluss 100 ml/min, Temperatur 24°C, Laufmittel: iso-Hexan / Ethylacetat 30:70] in die Enantiomeren getrennt. Beispiel 39-2 ist das unter diesen Bedingungen langsamer eluierende Enantiomer II.

Fp.: 148°C

Rₜ = 8.0 min [chiraler Selektor Poly(N-methacryloyl-L-leucin-L-menthylamid), 250 mm x 4.6 mm Säule; Fluss 1 ml/min; Temperatur 24°C; Laufmittel Ethylacetat].

### Beispiel 40

### 1-(2-Chlorphenyl)-6-(2-methylpropyl)-1,5-dihydro-4H-pyrazolo[3,4-d]pyrimidin-4-on

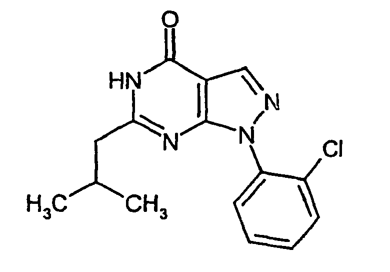

Analog zur Herstellung von Beispiel 1 werden ausgehend von 0.1 g (0.42 mmol) 5-Amino-1-(2-chlorphenyl)-1H-pyrazol-4-carbonsäureamid (Beispiel 27A), 0.344 g (2.96 mmol) 3-Methylbuttersäureethylester und 0.059 g (1.48 mmol) 60%-igem Natriumhydrid 57 mg (45% d.Th.) des gewünschten Produktes als farbloser Feststoff erhalten.

Fp.: 204°C

MS (ESI pos): m/z = 303 (M+H)⁺

¹H-NMR (200 MHz, DMSO-d₆): δ = 0.9 (d, 6H), 2.05 (m, 1H), 2.45 (d, 2H), 7.5-7.8 (m, 4H), 8.3 (s, 1H), 12.3 (s, 1H) ppm.

### Beispiel 41

### 6-(2-Ethylbutyl)-1-(4-methylpyridin-3-yl)-1,5-dihydro-4H-pyrazolo[3,4-d]pyrimidin-4-on

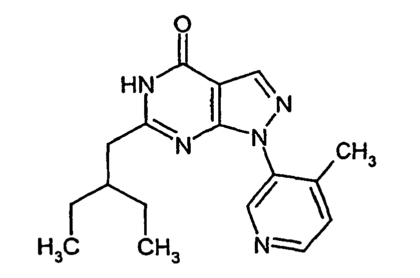

Analog zur Herstellung von Beispiel 1 werden ausgehend von 0.08 g (0.37 mmol) 5-Amino-1-(4-methylpyridin-3-yl)-1H-pyrazol-4-carbonsäureamid (Beispiel 35A), 0.303 g (1.84 mmol) 3-Ethylvaleriansäureethylester und 0.074 g (1.84 mmol) 60%-igem Natriumhydrid 56 mg (49% d.Th.) des gewünschten Produktes als farbloser Feststoff erhalten.

Fp.: 143°C

MS (ESI pos): m/z = 312 (M+H)⁺

¹H-NMR (200 MHz, DMSO-d₆): δ = 0.8 (t, 6H), 1.3 (m, 4H), 1.8 (m, 1H), 2.2 (s, 3H), 2.5 (d, 2H), 7.5 (d, 1H), 8.3 (s, 1H), 8.6 (m, 2H), 12.3 (s, 1H) ppm.

### Beispiel 42

### 6-Cyclopentylmethyl-1-(4-methyl-1-oxidopyridin-3-yl)-1,5-dihydro-4H-pyrazolo[3,4-d]pyrimidin-4-on

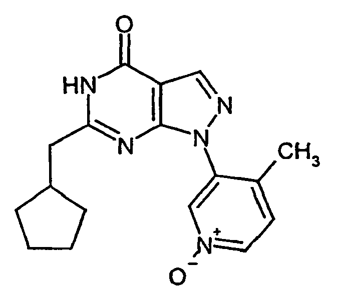

Eine Lösung von 40 mg (0.13 mmol) 6-Cyclopentylmethyl-1-(4-methylpyridin-3-yl)-1,5-dihydro-4H-pyrazolo[3,4-d]pyrimidin-4-on (Beispiel 38) in 2 ml Dichlormethan wird bei Raumtemperatur mit 48 mg (70% Reinheit, 0.195 mmol) meta-Chlorperbenzoesäure versetzt und über Nacht gerührt. Anschließend wird für 1.5 h bei 40°C gerührt, bis die Umsetzung laut Reaktionskontrolle (DC) vollständig ist. Zur Aufarbeitung wird mit gesättigter Natriumhydrogencarbonat-Lösung versetzt und die Mischung dreimal mit Dichlormethan extrahiert. Die vereinigten organischen Phasen werden über Natriumsulfat getrocknet und eingeengt. Das Rohprodukt wird über präparative HPLC gereinigt. Man erhält 32 mg (76% d.Th.) des gewünschten Produkts als farblosen Feststoff.

MS (ESI pos): m/z = 310 (M+H)⁺

¹H-NMR (400 MHz, DMSO-d₆): δ = 1.1-1.8 (m, 8H), 2.2 (s, 3H), 2.22 (m, 1H), 2.6 (d, 2H), 7.5 (d, 1H), 8.3 (s, 1H), 8.6 (m, 2H), 12.3 (s, 1H) ppm.

### Beispiel 43

### 6-Cyclohexylmethyl-1-(4-methylpyridin-3-yl)-1,5-dihydro-4H-pyrazolo[3,4-d]pyrimidin-4-on

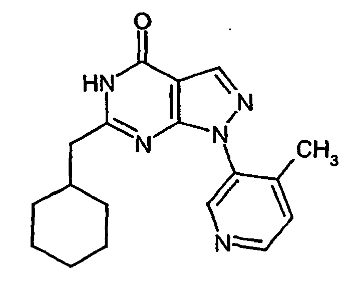

Analog zur Herstellung von Beispiel 1 werden ausgehend von 0.08 g (0.37 mmol) 5-Amino-1-(4-methylpyridin-3-yl)-1H-pyrazol-4-carbonsäureamid (Beispiel 35A), 0.32 g (1.84 mmol) Cyclohexylessigsäureethylester und 0.074 g (1.84 mmol) 60%-igem Natriumhydrid 68 mg (73% d.Th.) des gewünschten Produktes als farbloser Feststoff erhalten.

Fp.: 206°C

MS (ESI pos): m/z = 324 (M+H)⁺

¹H-NMR (400 MHz, DMSO-d₆): δ = 0.8-1.3 (m, 6H), 1.5-1.9 (m, 5H), 2.2 (s, 3H), 2.5 (d, 2H), 7.5 (d, 1H), 8.3 (s, 1H), 8.6 (m, 2H), 12.25 (s, 1H) ppm.

Die erfindungsgemäßen Verbindungen können folgendermaßen in pharmazeutische Zubereitungen überführt werden:

### Tablette:

### Zusammenfassung:

100 mg der erfindungsgemäßen Verbindung, 50 mg Lactose (Monohydrat) 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg, Durchmesser 8 mm, Wölbungsradius 12 mm.

### Herstellung:

Die Mischung aus erfindungsgemäßer Verbindung, Lactose und Stärke wird mit einer 5 %igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96 %), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugeführt. Unter Rühren erfolgt die Zugabe des Wassers. Bis zum Abschluß der Quellung des Rhodigels wird ca. 6 h gerührt.

### Oral applizierbare Lösung:

### Zusammenfassung:

500 mg der erfindungsgemäßen Verbindung, 2,5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5 % und/oder PEG 400 Lösung 30 %. Die Lösung wird steril filtriert und sterile und pyrogenfreie Injektionsbehältnisse aufgefüllt.

### Intravenös applizierbare Lösung:

### Zusammensetzung:

1 mg der erfindungsgemäßen Verbindung, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfilriert (Porendurchmesser 0,22 µm) und unter raseptischen Bedingungen in hitzesterilisierte Infusionsflaschen abgefüllt. Diese werden mit Infusionsstopfen und Bordelkappen verschlossen.

## Patentansprüche

1. Verbindungen der Formel 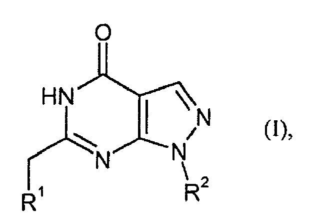 in welcher
R¹ C₁-C₈-Alkyl, C₂-C₆-Alkenyl, C₂-C₆-Alkinyl oder C₃-C₈-Cycloalkyl, wobei C₁-C₈- Alkyl gegebenenfalls mit Oxo substituiert ist, und
wobei C₁-C₈-Alkyl, C₂-C₆-Alkenyl, C₂-C₆-Alkinyl und C₃-C₈-Cycloalkyl ge- gebenenfalls mit bis zu 3 Resten unabhängig voneinander ausgewählt aus der Gruppe C₁-C₆-Alkyl, C₁-C₆-Alkoxy, Hydroxycarbonyl, Cyano, Amino, Nitro, Hydroxy, C₁-C₆- Alkylamino, Halogen, Trifluormethyl, Trifluormethoxy, C₆-C₁₀-Arylcarbonylamino, C₁-C₆-Alkylcarbonylamio, C₁-C₆-Alkylaminocarbonyl, C₁-C₆-Alkoxycarbonyl, C₆- C₁₀-Arylaminocarbonyl, Heteroarylaminocarbonyl, Heteroarylcarbonylamino, C₁-C₆- Alkylsulfonylamino, C₁-C₆-Alkylsulfonyl, C₁-C₆-Alkylthio substituiert sind,
wobei
C₁-C₆-Alkyl, C₁-C₆-Alkoxy, C₁-C₆-Alkylamino, C₆-C₁₀-Arylcarbonylamino, C₁-C₆- Alkylcarbonylamino, C₁-C₆-Alkylaminocarbonyl, C₁-C₆-Alkoxycarbonyl, C₆- C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit ein bis drei Resten unabhängig voneinander ausgewählt aus der Gruppe Hydroxy, Cyano, Halogen, Trifluormethyl, Trifluormethoxy, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8- gliedriges Heterocyclyl bedeuten,
substituiert sind,
R² Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆-Alkyl, C₁-C₆-Alkoxy, Hydroxycarbonyl, Cyano, Trifluormethyl, Trifluormethoxy, Amino, Nitro, Hydroxy, C₁-C₆-Alkylamino, Halogen, C₆-C₁₀- Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Hetero- arylcarbonylamino, C₁-C₆-Alkylsulfonylamino, C₁-C₆-Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆-Alkoxy- carbonyl, C₆-C₁₀-Arylaminocarbonyl, Heteroarylaminocarbonyl, Hetero- arylcarbonylamino, C₁-C₆-Alkylsulfonylamino, C₁-C₆-Alkylsulfonyl und C₁- C₆-Alkylthio gegebenenfalls mit ein bis drei Resten unabhängig voneinander ausgewählt aus der Gruppe Hydroxy, Cyano, Halogen, Trifluormethyl, Trifluormethoxy, Hydroxycarbonyl und einer Gruppe der Formel -NR³R⁴, wobei
R³ und R⁴ die oben angegebenen Bedeutungen aufweisen,
substituiert sind,
bedeuten, sowie deren Salze, Solvate und/oder Solvate der Salze,
wobei nachfolgende Verbindungen ausgenommen sind:

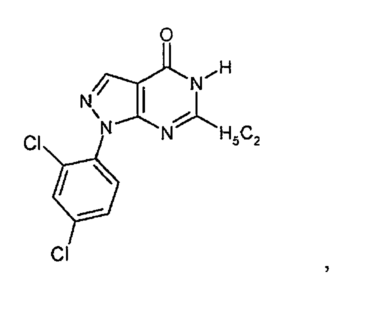

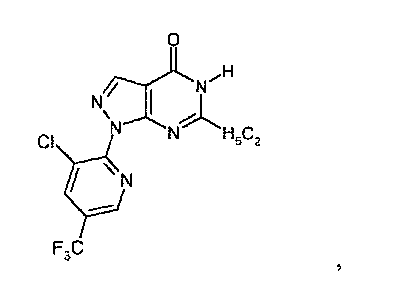

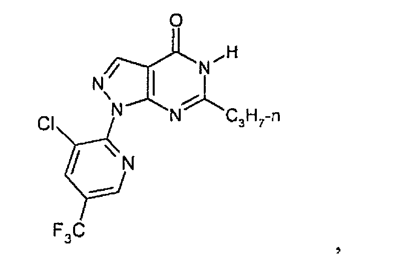

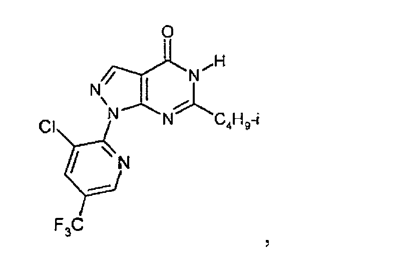

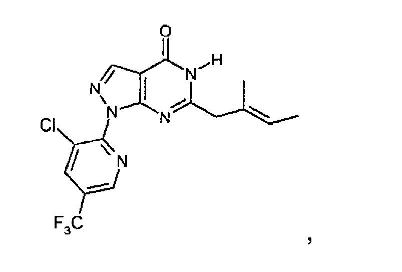

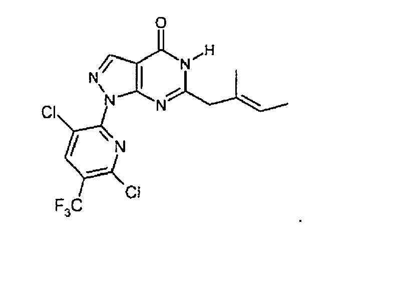

2. Verbindungen nach Anspruch 1, wobei
R¹ C₁-C₈-Alkyl, C₂-C₆-Alkenyl, C₂-C₆-Alkinyl oder C₃-C₈-Cycloalkyl, welche gegebenenfalls mit bis zu 3 Resten unabhängig voneinander ausgewählt aus der Gruppe C₁-C₆-Alkyl, C₁-C₆-Alkoxy, Hydroxycarbonyl, Cyano, Amino, Nitro, Hydroxy, C₁-C₆-Alkylamino, Halogen, C₆-C₁₀-Arylcarbonylamino, C₁-C₆-Alkyl- carbonylamino, C₁-C₆-Alkylaminocarbonyl, C₁-C₆-Alkoxycarbonyl, C₆-C₁₀-Aryl- aminocarbonyl, Heteroarylaminocarbonyl, Heteroarylcarbonylamino, C₁-C₆-Alkyl- sulfonylamino, C₁-C₆-Alkylsulfonyl, C₁-C₆-Alkylthio substituiert sind, wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8-gliedriges Heterocyclyl bedeuten,
substituiert sind,
R² Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆-Alkyl, C₁-C₆-Alkoxy, Hydroxycarbonyl, Cyano, Trifluormethyl, Amino, Nitro, Hydroxy, C₁-C₆-Alkylamino, Halogen, C₆-C₁₀-Arylcarbonylamino, C₁- C₆-Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl, C₁-C₆-Alkylthio substituiert sind, wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei
R³ und R⁴ die oben angegebenen Bedeutungen aufweisen,
substituiert sind,
bedeuten, sowie deren Salze, Solvate und/oder Solvate der Salze.

3. Verbindungen nach Ansprüchen 1 und 2, wobei
R¹ C₁-C₅-Alkyl oder C₃-C₆-Cycloalkyl, welche gegebenenfalls mit bis zu 3 Resten unabhängig voneinander ausgewählt aus der Gruppe C₁-C₄-Alkyl, C₁-C₄-Alkoxy, Hydroxycarbonyl, Cyano, Amino, Hydroxy, C₁-C₄-Alkylamino, Trifluomethyl, Fluor, Chlor, Brom, C₆-C₁₀-Arylcarbonylamino, C₁-C₄-Alkylcarbonylamino, C₁-C₄- Alkylaminocarbonyl, C₁-C₄-Alkoxycarbonyl, C₆-C₁₀-Arylaminocarbonyl, Heteroarylaminocarbonyl, Heteroarylcarbonylamino, C₁-C₄-Alkylsulfonylamino, C₁- C₄-Alkylsulfonyl, C₁-C₄-Alkylthio substituiert sind, wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ unabhängig voneinander Wasserstoff oder C₁-C₄-Alkyl,
oder
R³ und R⁴ zusammen mit dem Stickstoffatom, an das sie gebunden sind, 5- bis 6-gliedriges Heterocyclyl bedeuten,
substituiert sind,
R² Phenyl, Pyrimidyl, N-Oxidopyridyl oder Pyridyl, wobei Phenyl mit 1 bis 3 Resten und Pyrimidyl, N-Oxidopyridyl und Pyridyl gegebenenfalls mit 1 bis 3 Resten jeweils un- abhängig voneinander ausgewählt aus der Gruppe C₁-C₄-Alkyl, C₁-C₄-Alkoxy, Hydroxycarbonyl, Cyano, Trifluormethyl, Amino, Hydroxy, C₁-C₄-Alkylamino, Fluor, Chlor, Brom, C₆-C₁₀-Arylcarbonylamino, C₁-C₄-Alkylcarbonylamino, C₁-C₄- Alkylaminocarbonyl, C₁-C₄-Alkoxycarbonyl, C₆-C₁₀-Arylaminocarbonyl, Heteroarylaminocarbonyl, Heteroarylcarbonylamino, C₁-C₄-Alkylsulfonylamino, C₁- C₄-Alkylsulfonyl, C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ die in Anspruch 1 angegebenen Bedeutungen aufweisen,
substituiert sind,
bedeuten, sowie deren Salze, Solvate und/oder Solvate der Salze.

4. Verbindungen nach Ansprüchen 1 bis 3, wobei R¹ die in Ansprüchen 1 bis 3 angegebenen Bedeutungen aufweist und
R² Phenyl, N-Oxidopyridyl oder Pyridyl, wobei Phenyl mit 1 bis 3 Resten und Pyridyl und N-Oxidopyridyl gegebenenfalls mit 1 bis 3 Resten jeweils unabhängig voneinander ausgewählt aus der Gruppe Methyl, Ethyl, 2-Propyl, Trifluormethyl, Methoxy, Ethoxy, Fluor und Chlor substituiert sind,
bedeutet, sowie deren Salze, Solvate und/oder Solvate der Salze.

5. Verbindungen nach Ansprüchen 1 bis 4, wobei
R¹ C₁-C₅-Alkyl oder C₅-C₆-Cycloalkyl, welche gegebenenfalls mit bis zu 3 Resten unabhängig voneinander ausgewählt aus der Gruppe C₁-C₄-Alkyl, Trifluormethyl, Fluor, Hydroxy, Phenylcarbonylamino, C₁-C₄-Alkylcarbonylamino, C₁-C₄- Alkylaminocarbonyl oder Phenylaminocarbonyl substituiert sind,
R² Phenyl, N-Oxidopyridyl oder Pyridyl, wobei Phenyl mit 1 bis 3 Resten und Pyridyl und N-Oxidopyridyl gegebenenfalls mit 1 bis 3 Resten jeweils unabhängig voneinander ausgewählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze.

6. Verbindungen nach Ansprüchen 1 bis 5, wobei
R¹ C₁-C₅-Alkyl oder C₅-C₆-Cycloalkyl, welche gegebenenfalls mit bis zu 3 Resten unabhängig voneinander ausgewählt aus der Gruppe C₁-C₄-Alkyl, Fluor, Trifluormethyl, Hydroxy, Phenylcarbonylamino, C₁-C₄-Alkylcarbonylamino, C₁-C₄- Alkylaminocarbonyl oder Phenylaminocarbonyl substituiert sind, und
R² Phenyl, N-Oxidopyridyl oder Pyridyl, wobei Phenyl mit einem Rest und Pyridyl und N-Oxidopyridyl gegebenenfalls mit einem Rest jeweils unabhängig voneinander aus- gewählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze.

7. Verbindungen der Formel 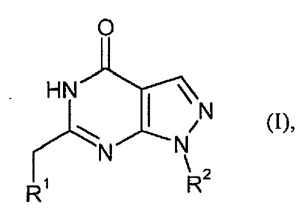 in welcher
R¹ C₁-C₈-Alkyl, C₂-C₆-Alkenyl, C₂-C₆-Alkinyl oder C₃-C₈-Cycloalkyl, wobei C₁-C₈- Alkyl gegebenenfalls mit Oxo substituiert ist, und wobei C₁-C₈-Alkyl, C₂-C₆-Alkenyl, C₂-C₆-Alkinyl und C₃-C₈-Cycloalkyl ge- gebenenfalls mit bis zu 3 Resten unabhängig voneinander ausgewählt aus der Gruppe C₁-C₆-Alkyl, C₁-C₆-Alkoxy, Hydroxycarbonyl, Cyano, Amino, Nitro, Hydroxy, C₁-C₆- Alkylamino, Halogen, Trifluormethyl, Trifluormethoxy, C₆-C₁₀-Arylcarbonylamino, C₁-C₆-Alkylcarbonylamino, C₁-C₆-Alkylaminocarbonyl, C₁-C₆-Alkoxycarbonyl, C₆- C₁₀-Arylaminocarbonyl, Heteroarylaminocarbonyl, Heteroarylcarbonylamino, C₁-C₆- Alkylsulfonylamino, C₁-C₆-Alkylsulfonyl, C₁-C₆-Alkylthio substituiert sind,
wobei
C₁-C₆-Alkyl, C₁-C₆-Alkoxy, C₁-C₆-Alkylamino, C₆-C₁₀-Arylcarbonylamino, C₁-C₆- Alkylcarbonylamino, C₁-C₆-Alkylaminocarbonyl, C₁-C₆-Alkoxycarbonyl, C₆- C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit ein bis drei Resten unabhängig voneinander ausgewählt aus der Gruppe Hydroxy, Cyano, Halogen, Trifluormethyl, Trifluormethoxy, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8- gliedriges Heterocyclyl bedeuten,
substituiert sind,
R² Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆-Alkyl, C₁-C₆-Alkoxy, Hydroxycarbonyl, Cyano, Trifluormethyl, Trifluormethoxy, Amino, Nitro, Hydroxy, C₁-C₆-Alkylamino, Halogen, C₆-C₁₀- Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Hetero- arylcarbonylamino, C₁-C₆-Alkylsulfonylamino, C₁-C₆-Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆-Alkoxy- carbonyl, C₆-C₁₀-Arylaminocarbonyl, Heteroarylaminocarbonyl, Hetero- arylcarbonylamino, C₁-C₆-Alkylsulfonylamino, C₁-C₆-Alkylsulfonyl und C₁- C₆-Alkylthio gegebenenfalls mit ein bis drei Resten unabhängig voneinander ausgewählt aus der Gruppe Hydroxy, Cyano, Halogen, Trifluormethyl, Trifluormethoxy, Hydroxycarbonyl und einer Gruppe der Formel-NR³R⁴,
wobei
R³ und R⁴ die oben angegebenen Bedeutungen aufweisen,
substituiert sind,
bedeuten, sowie deren Salze, Solvate und/oder Solvate der Salze
zur Behandlung und/oder Prophylaxe von Krankheiten bei Menschen.

8. Verbindungen nach Anspruch 7, wobei
R¹ C₁-C₈-Alkyl, C₂-C₆-Alkenyl, C₂-C₆-Alkinyl oder C₃-C₈-Cycloalkyl, welche gegebenenfalls mit bis zu 3 Resten unabhängig voneinander ausgewählt aus der Gruppe C₁-C₆-Alkyl, C₁-C₆-Alkoxy, Hydroxycarbonyl, Cyano, Amino, Nitro, Hydroxy, C₁-C₆-Alkylamino, Halogen, C₆-C₁₀-Arylcarbonylamino, C₁-C₆-Alkyl- carbonylamino, C₁-C₆-Alkylaminocarbonyl, C₁-C₆-Alkoxycarbonyl, C₆-C₁₀-Aryl- aminocarbonyl, Heteroarylaminocarbonyl, Heteroarylcarbonylamino, C₁-C₆-Alkyl- sulfonylamino, C₁-C₆-Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8-gliedriges Heterocyclyl bedeuten,
substituiert sind,
R² Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆-Alkyl, C₁-C₆-Alkoxy, Hydroxycarbonyl, Cyano, Trifluormethyl, Amino, Nitro, Hydroxy, C₁-C₆-Alkylamino, Halogen, C₆-C₁₀-Arylcarbonylamino, C₁- C₆-Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei
R³ und R⁴ die oben angegebenen Bedeutungen aufweisen,
substituiert sind,
bedeuten, sowie deren Salze, Solvate und/oder Solvate der Salze
zur Behandlung und/oder Prophylaxe von Krankheiten bei Menschen.

9. Verbindungen nach einem der Ansprüchen 7 oder 8, wobei
R¹ C₁-C₅-Alkyl oder C₃-C₆-Cycloalkyl, welche gegebenenfalls mit bis zu 3 Resten unabhängig voneinander ausgewählt aus der Gruppe C₁-C₄-Alkyl, C₁-C₄-Alkoxy, Hydroxycarbonyl, Cyano, Amino, Hydroxy, C₁-C₄-Alkylamino, Trifluormethyl, Fluor, Chlor, Brom, C₆-C₁₀-Arylcarbonylamino, C₁-C₄-Alkylcarbonylamino, C₁-C₄- Alkylaminocarbonyl, C₁-C₄-Alkoxycarbonyl, C₆-C₁₀-Arylaminocarbonyl, Heteroarylaminocarbonyl, Heteroarylcarbonylamino, C₁-C₄-Alkylsulfonylamino, C₁- C₄-Alkylsulfonyl, C,-C₄-Alkyithio substituiert sind, wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴, wobei
R³ und R⁴ unabhängig voneinander Wasserstoff oder C₁-C₄-Alkyl,
oder
R³ und R⁴ zusammen mit dem Stickstoffatom, an das sie gebunden sind, 5- bis 6-gliedriges Heterocyclyl bedeuten,
substituiert sind,
R² Phenyl, Pyrimidyl, N-Oxidopyridyl oder Pyridyl, wobei Phenyl mit 1 bis 3 Resten und Pyrimidyl, N-Oxidopyridyl und Pyridyl gegebenenfalls mit 1 bis 3 Resten jeweils un- abhängig voneinander ausgewählt aus der Gruppe C₁-C₄-Alkyl, C₁-C₄-Alkoxy, Hydroxycarbonyl, Cyano, Trifluormethyl, Amino, Hydroxy, C₁-C₄-Alkylamino, Fluor, Chlor, Brom, C₆-C₁₀-Arylcarbonylamino, C₁-C₄-Alkylcarbonylamino, C₁-C₄- Alkylaminocarbonyl, C₁-C₄-Alkoxycarbonyl, C₆-C₁₀-Arylaminocarbonyl, Heteroarylaminocarbonyl, Heteroarylcarbonylamino, C₁-C₄-Alkylsulfonylamino, C₁- C₄-Alkylsulfonyl, C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ die in Anspruch 7 angegebenen Bedeutungen aufweisen,
substituiert sind,
bedeuten, sowie deren Salze, Solvate und/oder Solvate der Salze
zur Behandlung und/oder Prophylaxe von Krankheiten bei Menschen.

10. Verbindungen nach einem der Ansprüche 7 b is 9, wobei R¹ die in Ansprüchen 7 bis 9 angegebenen Bedeutungen aufweist und
R² Phenyl, N-Oxidopyridyl oder Pyridyl, wobei Phenyl mit 1 bis 3 Resten und Pyridyl und N-Oxidopyridyl gegebenenfalls mit 1 bis 3 Resten jeweils unabhängig voneinander ausgewählt aus der Gruppe Methyl, Ethyl, 2-Propyl, Trifluormethyl, Methoxy, Ethoxy, Fluor und Chlor substituiert sind,
bedeutet, sowie deren Salze, Solvate und/oder Solvate der Salze
zur Behandlung und/oder Prophylaxe von Krankheiten bei Menschen.

11. Verbindungen nach einem der Ansprüche 7 bis 10, wobei
R¹ C₁-C₅-Alkyl oder C₅-C₆-Cycloalkyl, welche gegebenenfalls mit bis zu 3 Resten unabhängig voneinander ausgewählt aus der Gruppe C₁-C₄-Alkyl, Trifluormethyl, Fluor, Hydroxy, Phenylcarbonylamino, C₁-C₄-Alkylcarbonylamino, C₁-C₄- Alkylaminocarbonyl oder Phenylaminocarbonyl substituiert sind,
R² Phenyl, N-Oxidopyridyl oder Pyridyl, wobei Phenyl mit 1 bis 3 Resten und Pyridyl und N-Oxidopyridyl gegebenenfalls mit 1 bis 3 Resten jeweils unabhängig voneinander ausgewählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze
zur Behandlung und/oder Prophylaxe von Krankheiten bei Menschen.

12. Verbindungen nach einem der Ansprüche 7 bis 11, wobei
R¹ C₁-C₅-Alkyl oder C₅-C₆-Cycloalkyl, welche gegebenenfalls mit bis zu 3 Resten unabhängig voneinander ausgewählt aus der Gruppe C₁-C₄-Alkyl, Fluor, Trifluormethyl, Hydroxy, Phenylcarbonylamino, C₁-C₄-Alkylcarbonylamino, C₁-C₄- Alkylaminocarbonyl oder Phenylaminocarbonyl substituiert sind, und
R² Phenyl, N-Oxidopyridyl oder Pyridyl, wobei Phenyl mit einem Rest und Pyridyl und N-Oxidopyridyl gegebenenfalls mit einem Rest jeweils unabhängig voneinander aus- gewählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze
zur Behandlung und/oder Prophylaxe von Krankheiten bei Menschen.

13. Verfahren zur Herstellung von Verbindungen nach Anspruch 7, **dadurch gekennzeichnet, dass** man
[A] Verbindungen der Formel 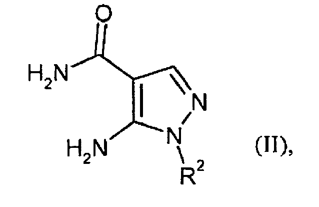 in welcher
R² die in Anspruch 7 angegebenen Bedeutungen hat,
durch Umsetzung mit einer Verbindung der Formel 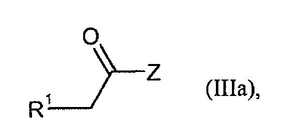 in welcher R¹ die in Anspruch 7 angegebenen Bedeutungen hat,
und
Z für Chlor oder Brom steht,
in einem inerten Lösemittel und in Anwesenheit einer Base zunächst in Verbindungen der Formel 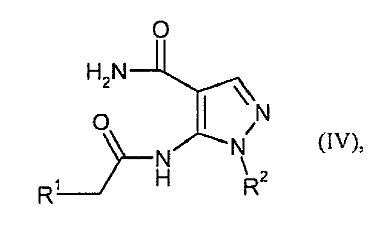 in welcher
R¹ und R² die in Anspruch 7 angegebenen Bedeutungen haben,
überführt, dann in einem inerten Lösemittel in Gegenwart einer Base zu Verbindungen der Formel (I) cyclisiert,
oder
[B] Verbindungen der Formel (II) unter direkter Cyclisierung zu (I) mit einer Verbindung der Formel 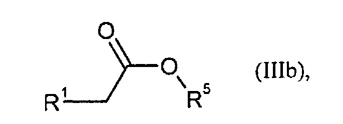 in welcher
R¹ die in Anspruch 7 angegebenen Bedeutungen hat
und
R⁵ für Methyl oder Ethyl steht,
in einem inerten Lösemittel und in Anwesenheit einer Base umsetzt,
oder
[C] Verbindungen der Formel 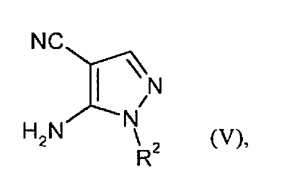 in welcher
R² die in Anspruch 7 angegebenen Bedeutungen hat,
zunächst durch Umsetzung mit einer Verbindung der Formel (IIIa) in einem inerten Lösemittel und in Anwesenheit einer Base in Verbindungen der Formel 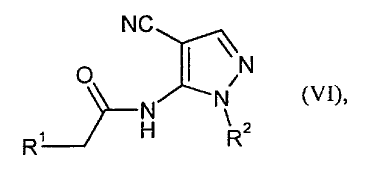 in welcher
R¹ und R² die in Anspruch 7 angegebenen Bedeutungen haben, überführt,
und diese in einem zweiten Schritt in einem inerten Lösemittel und in Anwesenheit einer Base und eines Oxidationsmittels zu (I) cyclisiert,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

14. Arzneimittel enthaltend mindestens eine der Verbindungen nach einem der Ansprüche 7 bis 12 und mindestens einen pharmazeutisch verträglichen, im wesentlichen nichtgiftigen Träger oder Exzipienten zur Behandlung und/oder Prophylaxe von Krankheiten bei Menschen.

15. Verwendung von Verbindungen nach einem der Ansprüche 7 bis 12 zur Herstellung von Arzneimitteln zur Behandlung von Krankheiten bei Menschen.

16. Verwendung der Verbindungen nach einem der Ansprüche 7 bis 12 zur Herstellung eines Arzneimittels zur Prophylaxe und/oder Behandlung von Störungen der Wahrnehmung, Konzentrationsleistung, Lern- und/oder Gedächtnisleistung.

17. Verwendung nach Anspruch 16, wobei die Störung eine Folge der Alzheimer'schen Krankheit ist.

18. Verwendung der Verbindungen nach einem der Ansprüche 7 bis 12 zur Herstellung eines Arzneimittels zur Verbesserung der Wahrnehmung, Konzentrationsleistung, Lern- und/oder Gedächtnisleistung.

19. Verwendung der Verbindungen nach einem der Ansprüche 7 bis 12 zur Herstellung eines Arzneimittels zur Inhibition von PDE9A.

## Claims

1. Compounds of formula 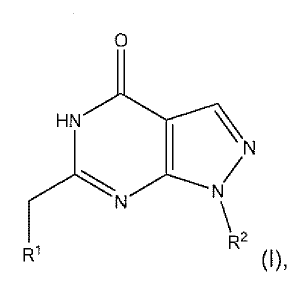 wherein
R¹ denotes C₁-C₈-alkyl, C₂-C₆-alkenyl, C₂-C₆-alkynyl or C₃-C₈-cycloalkyl, wherein C₁-C₈-alkyl is optionally substituted by oxo, and
wherein C₁-C₈-alkyl, C₂-C₆-alkenyl, C₂-C₆-alkynyl and C₃-C₈-cycloalkyl are optionally substituted by up to 3 groups selected independently of one another from among C₁-C₆-alkyl, C₁-C₆-alkoxy, hydroxycarbonyl, cyano, amino, nitro, hydroxy, C₁-C₆-alkylamino, halogen, trifluoromethyl, trifluoromethoxy, C₆-C₁₀-arylcarbonylamino, C₁-C₆-alkylcarbonylamino, C₁-C₆-alkylaminocarbonyl, C₁- C₆-alkoxycarbonyl, C₆-C₁₀- arylaminocarbonyl, heteroarylaminocarbonyl, heteroarylcarbonylamino, C₁-C₆-alkylsulphonylamino, C₁-C₆-alkylsulphonyl, C₁-C₆-alkylthio,
wherein
C₁-C₆-alkyl, C₁-C₆-alkoxy, C₁-C₆-alkylamino, C₆-C₁₀-arylcarbonylamino, C₁-C₆- alkylcarbonylamino, C₁-C₆-alkylaminocarbonyl, C₁-C₆- alkoxycarbonyl, C₆-C₁₀-arylaminocarbonyl, heteroarylaminocarbonyl, heteroarylcarbonylamino, C₁₋₆-alkylsulphonylamino, C₁-C₆-alkylsulphonyl and C₁-C₆-alkylthio are optionally substituted by one to three groups selected independently of one another from among hydroxy, cyano, halogen, trifluoromethyl, trifluoromethoxy, hydroxycarbonyl and a group of formula -NR³R⁴,
wherein
R³ and R⁴ independently of one another denote hydrogen or C₁-C₆-alkyl,
or
R³ and R⁴, together with the nitrogen atom to which they are bound, represent 5- to 8-membered heterocyclyl,
R² denotes phenyl or heteroaryl, wherein phenyl is substituted by 1 to 3 groups and heteroaryl is optionally substituted by 1 to 3 groups, each independently selected from among C₁-C₆-alkyl, C₁-C₆-alkoxy, hydroxycarbonyl, cyano, trifluoromethyl, trifluoromethoxy, amino, nitro, hydroxy, C₁-C₆-alkylamino, halogen, C₆-C₁₀-arylcarbonylamino, C₁-C₆-alkylcarbonylamino, C₁-C₆- alkylaminocarbonyl, C₁-C₆-alkoxycarbonyl, C₆-C₁₀-arylaminocarbonyl, heteroarylaminocarbonyl, heteroarylcarbonylamino, C₁-C₆- alkylsulphonylamino, C₁-C₆-alkylsulphonyl, C₁-C₆-alkylthio,
wherein C₁-C₆-alkyl, C₁-C₆-alkoxy, C₁-C₆-alkylamino, C₆-C₁₀-arylcarbonyl- amino, C₁-C₆-alkylcarbonylamino, C₁-C₆-alkylaminocarbonyl, C₁-C₆- alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio are optionally substituted by one to three groups selected independently of one another from among hydroxy, cyano, halogen, trifluoromethyl, trifluoromethoxy, hydroxycarbonyl and a group of the formula -NR³R⁴,
wherein
R³ and R⁴ have the meanings given above,
and the salts, solvates and/or solvates of the salts thereof,
with the exception of the following compounds:

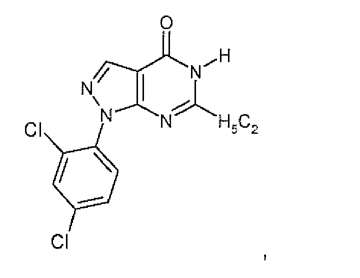

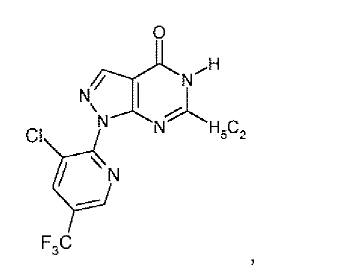

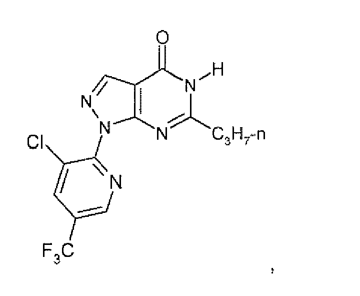

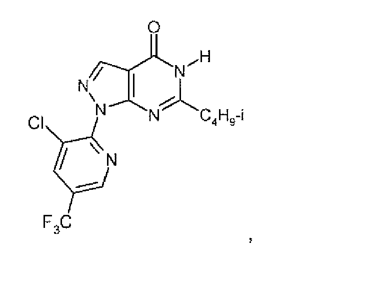

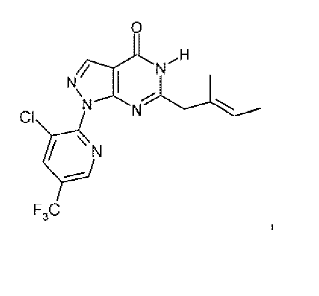

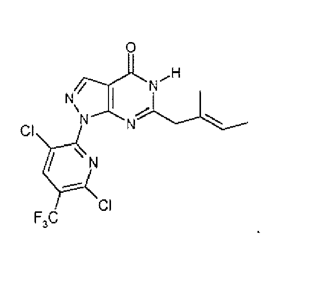

2. Compounds according to claim 1, wherein
R¹ denotes C₁-C₈-alkyl, C₂-C₆-alkenyl, C₂-C₆-alkynyl or C₃-C₈-cycloalkyl, which are optionally substituted by up to 3 groups selected independently of one another from C₁-C₆-alkyl, C₁-C₆-alkoxy, hydroxycarbonyl, cyano, amino, nitro, hydroxy, C₁-C₆-alkylamino, halogen, C₆-C₁₀-arylcarbonylamino, C₁-C₆-alkyl- carbonylamino, C₁-C₆-alkylaminocarbonyl, C₁-C₆-alkoxycarbonyl, C₆-C₁₀-aryl- aminocarbonyl, heteroarylaminocarbonyl, heteroarylcarbonylamino, C₁-C₆- alkyl-sulphonylamino, C₁-C₆-alkylsulphonyl, C₁-C₆-alkylthio,
wherein C₁-C₆-alkyl, C₁-C₆-alkoxy, C₁-C₆-alkylamino, C₆-C₁₀- arylcarbonylamino, C₁-C₆-alkylcarbonylamino, C₁-C₆-alkylaminocarbonyl, C₁-C₆-alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio are optionally substituted by a group selected from among hydroxy, cyano, halogen, hydroxycarbonyl and a group of the formula -NR³R⁴,
wherein R³ and R⁴ independently of one another denote hydrogen or C₁-C₆- alkyl,
or
R³ and R⁴ together with the nitrogen atom to which they are bound denote 5- to 8-membered heterocyclyl,
R² denotes phenyl or heteroaryl, wherein phenyl is substituted by 1 to 3 groups and heteroaryl is optionally substituted by 1 to 3 groups each selected independently of one another from among C₁-C₆-alkyl, C₁-C₆-alkoxy, hydroxycarbonyl, cyano, trifluoromethyl, amino, nitro, hydroxy, C₁-C₆- alkylamino, halogen, C₆-C₁₀-arylcarbonylamino, C₁-C₆-alkylcarbonylamino, C₁-C₆-alkylaminocarbonyl, C₁-C₆-alkoxycarbonyl, C₆-C₁₀-arylaminocarbonyl, heteroarylaminocarbonyl, heteroarylcarbonylamino, C₁-C₆- alkylsulphonylamino, C₁-C₆-alkylsulphonyl, C₁-C₆-alkylthio,
wherein C₁-C₆-alkyl, C₁-C₆-alkoxy, C₁-C₆-alkylamino, C₆-C₁₀-arylcarbonyl- amino, C₁-C₆-alkylcarbonylamino, C₁-C₆-alkylaminocarbonyl, C₁-C₆- alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio are optionally substituted by a group selected from among hydroxy, cyano, halogen, hydroxycarbonyl and a group of the formula -NR³R⁴,
wherein
R³ and R⁴ have the meanings given above,
and the salts, solvates and/or solvates of the salts thereof.

3. Compounds according to claims 1 and 2, wherein
R¹ denotes C₁-C₅-alkyl or C₃-C₆-cycloalkyl, which are optionally substituted by up to 3 groups selected independently of one another from among C₁-C₄- alkyl, C₁-C₄-alkoxy, hydroxycarbonyl, cyano, amino, hydroxy, C₁-C₄- alkylamino, trifluoromethyl, fluorine, chlorine, bromine, C₆-C₁₀- arylcarbonylamino, C₁-C₄-alkylcarbonylamino, C₁-C₄-alkylaminocarbonyl, C₁-C₄-alkoxycarbonyl, C₆-C₁₀-arylaminocarbonyl, heteroarylaminocarbonyl, heteroarylcarbonylamino, C₁-C₄-alkylsulphonylamino, C₁-C₄-alkylsulphonyl, C₁-C₄-alkylthio,
wherein C₁-C₄-alkyl and C₁-C₄-alkoxy are optionally substituted by a group selected from among hydroxy, cyano, fluorine, chlorine, bromine, hydroxycarbonyl and a group of formula -NR³R⁴,
wherein
R³ and R⁴ independently of one another denote hydrogen or C₁-C₄-alkyl,
or
R³ and R⁴ together with the nitrogen atom to which they are bound denote 5- to 6-membered heterocyclyl,
R² denotes phenyl, pyrimidyl, N-oxidopyridyl or pyridyl, wherein phenyl is substituted by 1 to 3 groups and pyrimidyl, N-oxidopyridyl and pyridyl are optionally substituted by 1 to 3 groups each selected independently of one another from among C₁-C₄-alkyl, C₁-C₄-alkoxy, hydroxycarbonyl, cyano, trifluoromethyl, amino, hydroxy, C₁-C₄-alkylamino, fluorine, chlorine, bromine, C₆-C₁₀-arylcarbonylamino, C₁-C₄-alkylcarbonylamino, C₁-C₄- alkylaminocarbonyl, C₁-C₄-alkoxycarbonyl, C₆-C₁₀-arylaminocarbonyl, heteroarylaminocarbonyl, heteroarylcarbonylamino, C₁-C₄- alkylsulphonylamino, C₁-C₄-alkylsulphonyl, C₁-C₄-alkylthio,
wherein C₁-C₄-alkyl and C₁-C₄-alkoxy are optionally substituted by a group selected from among hydroxy, cyano, fluorine, chlorine, bromine, hydroxycarbonyl and a group of the formula -NR³R⁴,
wherein
R³ and R⁴ have the meanings given in claim 1,
and the salts, solvates, and/or solvates of the salts thereof.

4. Compounds according to claims 1 to 3, wherein R¹ has the meanings given in claims 1 to 3 and
R² denotes phenyl, N-oxidopyridyl or pyridyl, wherein phenyl is substituted by 1 to 3 groups and pyridyl and N-oxidopyridyl are optionally substituted by 1 to 3 groups each selected independently of one another from among methyl, ethyl, 2-propyl, trifluoromethyl, methoxy, ethoxy, fluorine and chlorine,
and the salts, solvates and/or solvates of the salts thereof.

5. Compounds according to claims 1 to 4, wherein
R¹ denotes C₁-C₅-alkyl or C₅-C₆-cycloalkyl, which are optionally substituted by up to 3 groups selected independently of one another from among C₁-C₄- alkyl, trifluoromethyl, fluorine, hydroxy, phenylcarbonylamino, C₁-C₄- alkylcarbonylamino, C₁-C₄-alkylaminocarbonyl or phenylaminocarbonyl,
R² denotes phenyl, N-oxidopyridyl or pyridyl, wherein phenyl is substituted by 1 to 3 groups and pyridyl and N-oxidopyridyl are optionally substituted by 1 to 3 groups each selected independently of one another from among methyl, ethyl, 2-propyl, trifluoromethyl, methoxy, ethoxy, fluorine and chlorine,
and the salts, solvates and/or solvates of the salts thereof.

6. Compounds according to claims 1 to 5, wherein
R¹ denotes C₁-C₅-alkyl or C₅-C₆-cycloalkyl, which are optionally substituted by up to 3 groups selected independently of one another from among C₁-C₄- alkyl, fluorine, trifluoromethyl, hydroxy, phenylcarbonylamino, C₁-C₄- alkylcarbonylamino, C₁-C₄-alkylaminocarbonyl or phenylaminocarbonyl, and
R² denotes phenyl, N-oxidopyridyl or pyridyl, wherein phenyl is substituted by a group and pyridyl and N-oxidopyridyl are optionally each substituted by a group selected independently of one another from among methyl, ethyl, 2-propyl, trifluoromethyl, methoxy, ethoxy, fluorine and chlorine,
and the salts, solvates and/or solvates of the salts thereof.

7. Compounds of formula 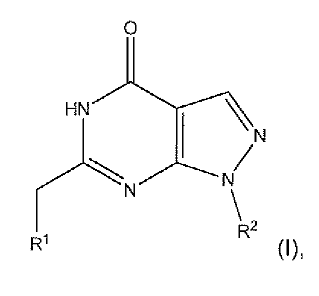 wherein
R¹ denotes C₁-C₈-alkyl, C₂-C₆-alkenyl, C₂-C₆-alkynyl or C₃-C₈-cycloalkyl, wherein C₁-C₈-alkyl is optionally substituted by oxo, and wherein C₁-C₈-alkyl, C₂-C₆-alkenyl, C₂-C₆-alkynyl and C₃-C₈-cycloalkyl are optionally substituted by up to 3 groups selected independently of R³ one another from among C₁-C₆-alkyl, C₁-C₆-alkoxy, hydroxycarbonyl, cyano, amino, nitro, hydroxy, C₁-C₆-alkylamino, halogen, trifluoromethyl, trifluoromethoxy, C₆-C₁₀-arylcarbonylamino, C₁-C₆-alkylcarbonylamino, C₁-C₆-alkylaminocarbonyl, C₁-C₆-alkoxycarbonyl, C₆-C₁₀- arylaminocarbonyl, heteroarylaminocarbonyl, heteroarylcarbonylamino, C₁-C₆-alkylsulphonylamino, C₁-C₆-alkylsulphonyl, C₁-C₆-alkylthio,
wherein
C₁-C₆-alkyl, C₁-C₆-alkoxy, C₁-C₆-alkylamino, C₆-C₁₀-arylcarbonylamino, C₁-C₆- alkylcarbonylamino, C₁-C₆-alkylaminocarbonyl, C₁-C₆- alkoxycarbonyl, C₆-C₁₀-arylaminocarbonyl, heteroarylaminocarbonyl, heteroarylcarbonylamino, C₁₋₆-alkylsulphonylamino, C₁-C₆-alkylsulphonyl and C₁-C₆-alkylthio are optionally substituted by one to three groups selected independently of one another from among hydroxy, cyano, halogen, trifluoromethyl, trifluoromethoxy, hydroxycarbonyl and a group of formula -NR³R⁴,
wherein
R³ and R⁴ independently of one another denote hydrogen or C₁-C₆-alkyl,
or
R³ and R⁴, together with the nitrogen atom to which they are bound, represent 5- to 8-membered heterocyclyl,
R² denotes phenyl or heteroaryl, wherein phenyl is substituted by 1 to 3 groups and heteroaryl is optionally substituted by 1 to 3 groups, each independently selected from among C₁-C₆-alkyl, C₁-C₆-alkoxy, hydroxycarbonyl, cyano, trifluoromethyl, trifluoromethoxy, amino, nitro, hydroxy, C₁-C₆-alkylamino, halogen, C₆-C₁₀-arylcarbonylamino, C₁-C₆-alkylcarbonylamino, C₁-C₆- alkylaminocarbonyl, C₁-C₆-alkoxycarbonyl, C₆-C₁₀-arylaminocarbonyl, heteroarylaminocarbonyl, heteroarylcarbonylamino, C₁-C₆- alkylsulphonylamino, C₁-C₆-alkylsulphonyl, C₁-C₆-alkylthio, wherein C₁-C₆-alkyl, C₁-C₆-alkoxy, C₁-C₆-alkylamino, C₆-C₁₀-arylcarbonyl- amino, C₁-C₆-alkylcarbonylamino, C₁-C₆-alkylaminocarbonyl, C₁-C₆- alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio are optionally substituted by one to three groups selected independently of one another from among hydroxy, cyano, halogen, trifluoromethyl, trifluoromethoxy, hydroxycarbonyl and a group of the formula -NR³R⁴,
wherein
R³ and R⁴ have the meanings given above,
and the salts, solvates and/or solvates of the salts thereof,
for the treatment and/or prophylaxis of diseases in humans.

8. Compounds according to claim 7, wherein
R¹ denotes C₁-C₈-alkyl, C₂-C₅-alkenyl, C₂-C₆-alkynyl or C₃-C₈-cycloalkyl, which are optionally substituted by up to 3 groups selected independently of one another from C₁-C₆-alkyl, C₁-C₆-alkoxy, hydroxycarbonyl, cyano, amino, nitro, hydroxy, C₁-C₆-alkylamino, halogen, C₆-C₁₀-arylcarbonylamino, C₁-C₆-alkyl- carbonylamino, C₁-C₆-alkylaminocarbonyl, C₁-C₆-alkoxycarbonyl, C₆-C₁₀-aryl- aminocarbonyl, heteroarylaminocarbonyl, heteroarylcarbonylamino, C₁-C₆- alkyl-sulphonylamino, C₁-C₆-alkylsulphonyl, C₁-C₆-alkylthio,
wherein C₁-C₆-alkyl, C₁-C₆-alkoxy, C₁-C₆-alkylamino, C₆-C₁₀- arylcarbonylamino, C₁-C₆-alkylcarbonylamino, C₁-C₆-alkylaminocarbonyl, C₁-C₆-alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio are optionally substituted by a group selected from among hydroxy, cyano, halogen, hydroxycarbonyl and a group of the formula -NR³R⁴,
wherein R³ and R⁴ independently of one another denote hydrogen or C₁-C₆- alkyl,
or
R³ and R⁴ together with the nitrogen atom to which they are bound denote 5- to 8-membered heterocyclyl,
R² denotes phenyl or heteroaryl, wherein phenyl is substituted by 1 to 3 groups and heteroaryl is optionally substituted by 1 to 3 groups each selected independently of one another from among C₁-C₆-alkyl , C₁-C₆-alkoxy, hydroxycarbonyl, cyano, trifluoromethyl, amino, nitro, hydroxy, C₁-C₆- alkylamino, halogen, C₆-C₁₀-arylcarbonylamino, C₁-C₆-alkylcarbonylamino, C₁-C₆-alkylaminocarbonyl, C₁-C₆-alkoxycarbonyl, C₆-C₁₀-arylaminocarbonyl, heteroarylaminocarbonyl, heteroarylcarbonylamino, C₁-C₆- alkylsulphonylamino, C₁-C₆-alkylsulphonyl, C₁-C₆-alkylthio,
wherein C₁-C₆-alkyl, C₁-C₆-alkoxy, C₁-C₆-alkylamino, C₆-C₁₀-arylcarbonyl- amino, C₁-C₆-alkylcarbonylamino, C₁-C₆-alkylaminocarbonyl, C₁-C₆- alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio are optionally substituted by a group selected from among hydroxy, cyano, halogen, hydroxycarbonyl and a group of the formula -NR³R⁴,
wherein
R³ and R⁴ have the meanings give above,
and the salts, solvates and/or solvates of the salts thereof,
for the treatment and/or prophylaxis of diseases in humans.

9. Compounds according to one of claims 7 or 8, wherein
R¹ denotes C₁-C₅-alkyl or C₃-C₆-cycloalkyl, which are optionally substituted by up to 3 groups selected independently of one another from among C₁-C₄- alkyl, C₁-C₄-alkoxy, hydroxycarbonyl, cyano, amino, hydroxy, C₁-C₄- alkylamino, trifluoromethyl, fluorine, chlorine, bromine, C₆-C₁₀- arylcarbonylamino, C₁-C₄-alkylcarbonylamino, C₁-C₄-alkylaminocarbonyl, C₁-C₄-alkoxycarbonyl, C₆-C₁₀-arylaminocarbonyl, heteroarylaminocarbonyl, heteroarylcarbonylamino, C₁-C₄-alkylsulphonylamino, C₁-C₄-alkylsulphonyl, C₁-C₄-alkylthio,
wherein C₁-C₄-alkyl and C₁-C₄-alkoxy are optionally substituted by a group selected from among hydroxy, cyano, fluorine, chlorine, bromine, hydroxycarbonyl and a group of formula -NR³R⁴,
wherein
R³ and R⁴ independently of one another denote hydrogen or C₁-C₄-alkyl,
or
R³ and R⁴ together with the nitrogen atom to which they are bound denote 5- to 6-membered heterocyclyl,
R² denotes phenyl, pyrimidyl, N-oxidopyridyl or pyridyl, wherein phenyl is substituted by 1 to 3 groups and pyrimidyl, N-oxidopyridyl and pyridyl are optionally substituted by 1 to 3 groups each selected independently of one another from among C₁-C₄-alkyl, C₁-C₄-alkoxy, hydroxycarbonyl, cyano, trifluoromethyl, amino, hydroxy, C₁-C₄-alkylamino, fluorine, chlorine, bromine, C₆-C₁₀-arylcarbonylamino, C₁-C₄-alkylcarbonylamino, C₁-C₄- alkylaminocarbonyl, C₁-C₄-alkoxycarbonyl, C₆-C₁₀-arylaminocarbonyl, heteroarylaminocarbonyl, heteroarylcarbonylamino, C₁-C₄- alkylsulphonylamino, C₁-C₄-alkylsulphonyl, C₁-C₄-alkylthio,
wherein C₁-C₄-alkyl and C₁-C₄-alkoxy are optionally substituted by a group selected from among hydroxy, cyano, fluorine, chlorine, bromine, hydroxycarbonyl and a group of the formula -NR³R⁴,
wherein
R³ and R⁴ have the meanings given in claim 7,
and the salts, solvates and/or solvates of the salts thereof,
for the treatment and/or prophylaxis of diseases in humans.

10. Compounds according to one of claims 7 to 9, wherein R¹ has the meanings given in claims 7 to 9 and
R² denotes phenyl, N-oxidopyridyl or pyridyl, wherein phenyl is substituted by 1 to 3 groups and pyridyl and N-oxidopyridyl are optionally substituted by 1 to 3 groups each selected independently of one another from among methyl, ethyl, 2-propyl, trifluoromethyl, methoxy, ethoxy, fluorine and chlorine,
and the salts, solvates and/or solvates of the salts thereof,
for the treatment and/or prophylaxis of diseases in humans.

11. Compounds according to one of claims 7 to 10, wherein
R¹ denotes C₁-C₅-alkyl or C₅-C₆-cycloalkyl, which are optionally substituted by up to 3 groups selected independently of one another from among C₁-C₄- alkyl, trifluoromethyl, fluorine, hydroxy, phenylcarbonylamino, C₁-C₄- alkylcarbonylamino, C₁-C₄-alkylaminocarbonyl or phenylaminocarbonyl,
R² denotes phenyl, N-oxidopyridyl or pyridyl, wherein phenyl, is substituted by 1 to 3 groups and pyridyl and N-oxidopyridyl are optionally substituted by 1 to 3 groups each selected independently of one another from among methyl, ethyl, 2-propyl, trifluoromethyl, methoxy, ethoxy, fluorine and chlorine,
and the salts, solvates and/or solvates of the salts thereof, for the treatment and/or prophylaxis of diseases in humans.

12. Compounds according to one of claims 7 to 11, wherein
R¹ denotes C₁-C₅-alkyl or C₅-C₆-cycloalkyl, which are optionally substituted by up to 3 groups selected independently of one another from among C₁-C₄- alkyl, fluorine, trifluoromethyl, hydroxy, phenylcarbonylamino, C₁-C₄- alkylcarbonylamino, C₁-C₄-alkylaminocarbonyl or phenylaminocarbonyl, and
R² denotes phenyl, N-oxidopyridyl or pyridyl, wherein phenyl is substituted by a group and pyridyl and N-oxidopyridyl are optionally each substituted by a group selected independently of one another from among methyl, ethyl, 2- propyl, trifluoromethyl, methoxy, ethoxy, fluorine and chlorine,
and the salts, solvates and/or solvates of the salts thereof,
for the treatment and/or prophylaxis of diseases in humans.

13. Process for preparing compounds according to claim 7, **characterised in that**
[A] compounds of formula 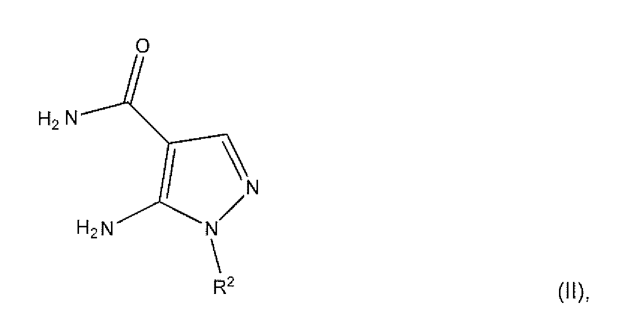 wherein R² has the meanings given in claim 7, are first converted, by reaction with a compound of formula 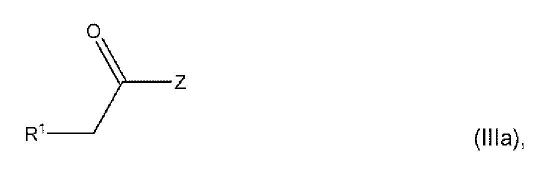 wherein R¹ has the meanings given in claim 7,
and
Z denotes chlorine or bromine,
in an inert solvent and in the presence of a base, into compounds of the formula 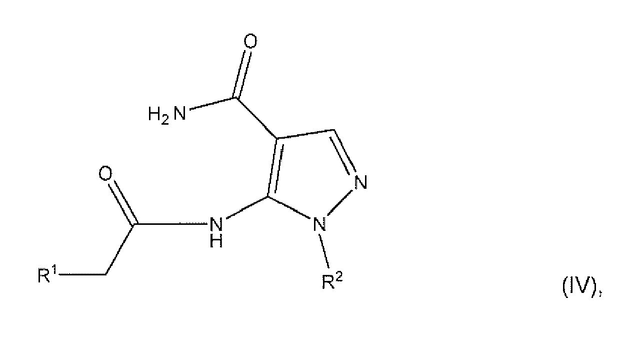 wherein
R¹ and R² have the meanings given in claim 7,
then cyclised in an inert solvent in the presence of a base to obtain compounds of formula (I),
or
[B] compounds of formula (II) are reacted, with direct cyclisation to form (I), with a compound of the formula 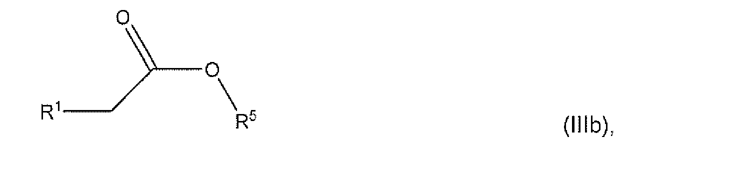 wherein
R¹ has the meanings given in claim 7,
and
R⁵ denotes methyl or ethyl,
in an inert solvent and in the presence of a base, or
[C] compounds of the formula 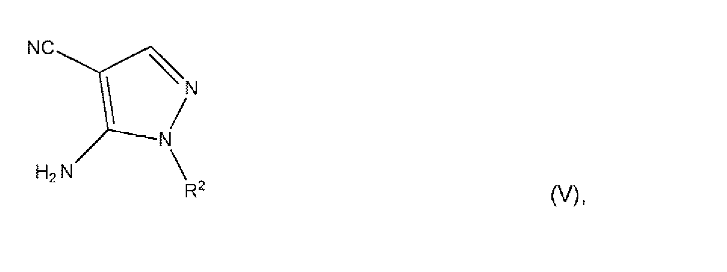 wherein
R² has the meanings given in claim 7,
are first converted by reacting with a compound of formula (IIIa) in an inert solvent and in the presence of a base, into compounds of formula 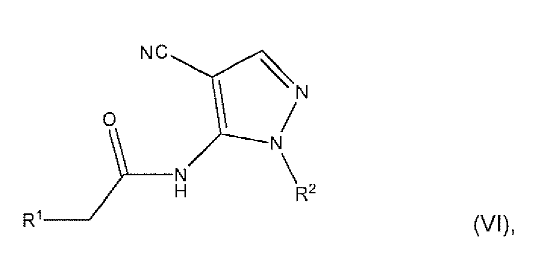 wherein
R¹ and R² have the meanings given in claim 7,
and in a second step these compounds are cyclised in an inert solvent and in the presence of a base and an oxidising agent to form (I),
and the resulting compounds of formula (I) are optionally reacted with the corresponding (i) solvents and/or (ii) bases or acids to form the solvates, salts and/or solvates of the salts thereof.

14. Medicaments containing at least one of the compounds according to one of claims 7 to 12 and at least one pharmaceutically acceptable, essentially nontoxic carrier or excipient for the treatment and/or prophylaxis of diseases in humans.

15. Use of compounds according to one of claims 7 to 12 for the preparation of medicaments for the treatment of diseases in humans.

16. Use of the compounds according to one of claims 7 to 12 for preparing a medicament for the prevention and/or treatment of disorders of cognition, concentration, learning and/or memory capacity.

17. Use according to claim 16, wherein the disorder is a consequence of Alzheimer's disease.

18. Use of the compounds according to one of claims 7 to 12 for preparing a medicament for improving cognition, concentration, learning and/or memory capacity.

19. Use of the compounds according to one of claims 7 to 12 for preparing a medicament for inhibiting PDE9A.

## Revendications

1. Composés de formule : 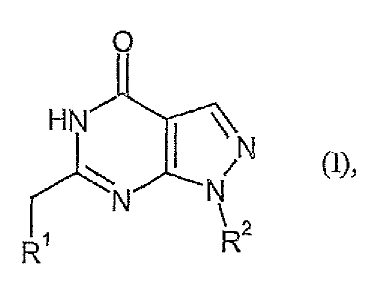 dans laquelle
R¹ représente alkyle en C₁-C₈, alcényle en C₂-C₆, alcynyle en C₂-C₆ ou cycloalkyle en C₃-C₈, où alkyle en C₁-C₈ est le cas échéant substitué par oxo,
où alkyle en C₁-C₈, alcényle en C₂-C₆, alcynyle en C₂-C₆ ou cycloalkyle en C₃-C₈ sont le cas échéant substitués par jusqu'à 3 restes, indépendamment choisis parmi le groupe consistant en alkyle en C₁-C₆, alcoxy en C₁-C₆, hydroxycarbonyle, cyano, amino, nitro, hydroxy, alkylamino en C₁-C₆, halogène, trifluorométhyle, trifluorométhoxy, (aryl en C₆-C₁₀) carbonylamino, (alkyl en C₁-C₆)carbonylamino, (alkyl en C₁-C₆) aminocarbonyle, (alcoxy en C₁-C₆) carbonyle, (aryl en C₆-C₁₀) aminocarbonyle, hétéroarylaminocarbonyle, hétéroarylcarbonylamino, (alkyl en C₁-C₆) sulfonylamino, alkylsulfonyle en C₁-C₆, alkylthio en C₁-C₆,
où
alkyle en C₁-C₆, alcoxy en C₁-C₆, alkylamino en C₁-C₆, (aryl en C₆-C₁₀) carbonylamino, (alkyl en C₁-C₆)carbonylamino, (alkyl en C₁-C₆) aminocarbonyle, (alcoxy en C₁-C₆) carbonyle, (aryl en C₆-C₁₀) - aminocarbonyle, hétéroarylaminocarbonyle, hétéroarylcarbonylamino, (alkyl en C₁-C₅)sulfonylamino, alkylsulfonyle en C₁-C₆, alkylthio en C₁-C₆ sont le cas échéant substitué par un à trois restes indépendamment choisis parmi le groupe consistant en hydroxy, cyano, halogène, trifluorométhyle, trifluorométhoxy, hydroxycarbonyle et un groupe de formule -NR³R⁴,
où
R³ et R⁴ sont indépendamment, hydrogène ou alkyle en C₁-C₆, ou
R³ et R⁴, avec l'atome d'azote auquel ils sont liés, représentent un hétérocyclyle de 5 à 8 membres,
R² représente phényle ou hétéroaryle, où phényle est substitué par 1 à 3 restes et hétéroaryle est le cas échéant substitué par 1 à 3 restes, chaque fois indépendamment choisis parmi le groupe consistant en alkyle en C₁-C₆, alcoxy en C₁-C₆, hydroxycarbonyle, cyano, trifluorométhyle, trifluorométhoxy, amino, nitro, hydroxy, alkylamino en C₁-C₆, halogène, (aryl en C₆-C₁₀)carbonylamino, (alkyl en C₁-C₆)carbonylamino, (alkyl en C₁-C₆)aminocarbonyle, (alcoxy en C₁-C₆) carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où
alkyle en C₁-C₆, alcoxy en C₁-C₆, alkylamino en C₁-C₆, (aryl en C₆-C₁₀)carbonylamino, (alkyl en C₁-C₆)carbonylamino, (alkyl en C₁-C₆)aminocarbonyle, (alcoxy en C₁-C₆)carbonyle, (aryl en C₆-C₁₀) - aminocarbonyle, hétéroarylaminocarbonyle, hétéroarylcarbonylamino, (alkyl en C₁-C₆)sulfonylamino, alkylsulfonyle en C₁-C₆ et alkylthio en C₁-C₆ sont le cas échéant substitué par un à trois restes indépendamment choisis parmi le groupe consistant en hydroxy, cyano, halogène, trifluorométhyle, trifluorométhoxy, hydroxycarbonyle et un groupe de formule -NR³R⁴,
où
R³ et R⁴ présentent les significations données ci-dessus, ainsi que leurs sels, solvates et/ou les solvates des sels, où les composés suivants sont exclus:

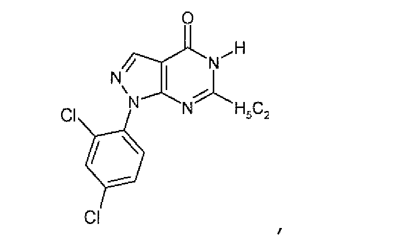

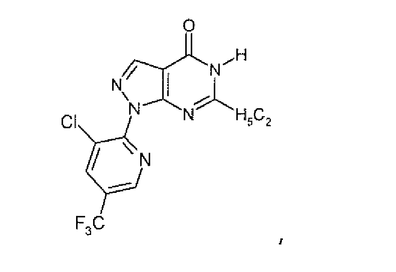

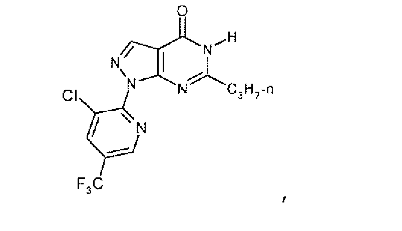

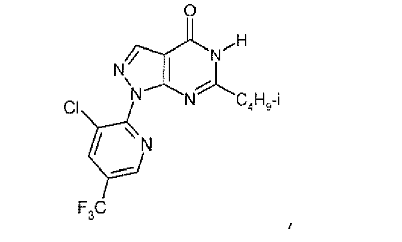

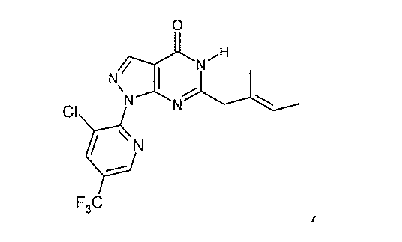

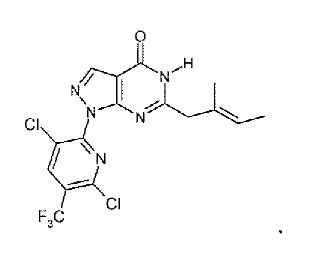

2. Composés selon la revendication 1, où
R¹ représente alkyle en C₁-C₈, alcényle en C₂-C₆, alcynyle en C₂-C₆ ou cycloalkyle en C₃-C₈, qui sont le cas échéant substitués par jusqu'à 3 restes, indépendamment choisis parmi le groupe consistant en alkyle en C₁-C₆, alcoxy en C₁-C₆, hydroxycarbonyle, cyano, amino, nitro, hydroxy, alkylamino en C₁-C₆, halogène, (aryl en C₆-C₁₀)carbonylamino, (alkyl en C₁-C₆) carbonylamino, (alkyl en C₁-C₆)aminocarbonyle, (alcoxy en C₁-C₆)carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où alkyle en C₁-C₆, alcoxy en C₁-C₆, alkylamino en C₁-C₆, (aryl en C₆-C₁₀)carbonylamino, (alkyl en C₁-C₆)carbonylamino, (alkyl en C₁-C₆)aminocarbonyle, (alcoxy en C₁-C₆)carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆ sont le cas échéant substitué par un reste indépendamment choisi parmi le groupe consistant en hydroxy, cyano, halogène, hydroxycarbonyle et un groupe de formule -NR³R⁴,
où
R³ et R⁴ sont indépendamment, hydrogène ou alkyle en C₁-C₆, ou
R³ et R⁴, avec l'atome d'azote auquel ils sont liés, représentent un hétérocyclyle de 5 à 8 membres,
R² représente phényle ou hétéroaryle, où phényle est substitué par 1 à 3 restes et hétéroaryle est le cas échéant substitué par 1 à 3 restes, chaque fois indépendamment choisis parmi le groupe consistant en alkyle en C₁-C₆, alcoxy en C₁-C₆, hydroxycarbonyle, cyano, trifluorométhyle, amino, nitro, hydroxy, alkylamino en C₁-C₆, halogène, (aryl en C₆-C₁₀)carbonylamino, (alkyl en C₁-C₆)carbonylamino, (alkyl en C₁-C₆)aminocarbonyle, (alcoxy en C₁-C₆)carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où alkyle en C₁-C₆, alcoxy en C₁-C₆, alkylamino en C₁-C₆, (aryl en C₆-C₁₀) carbonylamino, (alkyl en C₁-C₆)carbonylamino, (alkyl en C₁-C₆)aminocarbonyle, (alcoxy en C₁-C₆)carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆ et alkylthio en C₁-C₆ sont le cas échéant substitué par un à trois restes indépendamment choisis parmi le groupe consistant en hydroxy, cyano, halogène, hydroxycarbonyle et un groupe de formule -NR³R⁴,
où
R³ et R⁴ présentent les significations données ci-dessus, ainsi que leurs sels, solvates et/ou les solvates des sels.

3. Composés selon les revendications 1 et 2, où
R¹ représente alkyle en C₁-C₅ ou cycloalkyle en C₃-C₆, qui sont le cas échéant substitués par jusqu'à 3 restes, indépendamment choisis parmi le groupe consistant en alkyle en C₁-C₄, alcoxy en C₁-C₄, hydroxycarbonyle, cyano, amino, hydroxy, alkylamino en C₁-C₄, trifluorométhyle, fluor, chlore, brome, (aryl en C₆-C₁₀)carbonylamino, (alkyl en C₁-C₄)carbonylamino, (alkyl en C₁-C₄)aminocarbonyle, (alcoxy en C₁-C₄)carbonyle, (aryl en C₆-C₁₀)aminocarbonyle, hétéroarylaminocarbonyle, hétéroarylcarbonylamino, (alkyl en C₁-C₄)sulfonylamino, alkylsulfonyle en C₁-C₄, alkylthio en C₁-C₄,
où alkyle en C₁-C₄ et alcoxy en C₁-C₄ sont le cas échéant substitués par un reste choisi parmi le groupe consistant en hydroxy, cyano, fluor, chlore, brome, hydroxycarbonyle et un groupe de formule -NR³R⁴,
où
R³ et R⁴ sont indépendamment, hydrogène ou alkyle en C₁-C₄, ou
R³ et R⁴, avec l'atome d'azote auquel ils sont liés, représentent un hétérocyclyle de 5 à 6 membres,
R² représente phényle, pyrimidyle, N-oxydopyridyle ou pyridyle, où phényle est substitué par 1 à 3 restes et pyrimidyle, N-oxydopyridyle et pyridyle sont le cas échéant substitués par 1 à 3 restes, chaque fois indépendamment choisis parmi le groupe consistant en alkyle en C₁-C₄, alcoxy en C₁-C₄, hydroxycarbonyle, cyano, trifluorométhyle, amino, hydroxy, alkylamino en C₁-C₄, fluor, chlore, brome, (aryl en C₆-C₁₀)-carbonylamino, (alkyl en C₁-C₄)carbonylamino, (alkyl en C₁-C₄)aminocarbonyle, (alcoxy en C₁-C₄) carbonyle, (aryl en C₆-C₁₀)aminocarbonyle, hétéroarylaminocarbonyle, hétéroarylcarbonylamino, (alkyl en C₁-C₄)sulfonylamino, alkylsulfonyle en C₁-C₄, alkylthio en C₁-C₄,
où alkyle en C₁-C₄ et alcoxy en C₁-C₄ sont le cas échéant substitué par un reste choisi parmi le groupe consistant en hydroxy, cyano, fluor, chlore, brome, hydroxycarbonyle et un groupe de formule -NR³R⁴,
où
R³ et R⁴ présentent les significations données à la revendication 1,
ainsi que leurs sels, solvates et/ou les solvates des sels.

4. Composés selon les revendications 1 à 3, où R¹ présente les significations données aux revendications 1 à 3, et
R² représente phényle, N-oxydopyridyle ou pyridyle, où phényle est substitué par 1 à 3 restes et pyridyle et N-oxydopyridyle sont le cas échéant substitués par 1 à 3 restes, chaque fois indépendamment choisis parmi le groupe consistant en méthyle, éthyle, 2-propyle, trifluorométhyle, méthoxy, éthoxy, fluor et chlore,
ainsi que leurs sels, solvates et/ou les solvates des sels.

5. Composés selon les revendications 1 à 4, où
R¹ représente alkyle en C₁-C₅ ou cycloalkyle en C₅-C₆, qui sont le cas échéant substitués par jusqu'à 3 restes indépendamment choisis parmi le groupe consistant en alkyle en C₁-C₄, trifluorométhyle, fluor, hydroxy, phénylcarbonylamino, (alkyl en C₁-C₄)carbonylamino, (alkyl en C₁-C₄)aminocarbonyle ou phénylaminocarbonyle,
R² représente phényle, N-oxydopyridyle ou pyridyle, où phényle est substitué par 1 à 3 restes et pyridyle et N-oxydopyridyle sont le cas échéant substitués par 1 à 3 restes, chaque fois indépendamment choisis parmi le groupe consistant en méthyle, éthyle, 2-propyle, trifluorométhyle, méthoxy, éthoxy, fluor et chlore,
ainsi que leurs sels, solvates et/ou les solvates des sels.

6. Composés selon les revendications 1 à 5, où
R¹ représente alkyle en C₁-C₅ ou cycloalkyle en C₅-C₆, qui sont le cas échéant substitués par jusqu'à 3 restes indépendamment choisis parmi le groupe consistant en alkyle en C₁-C₄, fluor, trifluorométhyle, hydroxy, phénylcarbonylamino, (alkyl en C₂-C₄)carbonylamino, (alkyl en C₁-C₄)aminocarbonyle ou phénylaminocarbonyle,
R² représente phényle, N-oxydopyridyle ou pyridyle, où phényle est substitué par 1 reste et pyridyle et N-oxydopyridyle sont le cas échéant substitués par 1 reste, chaque fois indépendamment choisi parmi le groupe consistant en méthyle, éthyle, 2-propyle, trifluorométhyle, méthoxy, éthoxy, fluor et chlore,
ainsi que leurs sels, solvates et/ou les solvates des sels.

7. Composés de formule : 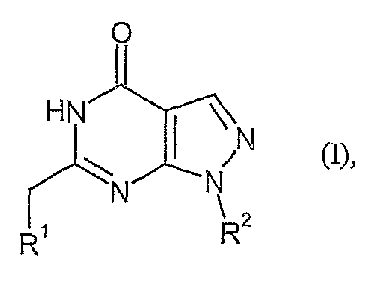 dans laquelle
R¹ représente alkyle en C₁-C₈, alcényle en C₂-C₆, alcynyle en C₂-C₆ ou cycloalkyle en C₃-C₈, où alkyle en C₁-C₈ est le cas échéant substitué par oxo, et
où alkyle en C₁-C₈, alcényle en C₂-C₆, alcynyle en C₂-C₆ et cycloalkyle en C₃-C₈ sont le cas échéant substitués par jusqu'à 3 restes, indépendamment choisis parmi le groupe consistant en alkyle en C₁-C₆, alcoxy en C₁-C₆, hydroxycarbonyle, cyano, amino, nitro, hydroxy, alkylamino en C₁-C₆, halogène, trifluorométhyle, trifluorométhoxy, (aryl en C₆-C₁₀)carbonylamino, (alkyl en C₁-C₆)carbonylamino, (alkyl en C₁-C₆)aminocarbonyle, (alcoxy en C₁-C₆) carbonyle, (aryl en C₆-C₁₀) aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où
alkyle en C₁-C₆, alcoxy en C₁-C₆, alkylamino en C₁-C₆, (aryl en C₆-C₁₀)carbonylamino, (alkyl en C₁-C₆)carbonylamino, (alkyl en C₁-C₆)aminocarbonyle, (alcoxy en C₁-C₆)carbonyle, (aryl en C₆-C₁₀)-aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆ sont le cas échéant substitué par un à trois restes indépendamment choisis parmi le groupe consistant en hydroxy, cyano, halogène, trifluorométhyle, trifluorométhoxy, hydroxycarbonyle et un groupe de formule -NR³R⁴,
où
R³ et R⁴ sont indépendamment, hydrogène ou alkyle en C₁-C₆, ou
R³ et R⁴, avec l'atome d'azote auquel ils sont liés, représentent un hétérocyclyle de 5 à 8 membres,
R² représente phényle ou hétéroaryle, où phényle est substitué par 1 à 3 restes et hétéroaryle est le cas échéant substitué par 1 à 3 restes, chaque fois indépendamment choisis parmi le groupe consistant en alkyle en C₁-C₆, alcoxy en C₁-C₆, hydroxycarbonyle, cyano, trifluorométhyle, trifluorométhoxy, amino, nitro, hydroxy, alkylamino en C₁-C₆, halogène, (aryl en C₆-C₁₀)carbonylamino, (alkyl en C₁-C₆)carbonylamino, (alkyl en C₁-C₆) aminocarbonyle, (alcoxy en C₁-C₆) carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où
alkyle en C₁-C₆, alcoxy en C₁-C₆, alkylamino en C₁-C₆, (aryl en C₆-C₁₀)carbonylamino, (alkyl en C₁-C₆)carbonylamino, (alkyl en C₁-C₆)aminocarbonyle, (alcoxy en C₁-C₆)carbonyle, (aryl en C₆-C₁₀)-aminocarbonyle, hétéroarylaminocarbonyle, hétéroarylcarbonylamino, (alkyl en C₁-C₆)sulfonylamino, alkylsulfonyle en C₁-C₆ et alkylthio en C₁-C₆ sont le cas échéant substitué par un à trois restes indépendamment choisis parmi le groupe consistant en hydroxy, cyano, halogène, trifluorométhyle, trifluorométhoxy, hydroxycarbonyle et un groupe de formule -NR³R⁴,
où
R³ et R⁴ présentent les significations données ci-dessus, ainsi que leurs sels, solvates et/ou les solvates des sels, pour le traitement et/ou la prophylaxie de maladies chez l'homme.

8. Composés selon la revendication 7, où
R¹ représente alkyle en C₁-C₈, alcényle en C₂-C₆, alcynyle en C₂-C₆ ou cycloalkyle en C₃-C₈, qui sont le cas échéant substitués par jusqu'à 3 restes, indépendamment choisis parmi le groupe consistant en alkyle en C₁-C₆, alcoxy en C₁-C₆, hydroxycarbonyle, cyano, amino, nitro, hydroxy, alkylamino en C₁-C₆, halogène, (aryl en C₆-C₁₀) carbonylamino, (alkyl en C₁-C₆)carbonylamino, (alkyl en C₁-C₆)aminocarbanyle, (alcoxy en C₁-C₆)carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où alkyle en C₁-C₆, (alcoxy en C₁-C₆, alkylamino en C₁-C₆, (aryl en C₆-C₁₀) carbonylamino, (alkyl en C₁-C₆)carbonylamino, (alkyl en C₁-C₆)aminocarbonyle, (alcoxy en C₁-C₆)carbonyle (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆ sont le cas échéant substitué par un reste choisi parmi le groupe consistant en hydroxy, cyano, halogène, hydroxycarbonyle et un groupe de formule -NR³R⁴,
où
R³ et R⁴ sont indépendamment, hydrogène ou alkyle en C₁-C₆, ou
R³ et R⁴, avec l'atome d'azote auquel ils sont liés, représentent un hétérocyclyle de 5 à 8 membres,
R² représente phényle ou hétéroaryle, où phényle est substitué par 1 à 3 restes et hétéroaryle est le cas échéant substitué par 1 à 3 restes, chaque fois indépendamment choisis parmi le groupe consistant en alkyle en C₁-C₆, alcoxy en C₁-C₆, hydroxycarbonyle, cyano, trifluorométhyle, amino, nitro, hydroxy, alkylamino en C₁-C₆, halogène, (aryl en C₆-C₁₀)carbonylamino, (alkyl en C₁-C₆)carbonylamino, (alkyl en C₁-C₆)aminocarbonyle, (alcoxy en C₁-C₆) carbonyle, (aryl en C₆-C₁₀) aminocarbonyle, hétéroarylaminocarbonyle, hétéroarylcarbonylamino, (alkyl en C₁-C₆)sulfonylamino, alkylsulfonyle en C₁-C₆, alkylthio en C₁-C₆,
où alkyle en C₁-C₆, alcoxy en C₁-C₆, alkylamino en C₁-C₆, (aryl en C₆-C₁₀) carbonylamino, (alkyl en C₁-C₆)carbonylamino, (alkyl en C₁-C₆)aminocarbonyle, (alcoxy en C₁-C₆)carbonyle, (aryl en C₆-C₁₀)aminocarbonyle, hétéroarylaminocarbonyle, hétéroarylcarbonylamino, (alkyl en C₁-C₆)sulfonylamino, alkylsulfonyle en C₁-C₆ et alkylthio en C₁-C₆ sont le cas échéant substitué par un à trois restes indépendamment choisis parmi le groupe consistant en hydroxy, cyano, halogène, hydroxycarbonyle et un groupe de formule -NR³R⁴,
où
R³ et R⁴ présentent les significations données ci-dessus,
ainsi que leurs sels, solvates et/ou les solvates des sels, pour le traitement et/ou la prophylaxie de maladies chez l'homme.

9. Composés selon une des revendications 7 ou 8, où
R¹ représente alkyle en C₁-C₅ ou cycloalkyle en C₃-C₆, qui sont le cas échéant substitués par jusqu'à 3 restes, indépendamment choisis parmi le groupe consistant en alkyle en C₁-C₄, alcoxy en C₁-C₄, hydroxycarbonyle, cyano, amino, hydroxy, alkylamino en C₁-C₄, trifluorométhyle, fluor, chlore, brome, (aryl en C₆-C₁₀)carbonylamino, (alkyl en C₁-C₄)carbonylamino, (alkyl en C₁-C₄)aminocarbonyle, (alcoxy en C₁-C₄)carbonyle, (aryl en C₆-C₁₀)aminocarbonyle, hétéroarylaminocarbonyle, hétéroarylcarbonylamino, (alkyl en C₁-C₄)sulfonylamino, alkylsulfonyle en C₁-C₄, alkylthio en C₁-C₄,
où alkyle en C₁-C₄ et alcoxy en C₁-C₄ sont le cas échéant substitués par un reste choisi parmi le groupe consistant en hydroxy, cyano, fluor, chlore, brome, hydroxycarbonyle et un groupe de formule -NR³R⁴,
où
R³ et R⁴ sont indépendamment, hydrogène ou alkyle en C₁-C₄, ou
R³ et R⁴, avec l'atome d'azote auquel ils sont liés, représentent un hétérocyclyle de 5 à 6 membres,
R² représente phényle, pyrimidyle, N-oxydopyridyle ou pyridyle, où phényle est substitué par 1 à 3 restes et pyrimidyle, N-oxydopyridyle et pyridyle sont le cas échéant substitués par 1 à 3 restes, chaque fois indépendamment choisis parmi le groupe consistant en alkyle en C₁-C₄, alcoxy en C₁-C₄, hydroxycarbonyle, cyano, trifluorométhyle, amino, hydroxy, alkylamino en C₁-C₄, fluor, chlore, brome, (aryl en C₆-C₁₀)-carbonylamino, (alkyl en C₁-C₄)carbonylamino, (alkyl en C₁-C₄)aminocarbonyle, (alcoxy en C₁-C₄) carbonyle, (aryl en C₆-C₁₀)aminocarbonyle, hétéroarylaminocarbonyle, hétéroarylcarbonylamino, (alkyl en C₁-C₄)sulfonylamino, alkylsulfonyle en C₁-C₄, alkylthio en C₁-C₄,
où alkyle en C₁-C₄ et alcoxy en C₁-C₄ sont le cas échéant substitué par un reste choisi parmi le groupe consistant en hydroxy, cyano, fluor, chlore, brome, hydroxycarbonyle et un groupe de formule -NR³R⁴,
où
R³ et R⁴ présentent les significations données à la revendication 7,
ainsi que leurs sels, solvates et/ou les solvates des sels, pour le traitement et/ou la prophylaxie de maladies chez l'homme.

10. Composés selon une des revendications 7 à 9, où R¹ présente les significations données aux revendications 7 à 9, et
R² représente phényle, N-oxydopyridyle ou pyridyle, où phényle est substitué par 1 à 3 restes et pyridyle et N-oxydopyridyle sont le cas échéant substitués par 1 à 3 restes, chaque fois indépendamment choisis parmi le groupe consistant en méthyle, éthyle, 2-propyle, trifluorométhyle, méthoxy, éthoxy, fluor et chlore,
ainsi que leurs sels, solvates et/ou les solvates des sels, pour le traitement de maladies chez l'homme.

11. Composés selon les revendications 7 à 10, où
R¹ représente alkyle en C₁-C₅ ou cycloalkyle en C₅-C₆, qui sont le cas échéant substitués par jusqu'à 3 restes indépendamment choisis parmi le groupe consistant en alkyle en C₁-C₄, trifluorométhyle, fluor, hydroxy, phénylcarbonylamino, (alkyl en C₁-C₄)carbonylamino, (alkyl en C₁-C₄)aminocarbonyle ou phénylaminocarbonyle,
R² représente phényle, N-oxydopyridyle ou pyridyle, où phényle est substitué par 1 à 3 restes et pyridyle et N-oxydopyridyle sont le cas échéant substitués par 1 à 3 restes, chaque fois indépendamment choisis parmi le groupe consistant en méthyle, éthyle, 2-propyle, trifluorométhyle, méthoxy, éthoxy, fluor et chlore,
ainsi que leurs sels, solvates et/ou les solvates des sels, pour le traitement et/ou la prophylaxie de maladies chez l'homme.

12. Composés selon une des revendications 7 à 11, où
R¹ représente alkyle en C₁-C₅ ou cycloalkyle en C₅-C₆, qui sont le cas échéant substitués par jusqu'à 3 restes indépendamment choisis parmi le groupe consistant en alkyle en C₁-C₄, fluor, trifluorométhyle, hydroxy, phénylcarbonylamino, (alkyl en C₁-C₄)carbonylamino, (alkyl en C₁-C₄)aminocarbonyle ou phénylaminocarbonyle,
R² représente phényle, N-oxydopyridyle ou pyridyle, où phényle est substitué par 1 reste et pyridyle et N-oxydopyridyle sont le cas échéant substitués par 1 reste, chaque fois indépendamment choisi parmi le groupe consistant en méthyle, éthyle, 2-propyle, trifluorométhyle, méthoxy, éthoxy, fluor et chlore,
ainsi que leurs sels, solvates et/ou les solvates des sels, pour le traitement et/ou la prophylaxie de maladies chez l'homme.

13. Procédé de préparation de composés selon la revendication 7, **caractérisé en ce que**
[A] on transforme des composés de formule : 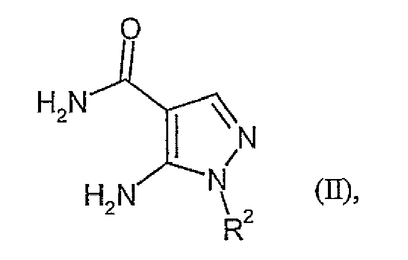 dans laquelle :
R² a les significations données à la revendication 7, par réaction avec un composé de formule : 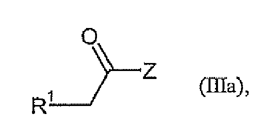
dans laquelle R¹ a les significations données à la revendication 7, et
Z représente chlore ou brome,
dans un solvant inerte et en présence d'une base, d'abord en des composés de formule : 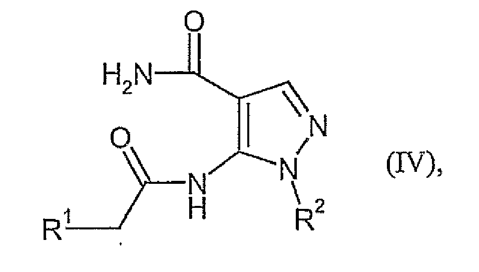 dans laquelle R¹ et R² ont les significations données à la revendication 7,
puis dans un solvant inerte en présence d'une base, on cycle en les composés de formule (I),
ou
[B] on transformer les composés de formule (II) par cyclisation directe en (I) avec un composé de formule : 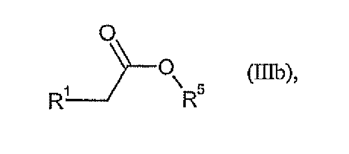 dans laquelle :
R¹ a les significations données à la revendication 7, et
R⁵ représente méthyle ou éthyle,
dans un solvant inerte et en présence d'une base, ou
[C] on transforme des composés de formule : 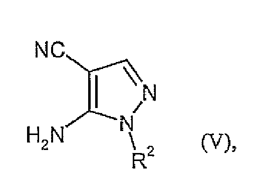 dans laquelle :
R² a les significations données à la revendication 7,
d'abord par réaction avec un composé de formule (IIIa) dans un solvant inerte et en présence d'une base, en les composés de formule : 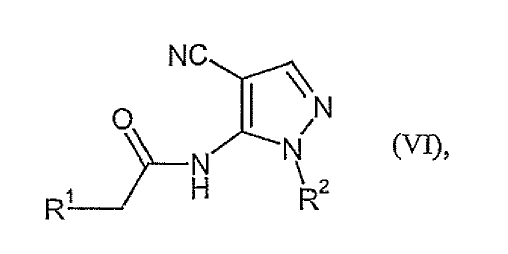
dans laquelle :
R¹ et R² ont les significations données à la revendication 7,
et ceux-ci sont cyclisés en (I), dans une deuxième étape, dans un solvant inerte et en présence d'une base et d'un agent d'oxydation,
et les composés de formule (I) résultants sont le cas échéant mis à réagir avec les solvants (i) et/ou bases ou acides (ii) appropriés en leurs solvates, sels et/ou solvates de sels.

14. Médicament contenant au moins un des composés selon l'une des revendications 7 à 12, et au moins un support ou excipient pharmaceutiquement compatible, essentiellement non toxique, pour le traitement et/ou la prophylaxie de maladies chez l'homme.

15. Utilisation des composés selon l'une des revendications 7 à 12, pour la préparation d'un médicament pour le traitement de maladies chez l'homme.

16. Utilisation des composés selon l'une des revendications 7 à 12, pour la préparation d'un médicament pour la prophylaxie et/ou le traitement de troubles de la perception, de la capacité de concentration, de l'aptitude à l'apprentissage et/ou à la mémorisation.

17. Utilisation selon la revendication 16, sachant que le trouble est une conséquence de la maladie d'Alzheimer.

18. Utilisation des composés selon l'une des revendications 7 à 12 pour la préparation d'un médicament pour l'amélioration de la perception, de la capacité de concentration, de l'aptitude à l'apprentissage et/ou à la mémorisation.

19. Utilisation des composés selon l'une des revendications 7 à 12, pour la préparation d'un médicament pour inhiber le PDE9A.
